# EUROPEAN PATENT APPLICATION

(11) **EP 4 361 147 A1**
(43) Date of publication of application: **01.05.2024**
(21) Application number: 22828590.4
(22) Date of filing: 25.04.2022
(51) Int. Cl.: C07D 409/04, C07D 491/048, C07D 403/04, C07D 405/14, C07D 409/14, C07D 403/10, H01L 51/00

(54) **HETEROCYCLIC COMPOUND, ORGANIC LIGHT-EMITTING DEVICE, COMPOSITION FOR ORGANIC MATERIAL LAYER OF ORGANIC LIGHT-EMITTING DEVICE**

(30) Priority: 24.06.2021 KR 20210082538
(71) Applicant: LT Materials Co., Ltd., Yongin-si, Gyeonggi-do 17118 (KR)
(72) Inventor: CHOI, Eui-Jeong, Yongin-si, Gyeonggi-do 17118 (KR); NO, Young-Seok, Yongin-si, Gyeonggi-do 17118 (KR); KIM, Dong-Jun, Yongin-si, Gyeonggi-do 17118 (KR)
(74) Representative: Goddar, Heinz J.
(86) International application number: PCT/KR2022/005865
(87) International publication number: WO 2022/270741

(57) **Abstract**

The present specification relates to a heterocyclic compound, an organic light emitting device, and a composition for an organic material layer of an organic light emitting device.

## Description

### [Technical Field]

This application claims priority to and the benefit of Korean Patent Application No. 10-2021-0082538 filed in the Korean Intellectual Property Office on June 24, 2021, the entire contents of which are incorporated herein by reference.

The present specification relates to a heterocyclic compound, an organic light emitting device, and a composition for an organic material layer of an organic light emitting device.

### [Background Art]

An electroluminescence device is a kind of self-emitting type display device, and has an advantage in that the viewing angle is wide, the contrast is excellent, and the response speed is fast.

An organic light emitting device has a structure in which an organic thin film is disposed between two electrodes. When a voltage is applied to an organic light emitting device having the structure, electrons and holes injected from the two electrodes combine with each other in an organic thin film to make a pair, and then, emit light while being extinguished. The organic thin film may be composed of a single layer or multi layers, if necessary.

A material for the organic thin film may have a light emitting function, if necessary. For example, as the material for the organic thin film, it is also possible to use a compound, which may itself constitute a light emitting layer alone, or it is also possible to use a compound, which may serve as a host or a dopant of a host-dopant-based light emitting layer. In addition, as a material for the organic thin film, it is also possible to use a compound, which may play a role such as a hole injection, hole transport, electron blocking, hole blocking, electron transport or electron injection.

In order to improve the performance, service life, or efficiency of the organic light emitting device, there is a continuous need for developing a material for an organic thin film.

### [Related Art Document]

### [Patent Document]

(Patent Document 1) US Patent No. 4,356,429

### [Disclosure]

### [Technical Problem]

The present specification has been made in an effort to provide a heterocyclic compound, an organic light emitting device, and a composition for an organic material layer of an organic light emitting device.

### [Technical Solution]

In an exemplary embodiment of the present specification, provided is a heterocyclic compound of the following Chemical Formula 1. 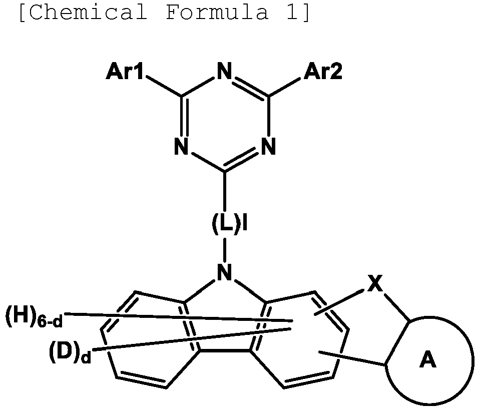 In Chemical Formula 1,
X is O; S; or CRR',
L is a direct bond; or a substituted or unsubstituted arylene group having 6 to 60 carbon atoms,
l is an integer from 1 to 3, and when l is 2 or higher, L's are the same as or different from each other,
A is an aryl ring having 6 to 60 carbon atoms, which is unsubstituted or substituted with deuterium; or a hetero ring having 2 to 60 carbon atoms, which is unsubstituted or substituted with deuterium,
R, R', Ar1 and Ar2 are each independently hydrogen; deuterium; a halogen group; a cyano group; a substituted or unsubstituted alkyl group having 1 to 60 carbon atoms; a substituted or unsubstituted cycloalkyl group having 3 to 60 carbon atoms; a substituted or unsubstituted heterocycloalkyl group having 2 to 60 carbon atoms; a substituted or unsubstituted aryl group having 6 to 60 carbon atoms; or a substituted or unsubstituted heteroaryl group having 2 to 60 carbon atoms,
H is hydrogen and D is deuterium,
d is an integer from 0 to 6, and
the deuterium content of 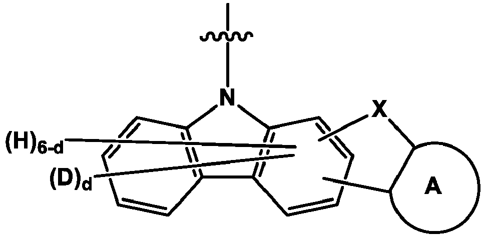 of Chemical Formula 1 is more than 0%.

In another exemplary embodiment of the present specification, provided is an organic light emitting device including: a first electrode; a second electrode disposed to face the first electrode; and an organic material layer having one or more layers disposed between the first electrode and the second electrode, in which one or more layers of the organic material layer include the heterocyclic compound.

In still another exemplary embodiment of the present specification, provided is a composition for an organic material layer of an organic light emitting device, including the heterocyclic compound.

### [Advantageous Effects]

When used in an organic light emitting device, the heterocyclic compound described in the present specification can lower the driving voltage of the device, improve the light efficiency, and improve the service life characteristics of the device. In particular, when the heterocyclic compound of Chemical Formula 1 is used as a material for a light emitting layer of the organic light emitting device by necessarily including deuterium at a position of 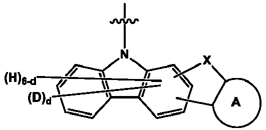 having a core structure, the stability of molecules is enhanced by reducing changes in vibrational frequency and thermal stability is increased due to a high single bond dissociation energy, so that excellent performance is provided in terms of driving voltage, light emitting efficiency and service life.

### [Description of Drawings]

FIGS. 1 to 3 are views each exemplarily illustrating a stacking structure of an organic light emitting device according to an exemplary embodiment of the present specification.
FIG. 4 is a view obtained by an experiment of confirming a recombination zone (RZ).
FIGS. 5 and 6 are views illustrating the results of an experiment of confirming the current density according to voltage by manufacturing a hole only device (HOD).

### [Best Mode]

Hereinafter, the present specification will be described in more detail.

When one part "includes" one constituent element in the present specification, unless otherwise specifically described, this does not mean that another constituent element is excluded, but means that another constituent element may be further included.

In the present specification, 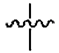 of a chemical formula means a position to which a constituent element is bonded.

The term "substitution" means that a hydrogen atom bonded to a carbon atom of a compound is changed into another substituent, and a position to be substituted is not limited as long as the position is a position at which the hydrogen atom is substituted, that is, a position at which the substituent may be substituted, and when two or more are substituted, the two or more substituents may be the same as or different from each other.

In the present specification, "substituted or unsubstituted" means being unsubstituted or substituted with one or more substituents selected from the group consisting of deuterium; a halogen group; a cyano group; a C1 to C60 alkyl group; a C2 to C60 alkenyl group; a C2 to C60 alkynyl group; a C3 to C60 cycloalkyl group; a C2 to C60 heterocycloalkyl group; a C6 to C60 aryl group; a C2 to C60 heteroaryl group; a silyl group; a phosphine oxide group; and an amine group, or with a substituent to which two or more substituents among the substituents are bonded.

In the present specification, "when a substituent is not indicated in the structure of a chemical formula or compound" means that a hydrogen atom is bonded to a carbon atom. However, since deuterium (²H) is an isotope of hydrogen, some hydrogen atoms may be deuterium.

In an exemplary embodiment of the present application, "when a substituent is not indicated in the structure of a chemical formula or compound" may mean that all the positions that may be reached by the substituent are hydrogen; or deuterium. That is, deuterium is an isotope of hydrogen, and some hydrogen atoms may be deuterium which is an isotope, and in this case, the content of deuterium may be 0% to 100%.

In an exemplary embodiment of the present application, in "the case where a substituent is not indicated in the structure of a chemical formula or compound", when the content of deuterium is 0%, the content of hydrogen is 100%, and all the substituents do not explicitly exclude deuterium such as hydrogen, hydrogen and deuterium may be mixed and used in the compound.

In an exemplary embodiment of the present application, deuterium is one of the isotopes of hydrogen, is an element that has a deuteron composed of one proton and one neutron as a nucleus, and may be represented by hydrogen-2, and the element symbol may also be expressed as D or ²H.

In an exemplary embodiment of the present application, the isotope means an atom with the same atomic number (Z), but different mass numbers (A), and the isotope may be interpreted as an element which has the same number of protons, but different number of neutrons.

In an exemplary embodiment of the present application, when the total number of substituents of a basic compound is defined as T1 and the number of specific substituents among the substituents is defined as T2, the content T% of the specific substituent may be defined as T2/T1×100 = T%.

That is, in an example, the deuterium content of 20% in a phenyl group represented by 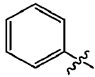 may be represented by 20% when the total number of substituents that the phenyl group can have is 5 (T1 in the formula) and the number of deuteriums among the substituents is 1 (T2 in the formula). That is, a deuterium content of 20% in the phenyl group may be represented by the following structural formula.

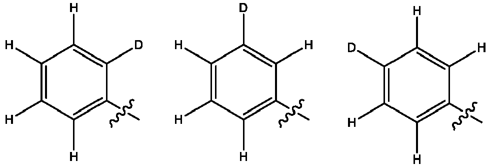

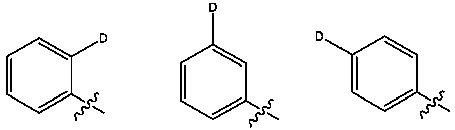

Further, in an exemplary embodiment of the present application, "a phenyl group having a deuterium content of 0%" may mean a phenyl group that does not include a deuterium atom, that is, has five hydrogen atoms.

In the present specification, the halogen may be fluorine, chlorine, bromine or iodine.

In the present specification, the alkyl group includes a straight-chain or branched-chain having 1 to 60 carbon atoms, and may be additionally substituted with another substituent. The number of carbon atoms of the alkyl group may be 1 to 60, specifically 1 to 40, and more specifically 1 to 20. Specific examples thereof include a methyl group, an ethyl group, a propyl group, an n-propyl group, an isopropyl group, a butyl group, an n-butyl group, an isobutyl group, a tert-butyl group, a sec-butyl group, a 1-methyl-butyl group, a 1-ethyl-butyl group, a pentyl group, an n-pentyl group, an isopentyl group, a neopentyl group, a tert-pentyl group, a hexyl group, an n-hexyl group, a 1-methylpentyl group, a 2-methylpentyl group, a 4-methyl-2-pentyl group, a 3,3-dimethylbutyl group, a 2-ethylbutyl group, a heptyl group, an n-heptyl group, a 1-methylhexyl group, a cyclopentylmethyl group, a cyclohexylmethyl group, an octyl group, an n-octyl group, a tert-octyl group, a 1-methylheptyl group, a 2-ethylhexyl group, a 2-propylpentyl group, an n-nonyl group, a 2,2-dimethylheptyl group, a 1-ethyl-propyl group, a 1,1-dimethyl-propyl group, an isohexyl group, a 2-methylpentyl group, a 4-methylhexyl group, a 5-methylhexyl group, and the like, but are not limited thereto.

In the present specification, the alkenyl group includes a straight-chain or branched-chain having 2 to 60 carbon atoms, and may be additionally substituted with another substituent. The number of carbon atoms of the alkenyl group may be 2 to 60, specifically 2 to 40, and more specifically 2 to 20. Specific examples thereof include a vinyl group, a 1-propenyl group, an isopropenyl group, a 1-butenyl group, a 2-butenyl group, a 3-butenyl group, a 1-pentenyl group, a 2-pentenyl group, a 3-pentenyl group, a 3-methyl-1-butenyl group, a 1,3-butadienyl group, an allyl group, a 1-phenylvinyl-1-yl group, a 2-phenylvinyl-1-yl group, a 2,2-diphenylvinyl-1-yl group, a 2-phenyl-2-(naphthyl-1-yl)vinyl-1-yl group, a 2,2-bis(diphenyl-1-yl)vinyl-1-yl group, a stilbenyl group, a styrenyl group, and the like, but are not limited thereto.

In the present specification, the alkynyl group includes a straight-chain or branched-chain having 2 to 60 carbon atoms, and may be additionally substituted with another substituent. The number of carbon atoms of the alkynyl group may be 2 to 60, specifically 2 to 40, and more specifically 2 to 20.

In the present specification, an alkoxy group may be straight-chained, branched, or cyclic. The number of carbon atoms of the alkoxy group is not particularly limited, but is preferably 1 to 20. Specific examples thereof include methoxy, ethoxy, n-propoxy, isopropoxy, n-butoxy, isobutoxy, tert-butoxy, sec-butoxy, n-pentyloxy, neopentyloxy, isopentyloxy, n-hexyloxy, 3,3-dimethylbutyloxy, 2-ethylbutyloxy, n-octyloxy, n-nonyloxy, n-decyloxy, benzyloxy, p-methylbenzyloxy, and the like, but are not limited thereto.

In the present specification, the cycloalkyl group includes a monocycle or polycycle having 3 to 60 carbon atoms, and may be additionally substituted with another substituent. Here, the polycycle means a group in which a cycloalkyl group is directly linked to or fused with another cyclic group. Here, another cyclic group may also be a cycloalkyl group, but may also be another kind of cyclic group, for example, a heterocycloalkyl group, an aryl group, a heteroaryl group, and the like. The number of carbon atoms of the cycloalkyl group may be 3 to 60, specifically 3 to 40, and more specifically 5 to 20. Specific examples thereof include a cyclopropyl group, a cyclobutyl group, a cyclopentyl group, a 3-methylcyclopentyl group, a 2,3-dimethylcyclopentyl group, a cyclohexyl group, a 3-methylcyclohexyl group, a 4-methylcyclohexyl group, a 2,3-dimethylcyclohexyl group, a 3,4,5-trimethylcyclohexyl group, a 4-tert-butylcyclohexyl group, a cycloheptyl group, a cyclooctyl group, and the like, but are not limited thereto.

In the present specification, the heterocycloalkyl group includes O, S, Se, N, or Si as a heteroatom, includes a monocycle or polycycle having 2 to 60 carbon atoms, and may be additionally substituted with another substituent. Here, the polycycle means a group in which a heterocycloalkyl group is directly linked to or fused with another cyclic group. Here, another cyclic group may also be a heterocycloalkyl group, but may also be another kind of cyclic group, for example, a cycloalkyl group, an aryl group, a heteroaryl group, and the like. The number of carbon atoms of the heterocycloalkyl group may be 2 to 60, specifically 2 to 40, and more specifically 3 to 20.

In the present specification, the aryl group includes a monocycle or polycycle having 6 to 60 carbon atoms, and may be additionally substituted with another substituent. Here, the polycycle means a group in which an aryl group is directly linked to or fused with another cyclic group. Here, another cyclic group may also be an aryl group, but may also be another kind of cyclic group, for example, a cycloalkyl group, a heterocycloalkyl group, a heteroaryl group, and the like. The aryl group includes a spiro group. The number of carbon atoms of the aryl group may be 6 to 60, specifically 6 to 40, and more specifically 6 to 25. Specific examples of the aryl group include a phenyl group, a biphenyl group, a terphenyl group, a naphthyl group, an anthryl group, a chrysenyl group, a phenanthrenyl group, a perylenyl group, a fluoranthenyl group, a triphenylenyl group, a phenalenyl group, a pyrenyl group, a tetracenyl group, a pentacenyl group, a fluorenyl group, an indenyl group, an acenaphthylenyl group, a benzofluorenyl group, a spirobifluorenyl group, a 2,3-dihydro-1H-indenyl group, a fused cyclic group thereof, and the like, but are not limited thereto.

In the present specification, the terphenyl group may be selected from the following structures.

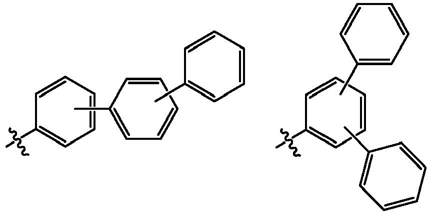

In the present specification, the fluorenyl group may be substituted, and adjacent substituents may be bonded to each other to form a ring.

When the fluorenyl group is substituted, the substituent may be 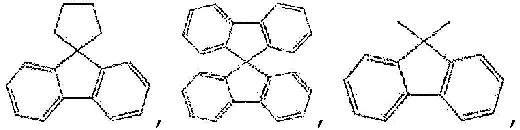 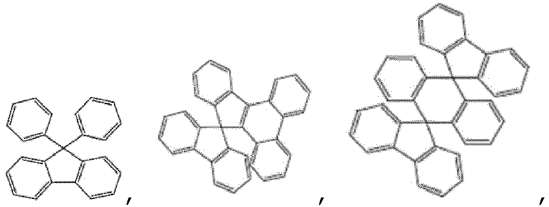 and the like, but is not limited thereto.

In the present specification, the heteroaryl group includes S, O, Se, N, or Si as a heteroatom, includes a monocycle or polycycle having 2 to 60 carbon atoms, and may be additionally substituted with another substituent. Here, the polycycle means a group in which a heteroaryl group is directly linked to or fused with another cyclic group. Here, another cyclic group may also be a heteroaryl group, but may also be another kind of cyclic group, for example, a cycloalkyl group, a heterocycloalkyl group, an aryl group, and the like. The number of carbon atoms of the heteroaryl group may be 2 to 60, specifically 2 to 40, and more specifically 3 to 25. Specific examples of the heteroaryl group include a pyridyl group, a pyrrolyl group, a pyrimidyl group, a pyridazinyl group, a furanyl group, a thiophene group, an imidazolyl group, a pyrazolyl group, an oxazolyl group, an isoxazolyl group, a thiazolyl group, an isothiazolyl group, a triazolyl group, a furazanyl group, an oxadiazolyl group, a thiadiazolyl group, a dithiazolyl group, a tetrazolyl group, a pyranyl group, a thiopyranyl group, a diazinyl group, an oxazinyl group, a thiazinyl group, a dioxynyl group, a triazinyl group, a tetrazinyl group, a quinolyl group, an isoquinolyl group, a quinazolinyl group, an isoquinazolinyl group, a quinozolilyl group, a naphthyridyl group, an acridinyl group, a phenanthridinyl group, an imidazopyridinyl group, a diaza naphthalenyl group, a triazaindene group, an indolyl group, an indolizinyl group, a benzothiazolyl group, a benzoxazolyl group, a benzimidazolyl group, a benzothiophene group, a benzofuran group, a dibenzothiophene group, a dibenzofuran group, a carbazolyl group, a benzocarbazolyl group, a dibenzocarbazolyl group, a phenazinyl group, a dibenzosilole group, spirobi (dibenzosilole), a dihydrophenazinyl group, a phenoxazinyl group, a phenanthridyl group, an imidazopyridinyl group, a thienyl group, an indolo[2,3-a]carbazolyl group, an indolo[2,3-b]carbazolyl group, an indolinyl group, a 10,11-dihydro-dibenzo[b,f]azepin group, a 9,10-dihydroacridinyl group, a phenanthrazinyl group, a phenothiazinyl group, a phthalazinyl group, a naphthylidinyl group, a phenanthrolinyl group, a benzo[c][1,2,5]thiadiazolyl group, 2,3-dihydrobenzo[b]thiophene, 2,3-dihydrobenzofuran, a 5,10-dihydrodibenzo[b,e][1,4]azasilinyl, a pyrazolo[1,5-c]quinazolinyl group, a pyrido[1,2-b]indazolyl group, a pyrido[1,2-a]imidazo[1,2-e]indolinyl group, a 5,11-dihydroindeno[1,2-b]carbazolyl group, and the like, but are not limited thereto.

In the present specification, a silyl group includes Si and is a substituent to which the Si atom is directly linked as a radical, and is represented by - Si(R101)(R102)(R103), and R101 to R103 are the same as or different from each other, and may be each independently a substituent composed of at least one of hydrogen; deuterium; a halogen group; an alkyl group; an alkenyl group; an alkoxy group; a cycloalkyl group; a heterocycloalkyl group; an aryl group; and a heteroaryl group. Specific examples of the silyl group include 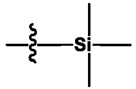 (a trimethylsilyl group), 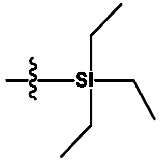 (a triethylsilyl group), 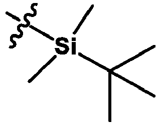 (a t-butyldimethylsilyl group), 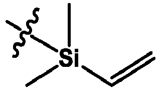 (a vinyldimethylsilyl group), 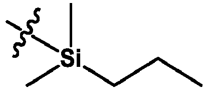 (a propyldimethylsilyl group), 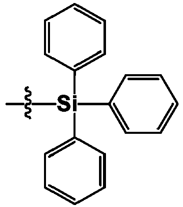 (a triphenylsilyl group), 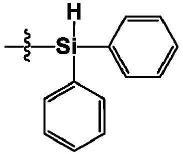 (a diphenylsilyl group), 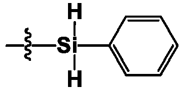 (a phenylsilyl group) and the like, but are not limited thereto.

In the present specification, the phosphine oxide group is represented by -P(=O)(R104)(R105), and R104 and R105 are the same as or different from each other, and may be each independently a substituent composed of at least one of hydrogen; deuterium; a halogen group; an alkyl group; an alkenyl group; an alkoxy group; a cycloalkyl group; a heterocycloalkyl group; an aryl group; and a heteroaryl group. Specifically, the phosphine oxide group may be substituted with an alkyl group or an aryl group, and the above-described example may be applied to the alkyl group and the aryl group. Examples of the phosphine oxide group include a dimethylphosphine oxide group, a diphenylphosphine oxide group, dinaphthylphosphine oxide, and the like, but are not limited thereto.

In the present specification, the amine group is represented by -N(R106)(R107), and R106 and R107 are the same as or different from each other, and are each independently a substituent composed of at least one of hydrogen; deuterium; a halogen group; an alkyl group; an alkenyl group; an alkoxy group; a cycloalkyl group; a heterocycloalkyl group; an aryl group; and a heteroaryl group. The amine group may be selected from the group consisting of -NH₂; a monoalkylamine group; a monoarylamine group; a monoheteroarylamine group; a dialkylamine group; a diarylamine group; a diheteroarylamine group; an alkylarylamine group; an alkylheteroarylamine group; and an arylheteroarylamine group, and the number of carbon atoms thereof is not particularly limited, but is preferably 1 to 30. Specific examples of the amine group include a methylamine group, a dimethylamine group, an ethylamine group, a diethylamine group, a phenylamine group, a naphthylamine group, a biphenylamine group, a dibiphenylamine group, an anthracenylamine group, a 9-methyl-anthracenylamine group, a diphenylamine group, a phenylnaphthylamine group, a ditolylamine group, a phenyltolylamine group, a triphenylamine group, a biphenylnaphthylamine group, a phenylbiphenylamine group, a biphenylfluorenylamine group, a phenyltriphenylenylamine group, a biphenyltriphenylenylamine group, and the like, but are not limited thereto.

In the present specification, the above-described examples of the aryl group may be applied to an arylene group except for a divalent arylene group.

In the present specification, the above-described examples of the heteroaryl group may be applied to a heteroarylene group except for a divalent heteroarylene group.

An exemplary embodiment of the present specification provides the heterocyclic compound represented by Chemical Formula 1.

In an exemplary embodiment of the present specification, A is an aryl ring having 6 to 30 carbon atoms, which is unsubstituted or substituted with deuterium; or a hetero ring having 2 to 30 carbon atoms, which is unsubstituted or substituted with deuterium.

In an exemplary embodiment of the present specification, A is an aryl ring having 6 to 15 carbon atoms, which is unsubstituted or substituted with deuterium; or a hetero ring having 2 to 15 carbon atoms, which is unsubstituted or substituted with deuterium.

In an exemplary embodiment of the present specification, A is an aryl ring having 6 to 15 carbon atoms, which is unsubstituted or substituted with deuterium.

In an exemplary embodiment of the present specification, A is a benzene ring which is unsubstituted or substituted with deuterium.

In an exemplary embodiment of the present specification, Chemical Formula 1 may be represented by the following Chemical Formula 1-1.

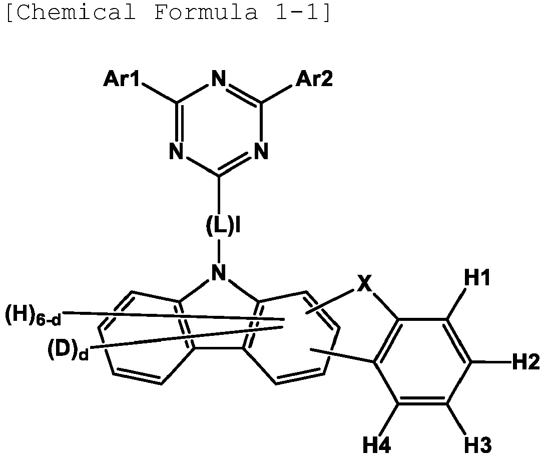

In Chemical Formula 1-1, H1 to H4 are each independently hydrogen; or deuterium, and the definitions of the other substituents are the same as the definitions in Chemical Formula 1.

In an exemplary embodiment of the present specification, Chemical Formula 1 may be represented by any one of the following Chemical Formulae 1-1-1 to 1-1-6.

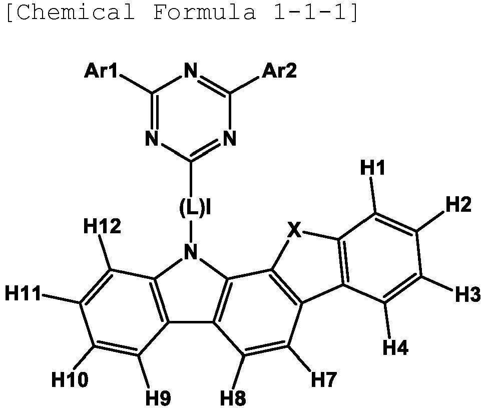

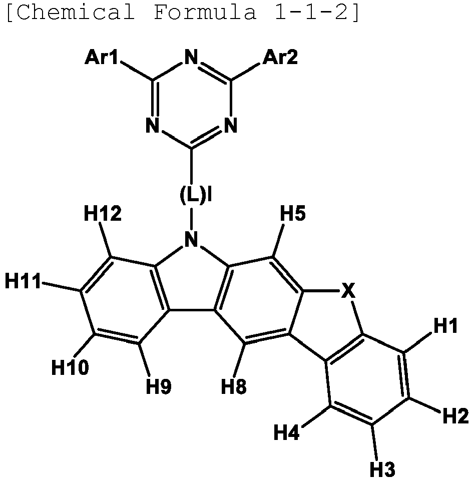

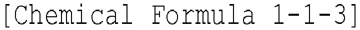

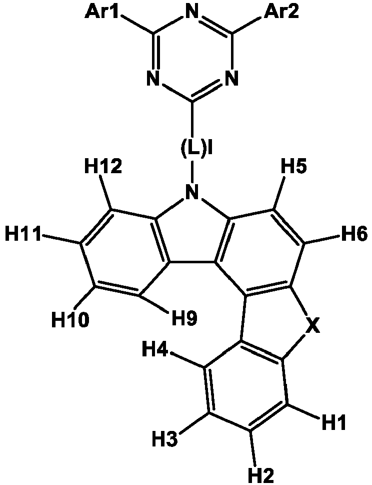

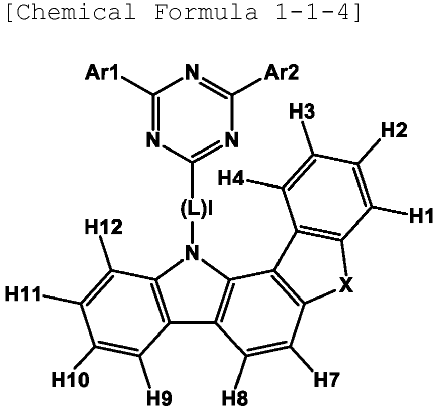

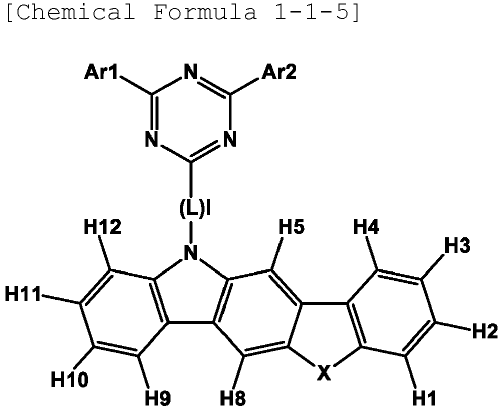

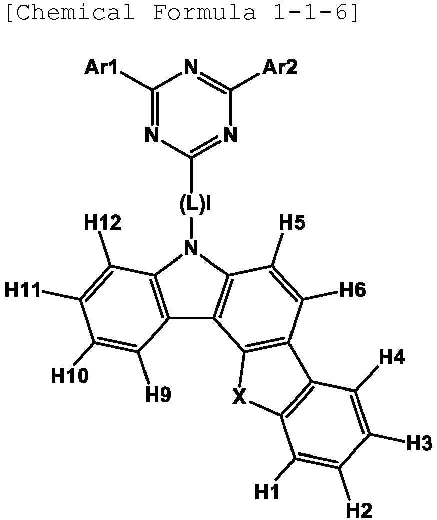

In Chemical Formulae 1-1-1 to 1-1-6,
H1 to H12 are each independently hydrogen; or deuterium, at least one of H1 to H12 is deuterium, and the definitions of the other substituents are the same as the definitions in Chemical Formula 1.

In an exemplary embodiment of the present specification, X is O; S; or CRR', R and R' are each independently a substituted or unsubstituted alkyl group having 1 to 30 carbon atoms; a substituted or unsubstituted cycloalkyl group having 3 to 30 carbon atoms; a substituted or unsubstituted heterocycloalkyl group having 2 to 30 carbon atoms; a substituted or unsubstituted aryl group having 6 to 30 carbon atoms; or a substituted or unsubstituted heteroaryl group having 2 to 30 carbon atoms.

In an exemplary embodiment of the present specification, X is O; S; or CRR', and R and R' are each independently a substituted or unsubstituted alkyl group having 1 to 30 carbon atoms; a substituted or unsubstituted aryl group having 6 to 30 carbon atoms; or a substituted or unsubstituted heteroaryl group having 2 to 30 carbon atoms.

In an exemplary embodiment of the present specification, X is O; S; or CRR', and R and R' are each independently a substituted or unsubstituted alkyl group having 1 to 30 carbon atoms.

In an exemplary embodiment of the present specification, X is O; S; or CRR', and R and R' are each independently a substituted or unsubstituted alkyl group having 1 to 10 carbon atoms.

In an exemplary embodiment of the present specification, X is O; S; or CRR', and R and R' are each independently an alkyl group having 1 to 5 carbon atoms, which is unsubstituted or substituted with deuterium.

In an exemplary embodiment of the present specification, X is O; S; or CRR', and R and R' are each independently a methyl group, which is unsubstituted or substituted with deuterium.

In an exemplary embodiment of the present specification, L is a direct bond; or a substituted or unsubstituted C6 to C30 arylene group.

In an exemplary embodiment of the present specification, L is a direct bond; or a substituted or unsubstituted C6 to C12 arylene group.

In an exemplary embodiment of the present specification, L is a direct bond; a substituted or unsubstituted phenylene group; a substituted or unsubstituted biphenylene group; or a substituted or unsubstituted naphthylene group.

In an exemplary embodiment of the present specification, L is a direct bond; a phenylene group; a biphenylene group; or a naphthylene group.

In an exemplary embodiment of the present specification, L may be a direct bond or represented by any one of the following structural formulae.

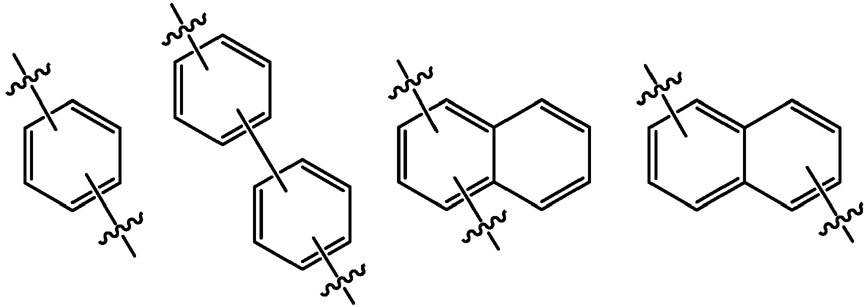

In the structural formulae, 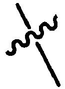 means a position where triazine and condensed carbazole are each bonded.

In an exemplary embodiment of the present specification, Ar1 and Ar2 are each independently a substituted or unsubstituted aryl group having 6 to 60 carbon atoms; or a substituted or unsubstituted heteroaryl group having 2 to 60 carbon atoms.

In an exemplary embodiment of the present specification, Ar1 and Ar2 are each independently a substituted or unsubstituted aryl group having 6 to 30 carbon atoms; or a substituted or unsubstituted heteroaryl group having 2 to 30 carbon atoms.

In an exemplary embodiment of the present specification, Ar1 and Ar2 are each independently a substituted or unsubstituted aryl group having 6 to 30 carbon atoms; or a heteroaryl group having 2 to 30 carbon atoms, which is unsubstituted or substituted and includes O or S.

In an exemplary embodiment of the present specification, Ar1 and Ar2 are each independently an aryl group having 6 to 30 carbon atoms, which is unsubstituted or substituted with an aryl group having 6 to 20 carbon atoms; or a heteroaryl group having 2 to 30 carbon atoms.

In an exemplary embodiment of the present specification, Ar1 and Ar2 are each independently a substituted or unsubstituted phenyl group; a substituted or unsubstituted biphenyl group; a substituted or unsubstituted terphenyl group; a substituted or unsubstituted dibenzofuran group; or a substituted or unsubstituted dibenzothiophene group.

In an exemplary embodiment of the present specification, Ar1 and Ar2 are each independently a phenyl group which is unsubstituted or substituted with an aryl group having 6 to 20 carbon atoms; a biphenyl group which is unsubstituted or substituted with an aryl group having 6 to 20 carbon atoms; a terphenyl group which is unsubstituted or substituted with an aryl group having 6 to 20 carbon atoms; a dibenzofuran group; or a dibenzothiophene group.

In an exemplary embodiment of the present specification, Ar1 is a substituted or unsubstituted phenyl group; a substituted or unsubstituted biphenyl group; a substituted or unsubstituted dibenzofuran group; or a substituted or unsubstituted dibenzothiophene group.

In an exemplary embodiment of the present specification, Ar2 is a substituted or unsubstituted phenyl group; a substituted or unsubstituted biphenyl group; a substituted or unsubstituted terphenyl group; a substituted or unsubstituted dibenzofuran group; or a substituted or unsubstituted dibenzothiophene group.

In an exemplary embodiment of the present specification, when Ar1 is a substituted or unsubstituted phenyl group, Ar2 is a substituted or unsubstituted phenyl group; a substituted or unsubstituted biphenyl group; a substituted or unsubstituted terphenyl group; a substituted or unsubstituted dibenzofuran group; or a substituted or unsubstituted dibenzothiophene group.

In an exemplary embodiment of the present specification, when Ar1 is a substituted or unsubstituted biphenyl group, Ar2 is a substituted or unsubstituted biphenyl group; a substituted or unsubstituted dibenzofuran group; or a substituted or unsubstituted dibenzothiophene group.

In an exemplary embodiment of the present specification, when Ar1 is a substituted or unsubstituted dibenzofuran group, Ar2 is a substituted or unsubstituted dibenzofuran group; or a substituted or unsubstituted dibenzothiophene group.

In an exemplary embodiment of the present specification, when Ar1 is a substituted or unsubstituted dibenzothiophene group, Ar2 is a substituted or unsubstituted dibenzofuran group; or a substituted or unsubstituted dibenzothiophene group.

In an exemplary embodiment of the present specification, 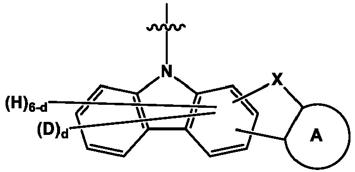 of Chemical Formula 1 necessarily includes deuterium.

In an electron transport moiety and a hole transport moiety, which directly exchange electrons, the vibrational frequency of interatomic bond in molecules changes continuously as electrons move, affecting the stability of interatomic bond in molecules and the stability of the molecular structure. By substituting the compound with deuterium, which has a higher molecular weight than hydrogen, the energy of the molecule is lowered by reducing the change in vibrational frequency, and thus the stability of molecules is enhanced.

In addition, since the single bond dissociation energy of carbon and deuterium is higher than the single bond dissociation energy of carbon and hydrogen, the thermal stability of molecule is increased, so that there is an effect of improving the service life of the device.

In an exemplary embodiment of the present specification, Chemical Formula 1 includes the structures of the following Chemical Formulae A and B, and selectively may include the structure of the following Chemical Formula C.

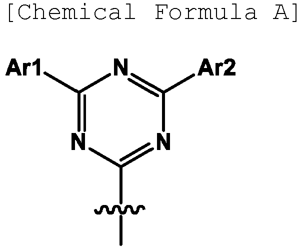

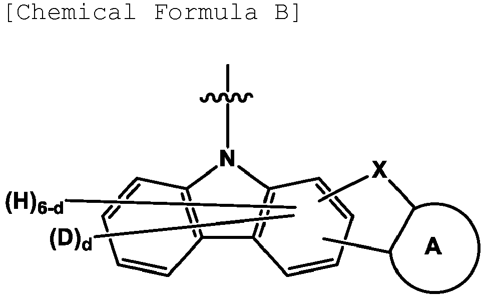

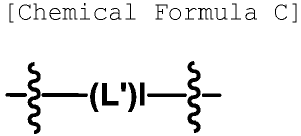

In Chemical Formulae A to C, 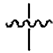 of each structure means a site that is bonded to another structure, and
L' is a substituted or unsubstituted arylene group having 6 to 60 carbon atoms, and the definitions of the other substituents are the same as the definitions in Chemical Formula 1.

In an exemplary embodiment of the present specification, when L of Chemical Formula 1 is a direct bond, Chemical Formula 1 may be represented by the bond of [Chemical Formula A]-[Chemical Formula B].

In an exemplary embodiment of the present specification, when L of Chemical Formula 1 is not a direct bond, Chemical Formula 1 may be represented by the bond of [Chemical Formula A]-[Chemical Formula C]-[Chemical Formula B] .

In an exemplary embodiment of the present application, provided is a heterocyclic compound in which Chemical Formula 1 includes the structures of Chemical Formulae A and B and selectively includes the structure of Chemical Formula C, and a deuterium content of Chemical Formula B is 50% or more and 100% or less.

In an exemplary embodiment of the present specification, a deuterium content of B may be more than 0%, 30% or more, 50% or more, and 100% or less.

In an exemplary embodiment of the present specification, deuterium contents of Chemical Formulae A and C may be 0%.

In an exemplary embodiment of the present specification, the photochemical characteristics of a compound which includes deuterium and a compound which does not include deuterium are almost similar, but when deposited on a thin film, the deuterium-containing material tends to be packed with a narrower intermolecular distance.

Accordingly, when an electron only device (EOD) and a hole only device (HOD) are manufactured and the current density thereof according to voltage is confirmed, it can be confirmed that the compound of Chemical Formula 1 according to the present application, which includes deuterium, exhibits a much more balanced charge transport characteristics than the compound which does not include deuterium.

Further, when the surface of a thin film is observed using an atomic force microscope (AFM), it can be confirmed that the thin film made of a compound including deuterium is deposited with a more uniform surface without any aggregated portion.

In an exemplary embodiment of the present specification, Chemical Formula 1 may be represented by any one of the following compounds.

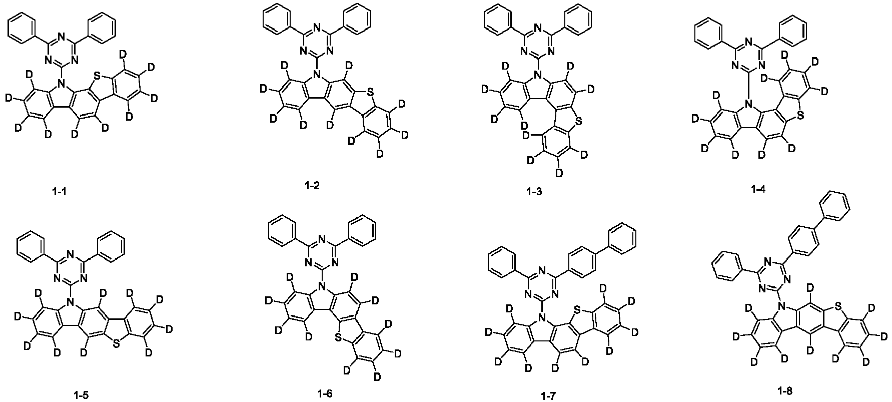

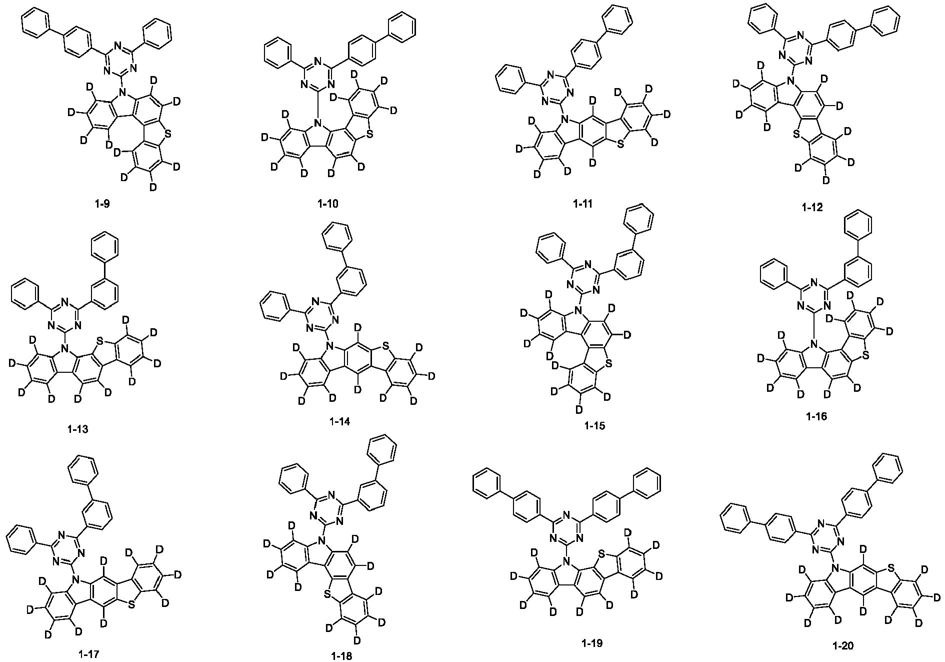

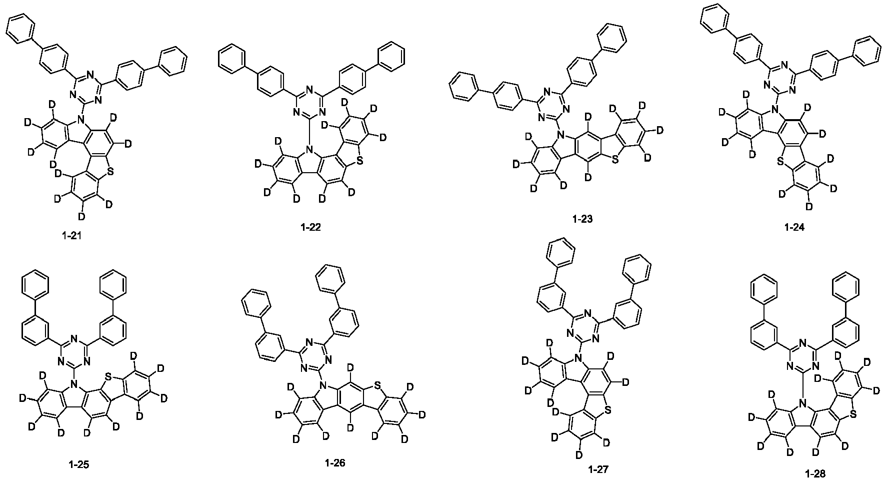

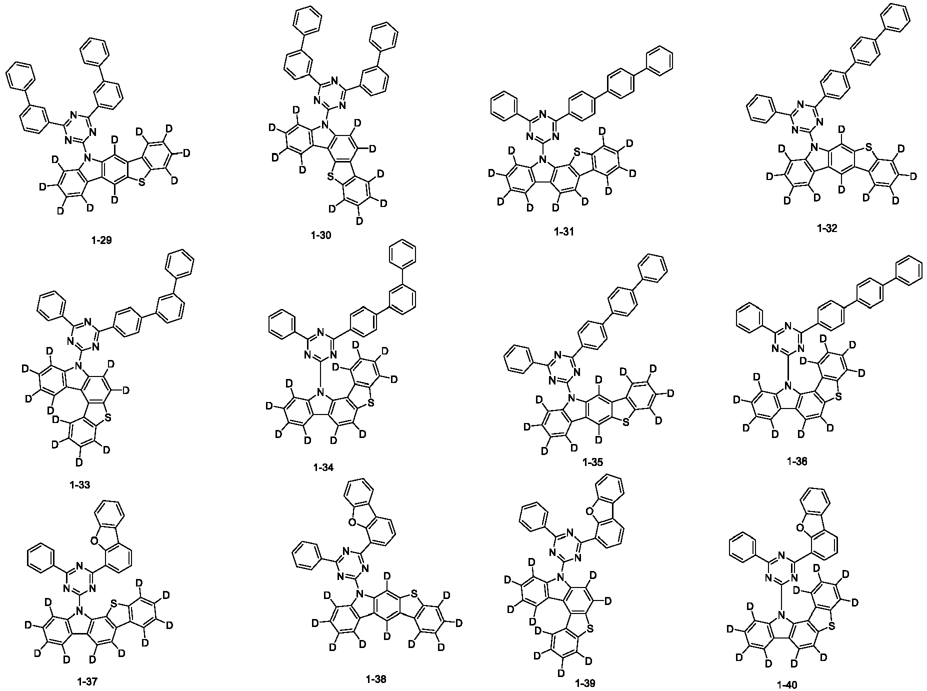

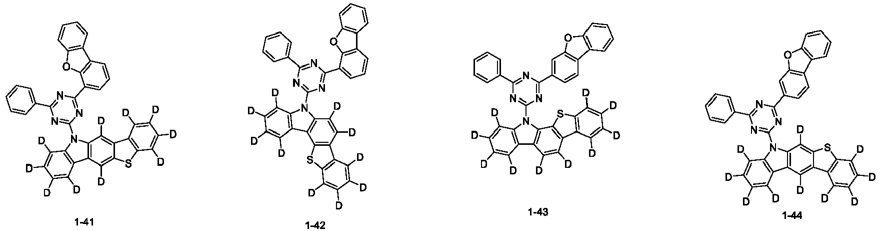

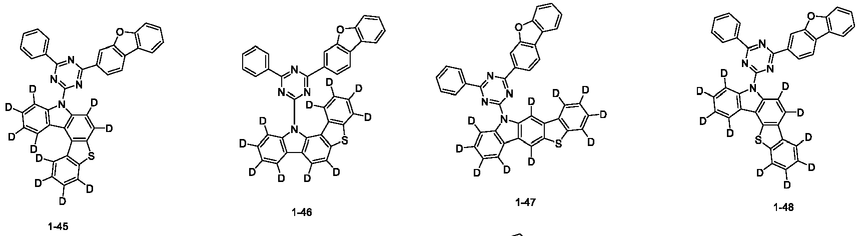

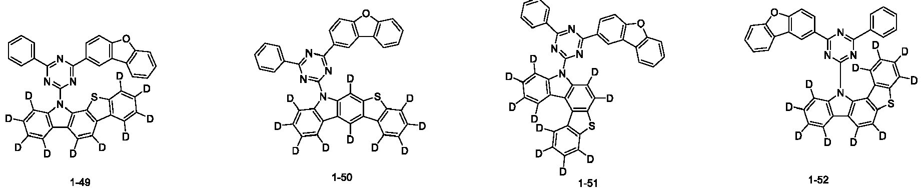

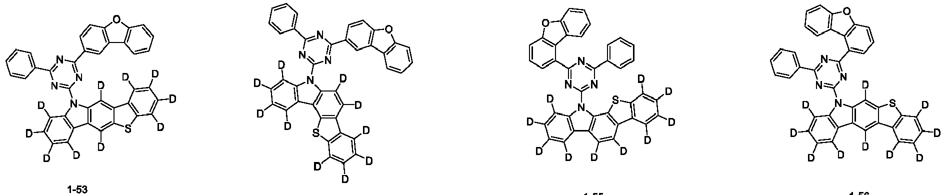

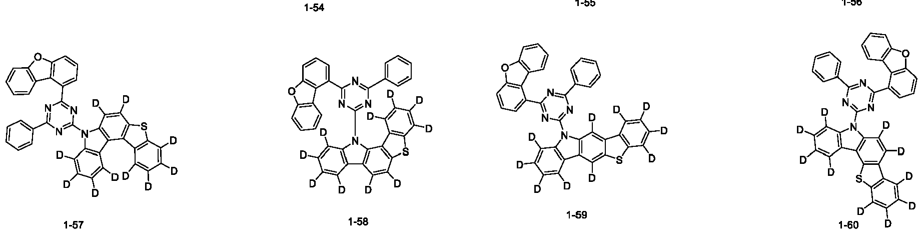

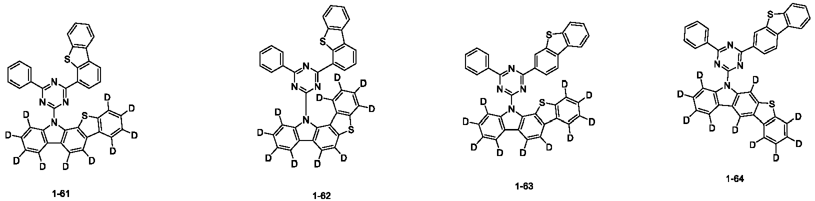

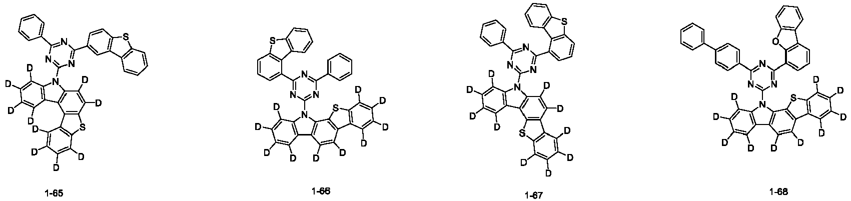

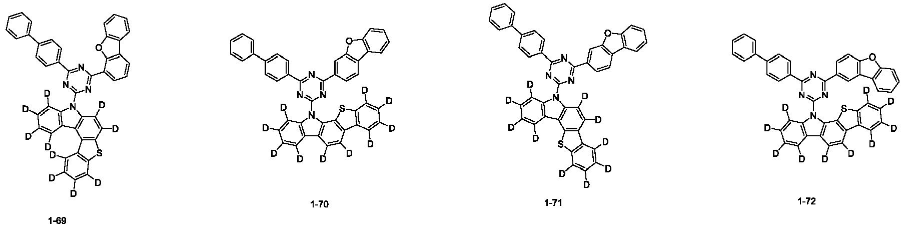

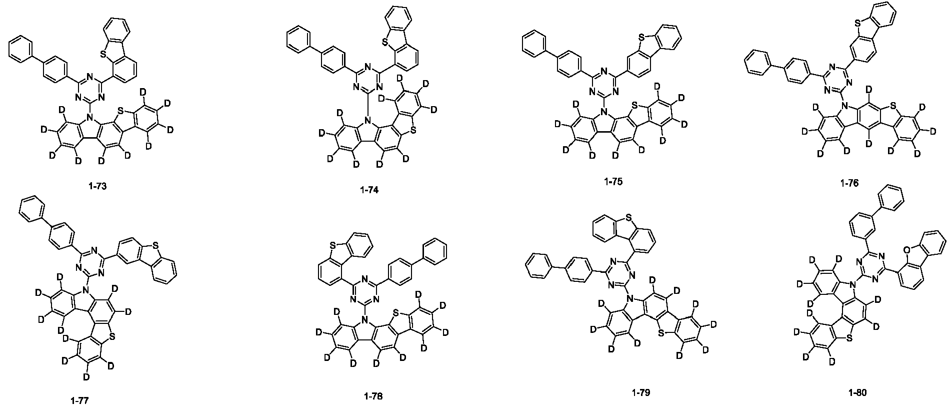

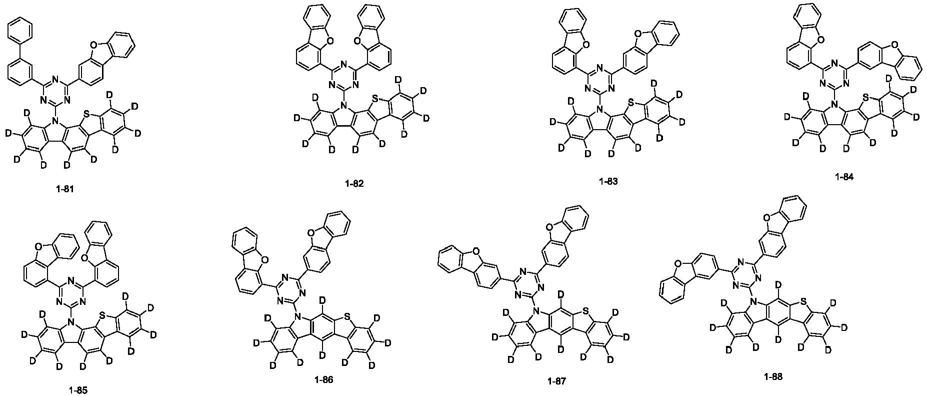

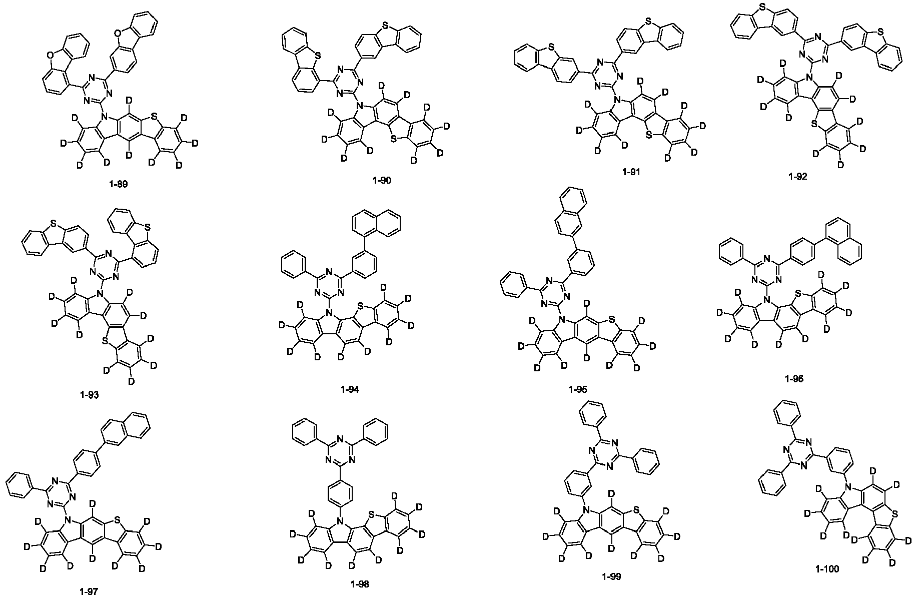

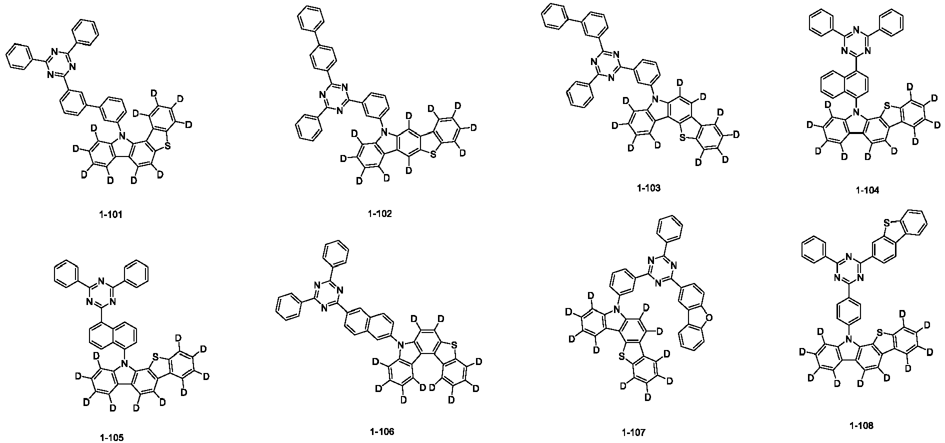

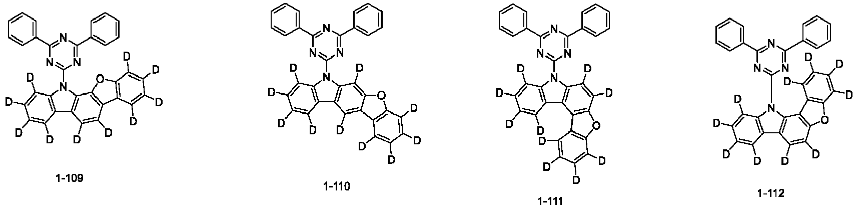

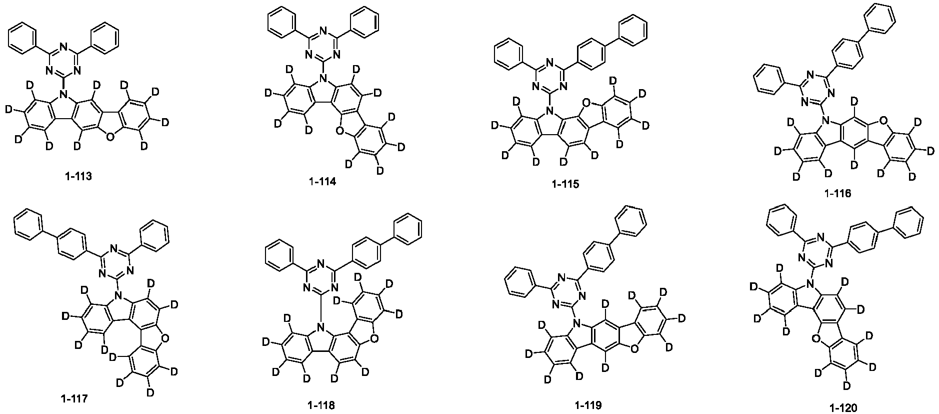

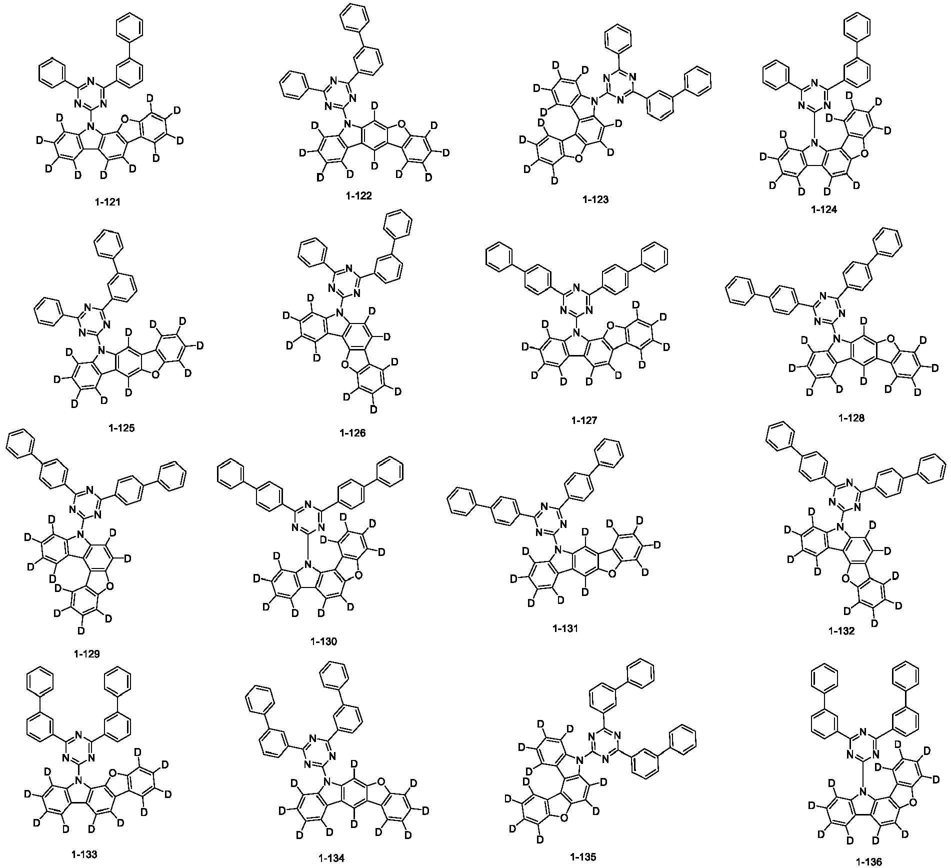

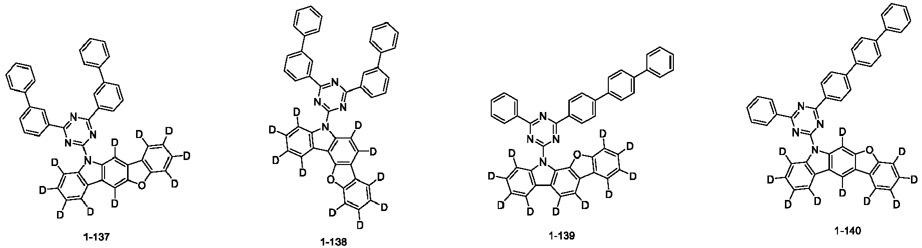

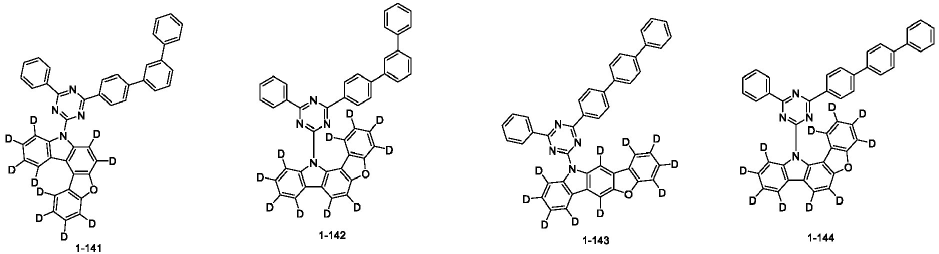

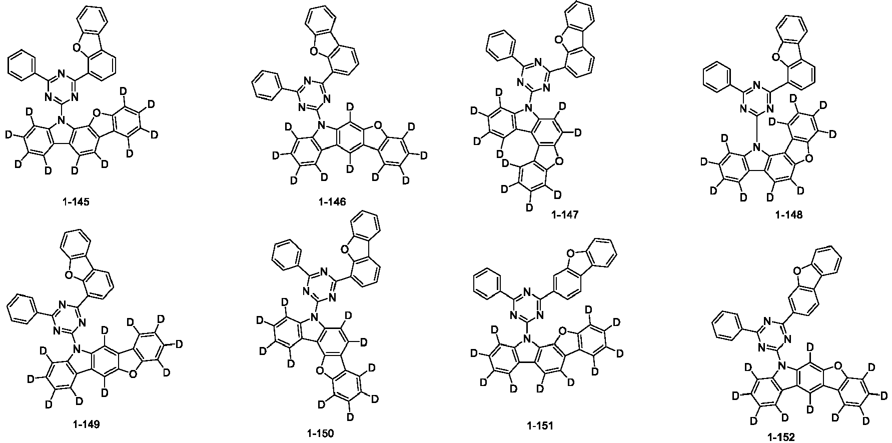

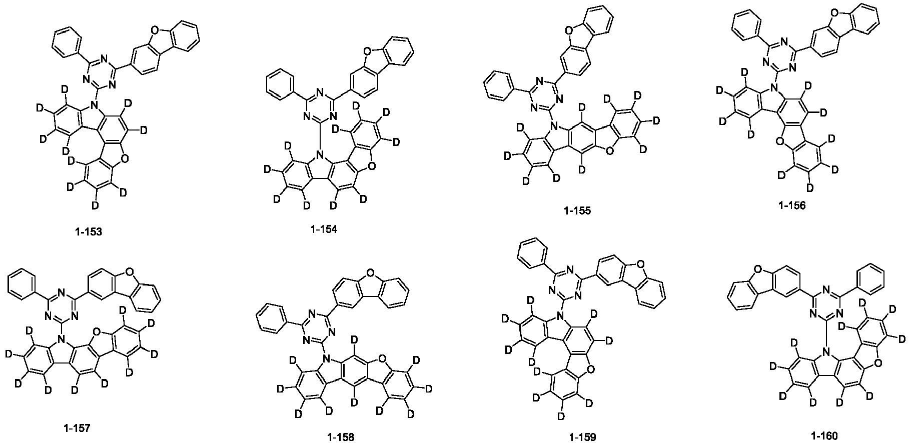

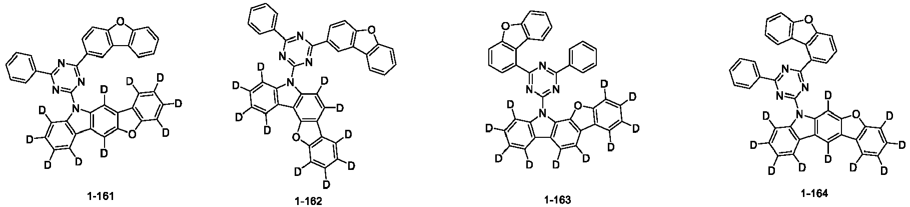

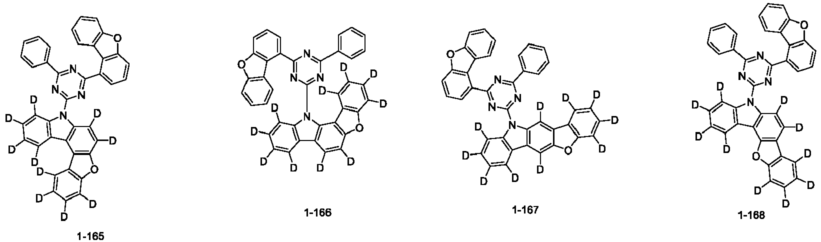

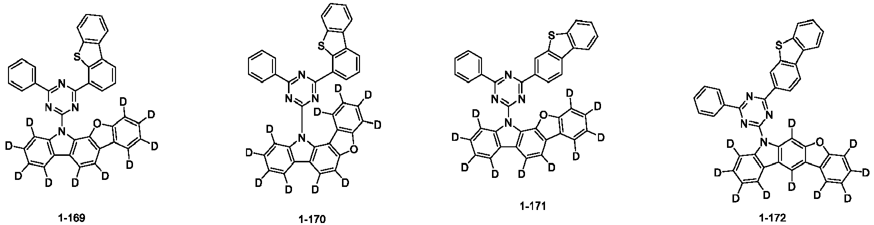

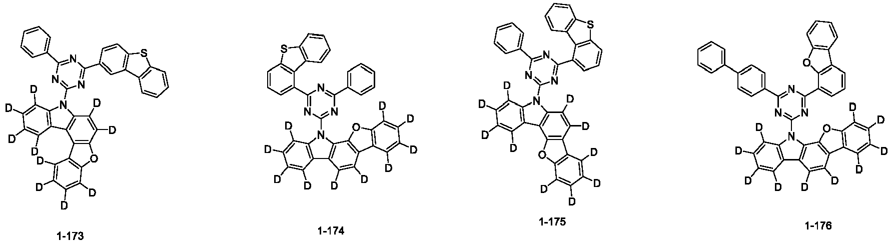

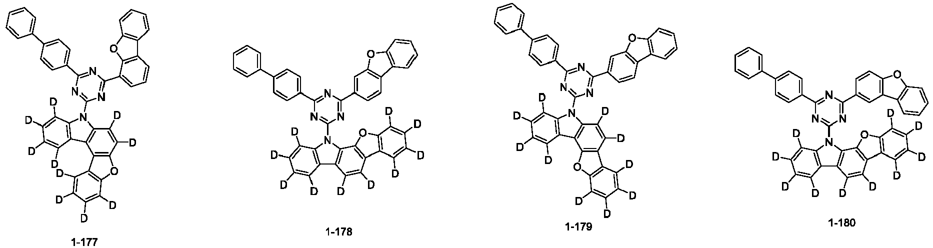

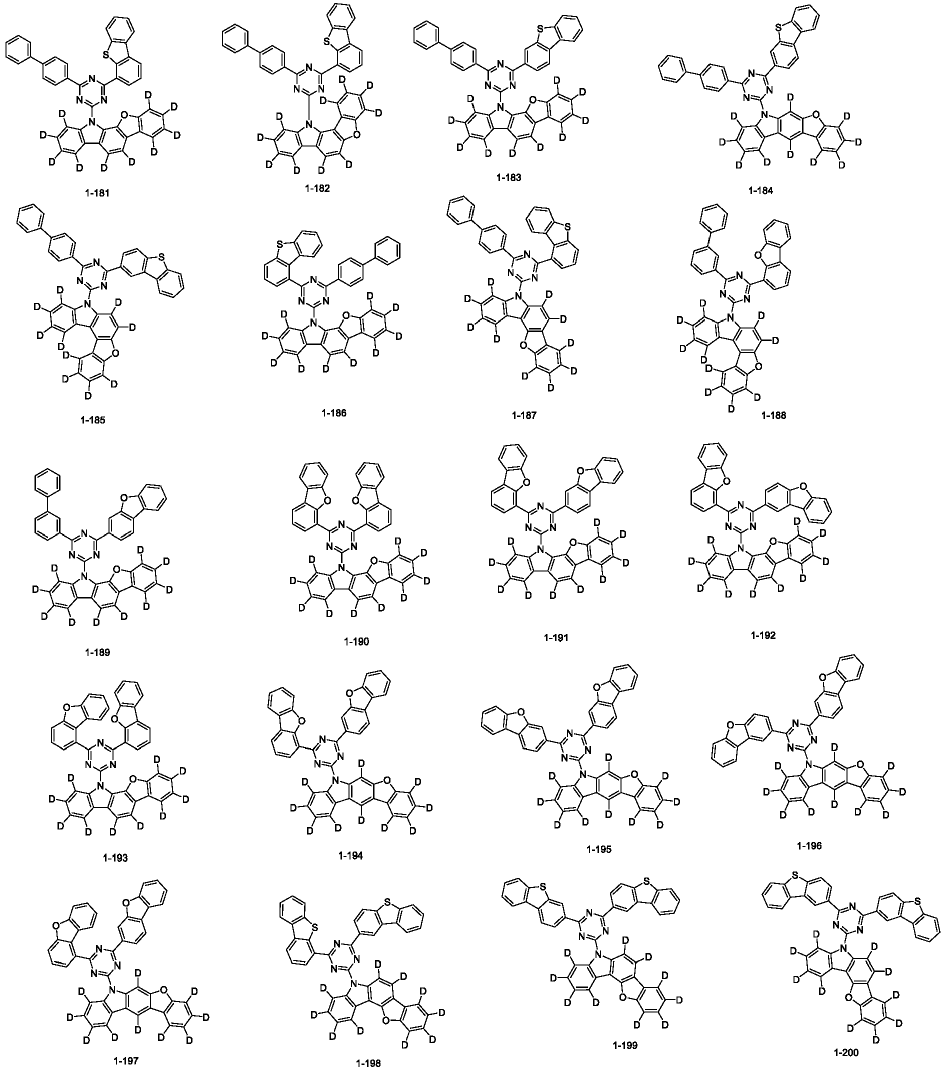

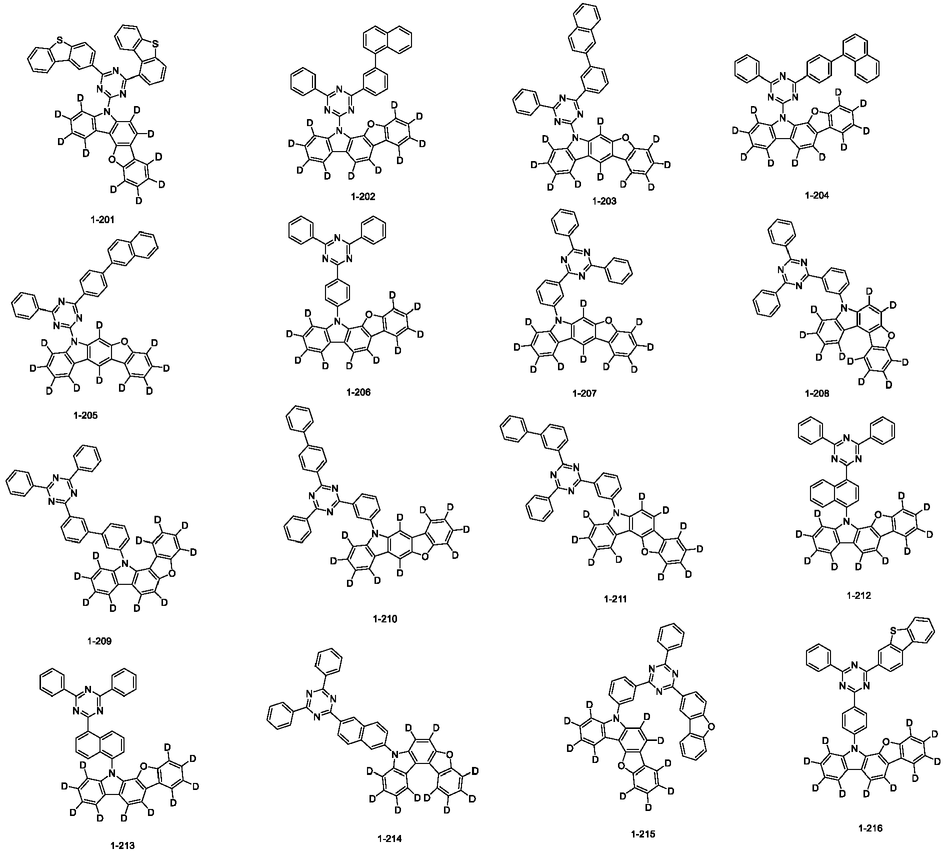

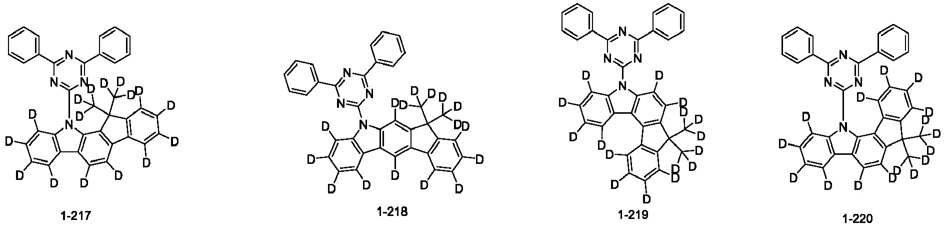

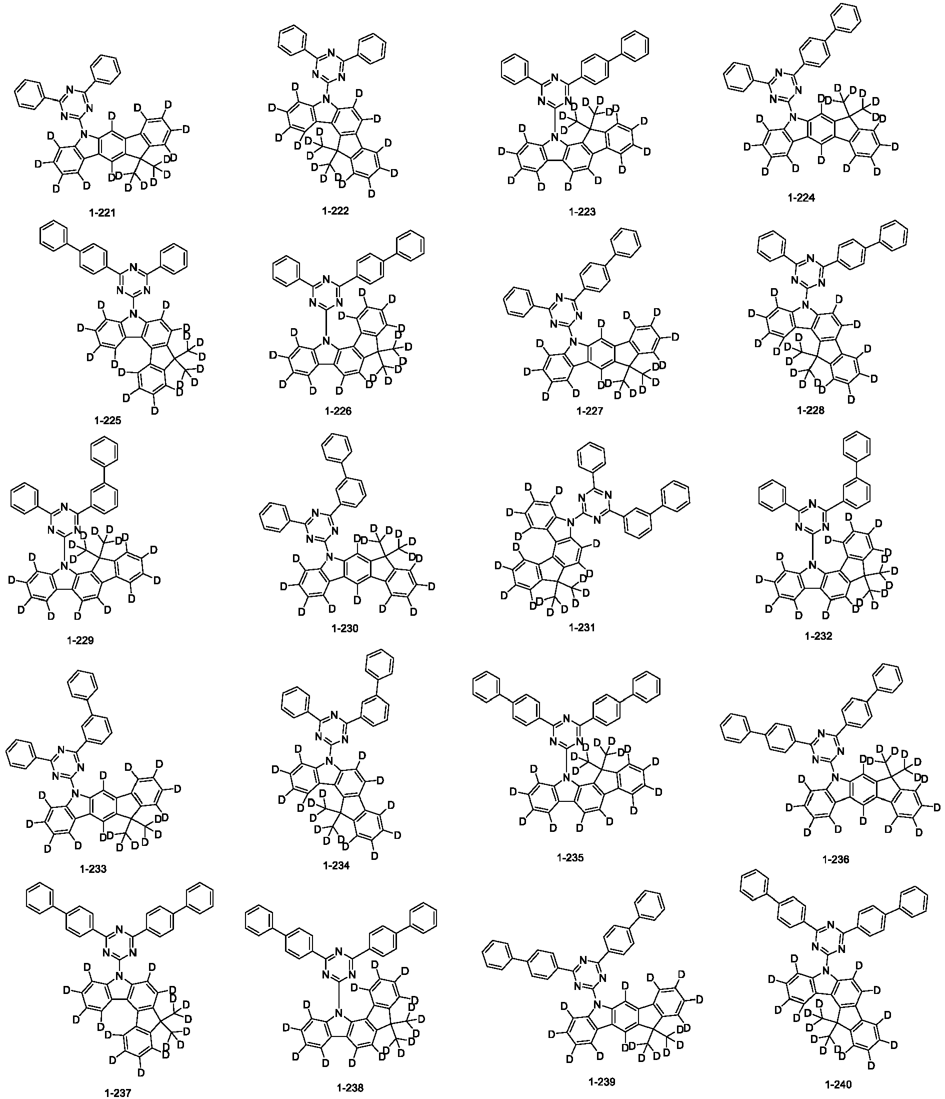

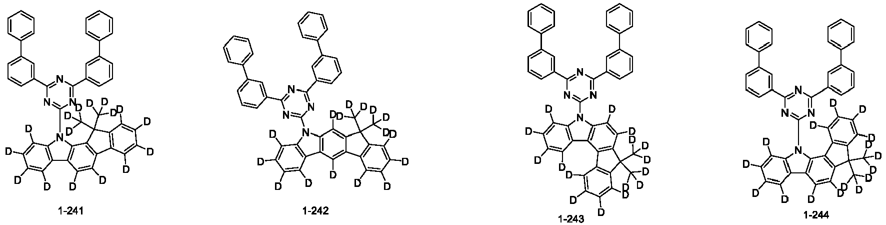

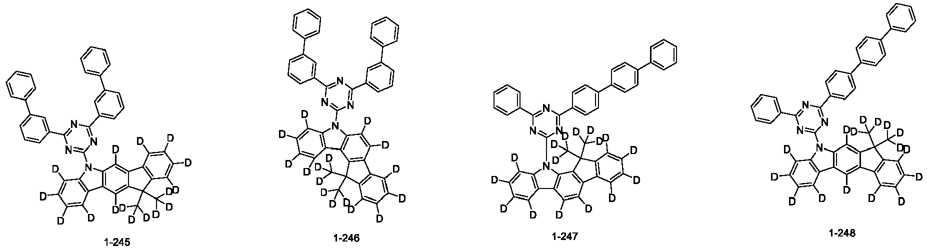

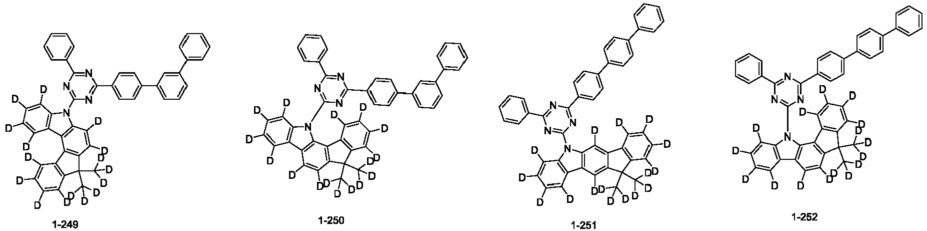

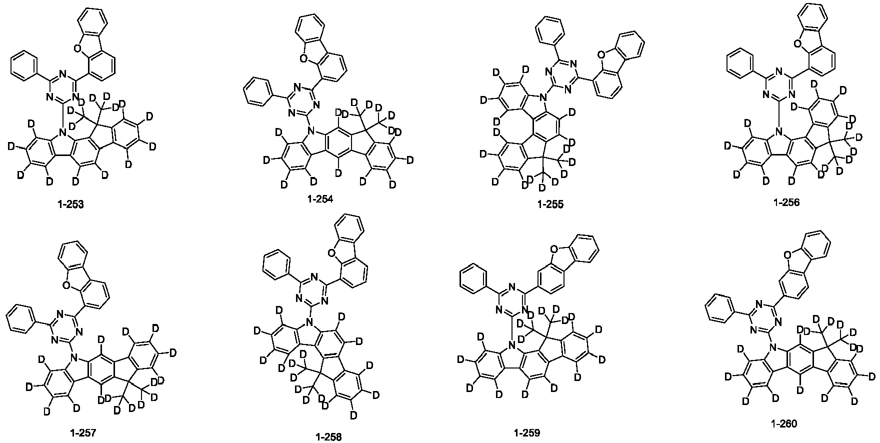

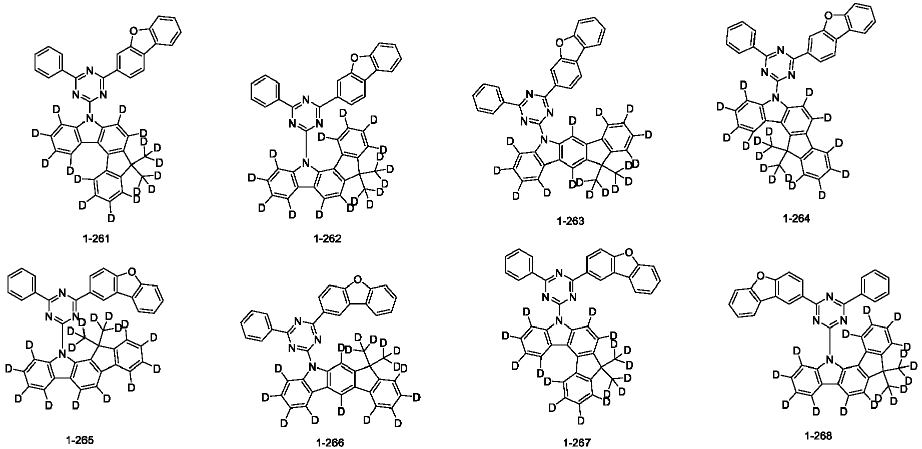

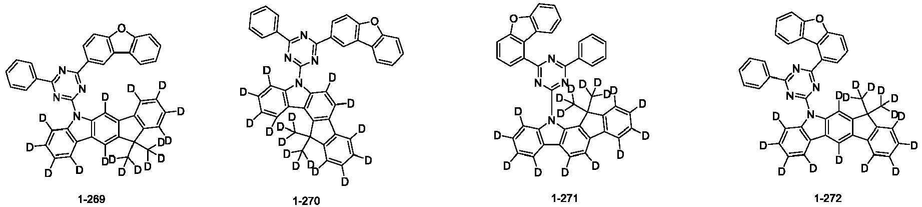

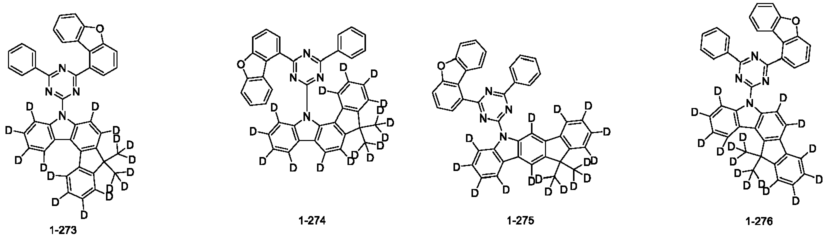

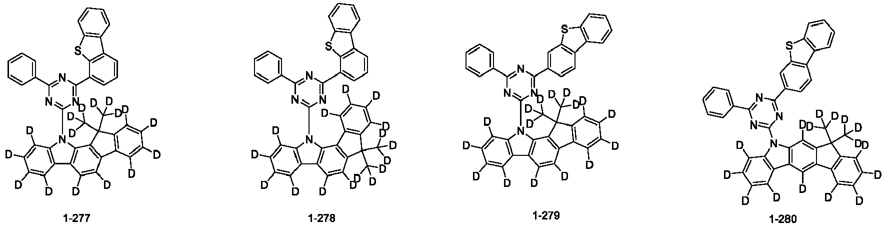

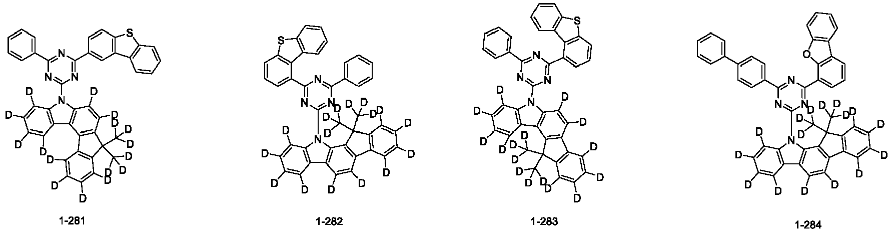

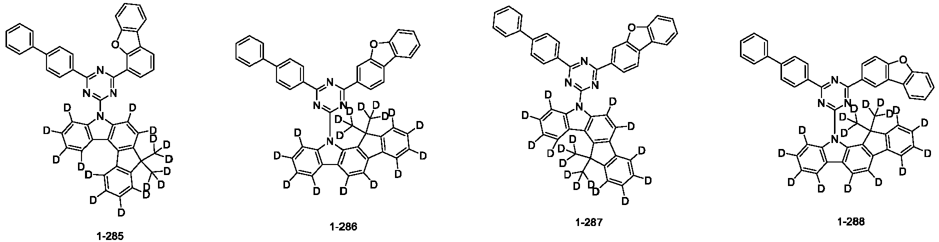

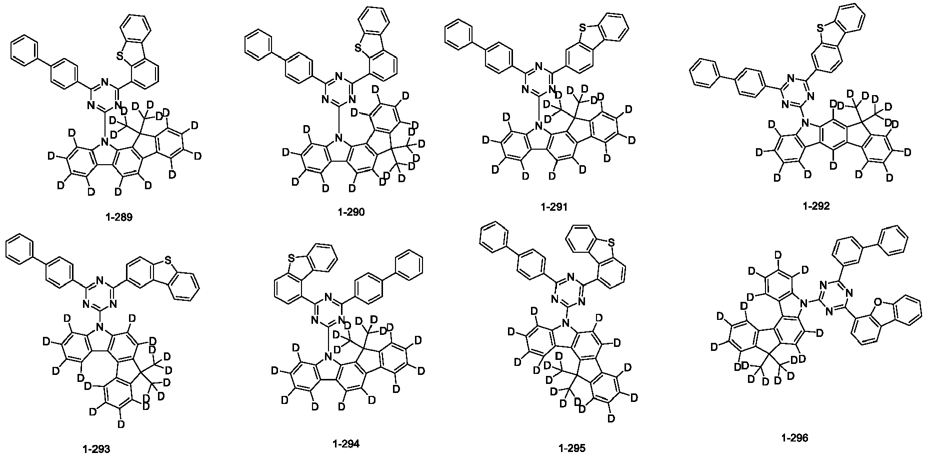

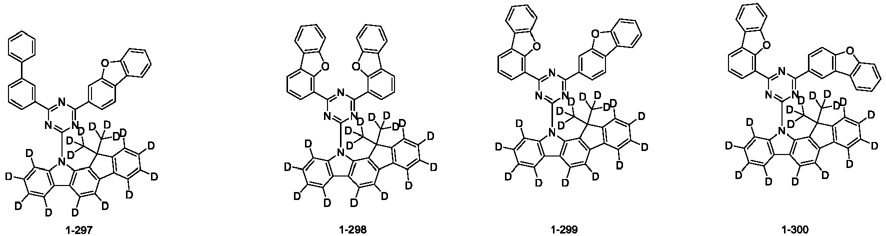

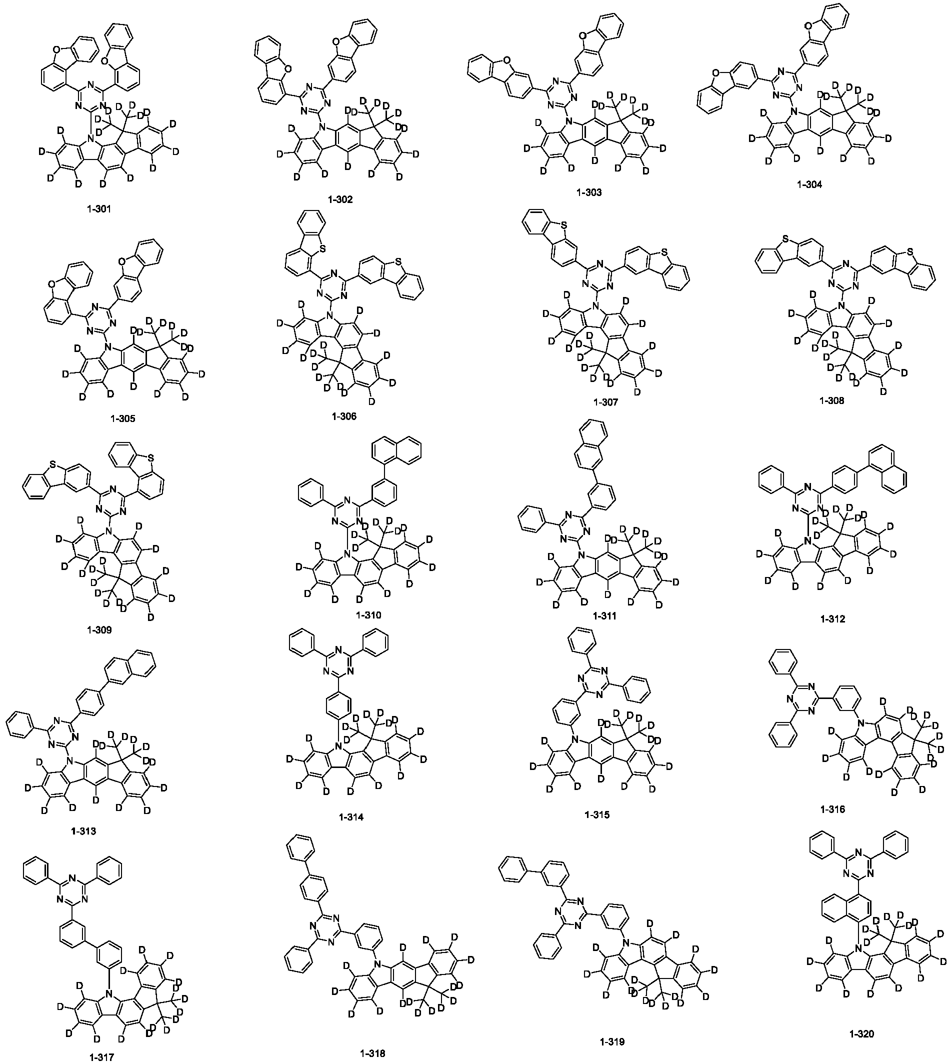

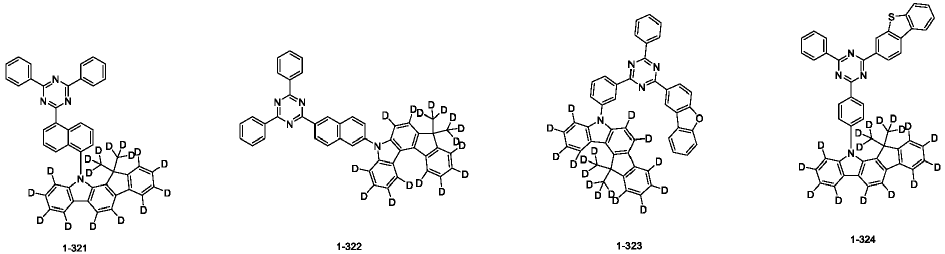

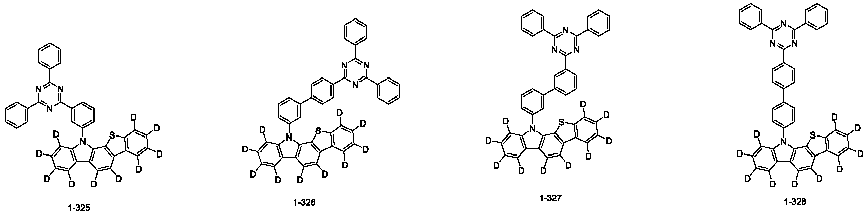

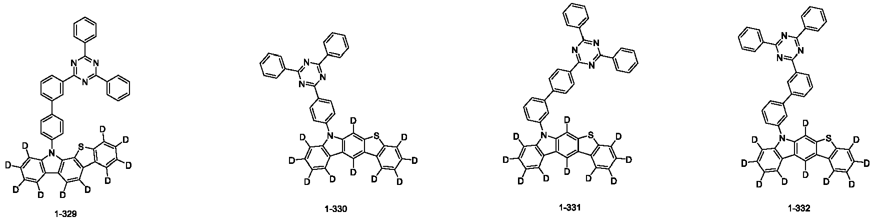

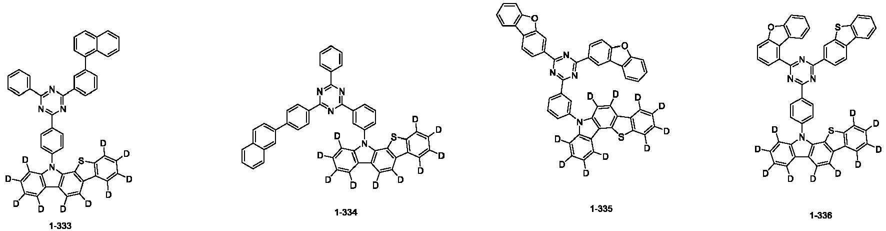

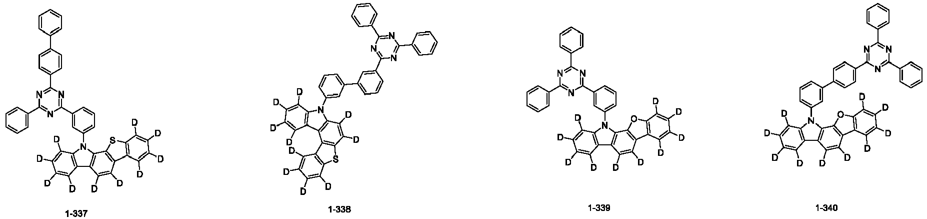

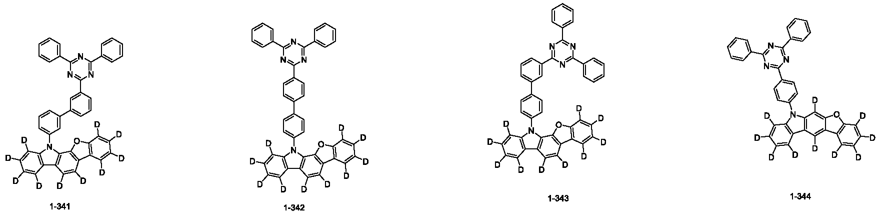

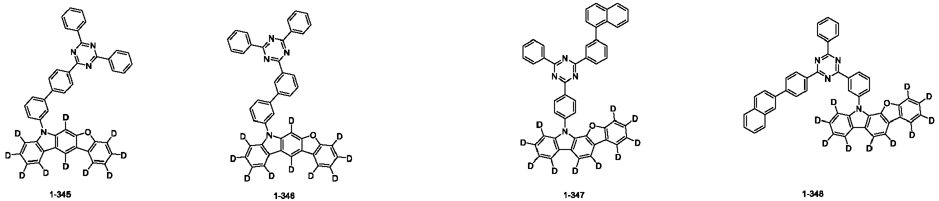

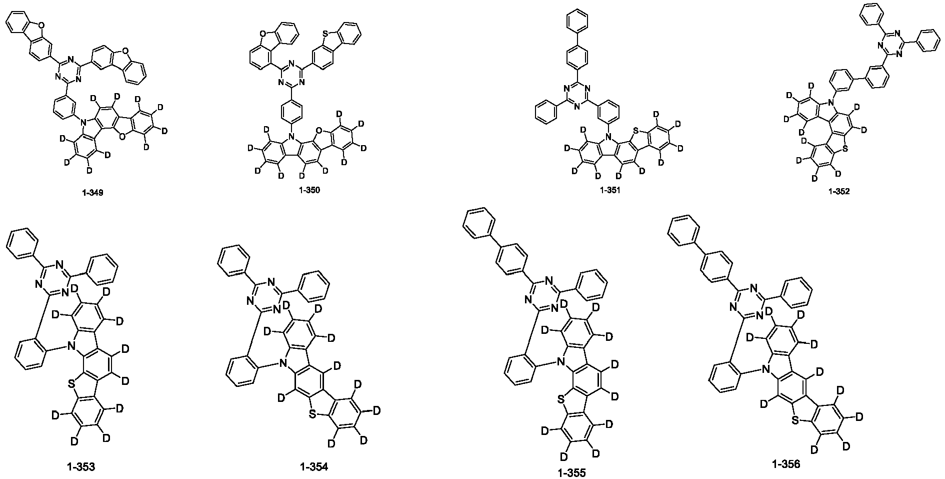

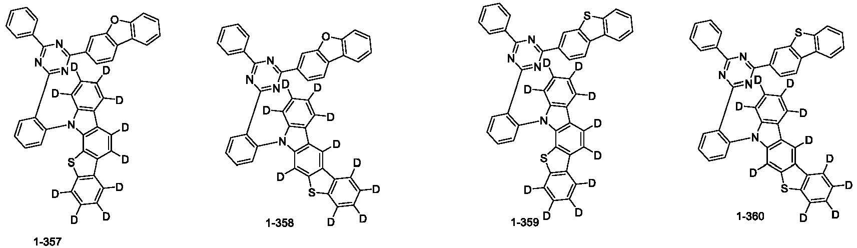

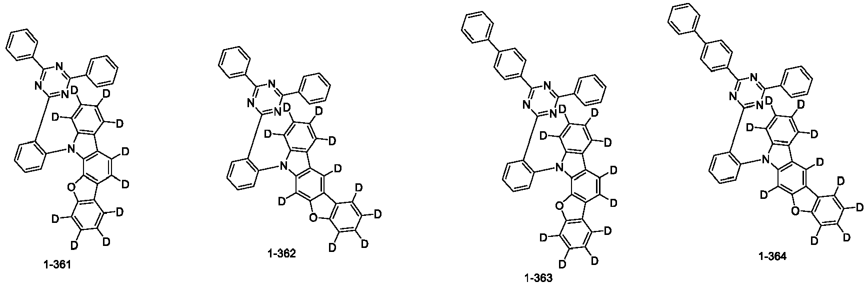

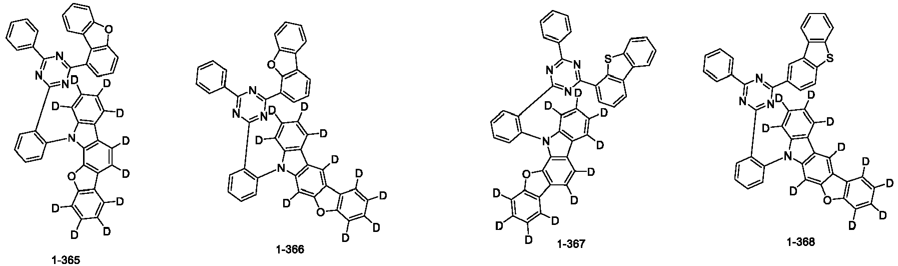

In another exemplary embodiment of the present specification, a first electrode; a second electrode; and an organic material layer having one or more layers provided between the first electrode and the second electrode are included, and one or more layers of the organic material layer include the heterocyclic compound of Chemical Formula 1.

In an exemplary embodiment of the present specification, the organic material layer including the heterocyclic compound may further include a compound of the following Chemical Formula 2. 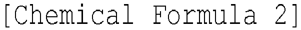 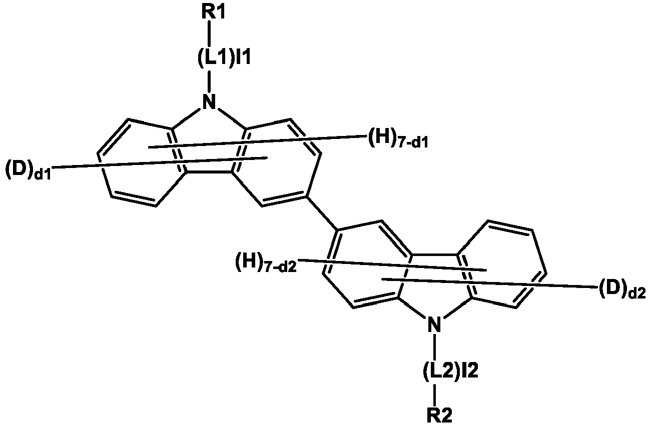 in Chemical Formula 2,
L1 and L2 are each independently a direct bond; or a substituted or unsubstituted arylene group having 6 to 60 carbon atoms,
l1 and l2 are each an integer from 1 to 3, and when l1 and l2 are each 2 or higher, substituents in the parenthesis are the same as or different from each other,
R1 and R2 are each independently hydrogen; deuterium; a halogen group; a cyano group; a substituted or unsubstituted alkyl group having 1 to 60 carbon atoms; a substituted or unsubstituted cycloalkyl group having 3 to 60 carbon atoms; a substituted or unsubstituted heterocycloalkyl group having 2 to 60 carbon atoms; a substituted or unsubstituted aryl group having 6 to 60 carbon atoms; or a substituted or unsubstituted heteroaryl group having 2 to 60 carbon atoms,
H is hydrogen and D is deuterium, and
d1 and d2 are each independently an integer from 0 to 7.

In the case of an organic light emitting device simultaneously including the heterocyclic compound of Chemical Formula 1 and the compound of Chemical Formula 2, better efficiency and service life effects are exhibited. From this result, it can be expected that an exciplex phenomenon will occur when two compounds are simultaneously included.

The exciplex phenomenon is a phenomenon in which energy with a magnitude of the HOMO level of a donor (p-host) and the LUMO level of an acceptor (n-host) is released as an electron exchange between two molecules. When the exciplex phenomenon between two molecules occurs, a reverse intersystem crossing (RISC) occurs, and the internal quantum efficiency of fluorescence can be increased to 100% due to the RISC. When a donor with a good hole transport capacity (p-host) and an acceptor with a good electron transport capacity (n-host) are used as hosts for the light emitting layer, holes are injected into the p-host and electrons are injected into the n-host, so that the driving voltage can be lowered, which can help to improve the service life. In the present invention, the compound of Chemical Formula 2 serves as a donor and the compound of Chemical Formula 1 serves as an acceptor, so that it could be confirmed that when the compounds were used as a host of a light emitting layer, excellent device characteristics were exhibited.

In an exemplary embodiment of the present specification, L1 and L2 are each independently a direct bond; or a substituted or unsubstituted C6 to C30 arylene group.

In an exemplary embodiment of the present specification, L1 and L2 are each independently a direct bond; or a C6 to C30 arylene group which is unsubstituted or substituted with deuterium.

In an exemplary embodiment of the present specification, L1 and L2 are each independently a direct bond; or a C6 to C12 arylene group which is unsubstituted or substituted with deuterium.

In an exemplary embodiment of the present specification, L1 and L2 are each independently a direct bond; a phenylene group which is unsubstituted or substituted with deuterium; or a biphenylene group which is unsubstituted or substituted with deuterium.

In an exemplary embodiment of the present specification, R1 and R2 are each independently a substituted or unsubstituted C1 to C60 alkyl group; a substituted or unsubstituted C3 to C60 cycloalkyl group; a substituted or unsubstituted C2 to C60 heterocycloalkyl group; a substituted or unsubstituted C6 to C60 aryl group; or a substituted or unsubstituted C2 to C60 heteroaryl group.

In an exemplary embodiment of the present specification, R1 and R2 are each independently a substituted or unsubstituted C1 to C30 alkyl group; a substituted or unsubstituted C3 to C30 cycloalkyl group; a substituted or unsubstituted C2 to C30 heterocycloalkyl group; a substituted or unsubstituted C6 to C30 aryl group; or a substituted or unsubstituted C2 to C30 heteroaryl group.

In an exemplary embodiment of the present specification, R1 and R2 are each independently a substituted or unsubstituted C1 to C30 alkyl group; a substituted or unsubstituted C3 to C30 cycloalkyl group; a substituted or unsubstituted C6 to C30 aryl group; or a substituted or unsubstituted C2 to C30 heteroaryl group.

In an exemplary embodiment of the present specification, R1 and R2 are each independently a substituted or unsubstituted C6 to C30 aryl group; or a substituted or unsubstituted C2 to C30 heteroaryl group.

In an exemplary embodiment of the present specification, R1 and R2 are each independently a substituted or unsubstituted C6 to C30 aryl group; or a C2 to C30 heteroaryl group which is substituted or unsubstituted and includes O or S.

In an exemplary embodiment of the present specification, R1 and R2 are each independently a substituted or unsubstituted phenyl group; a substituted or unsubstituted biphenyl group; a substituted or unsubstituted terphenyl group; a substituted or unsubstituted triphenylene group; a substituted or unsubstituted fluorene group; a substituted or unsubstituted dibenzofuran group; or a substituted or unsubstituted dibenzothiophene group.

In an exemplary embodiment of the present specification, R1 and R2 are each independently a phenyl group which is unsubstituted or substituted with deuterium; a biphenyl group which is unsubstituted or substituted with deuterium; a terphenyl group which is unsubstituted or substituted with deuterium; a triphenylene group which is unsubstituted or substituted with deuterium; a fluorene group which is unsubstituted or substituted with one or more substituents selected among deuterium and an alkyl group; a dibenzofuran group which is unsubstituted or substituted with deuterium; or a dibenzothiophene group which is unsubstituted or substituted with deuterium.

In an exemplary embodiment of the present specification, R1 and R2 are each independently a C6 to C30 aryl group which is unsubstituted or substituted with one or more substituents selected among deuterium and an alkyl group; or a C2 to C30 heteroaryl group which is unsubstituted or substituted with deuterium.

In an exemplary embodiment of the present specification, d1 and d2 may be each independently 0 or 7.

In an exemplary embodiment of the present specification, Chemical Formula 2 may be represented by the following Chemical Formula 2-1 or 2-2.

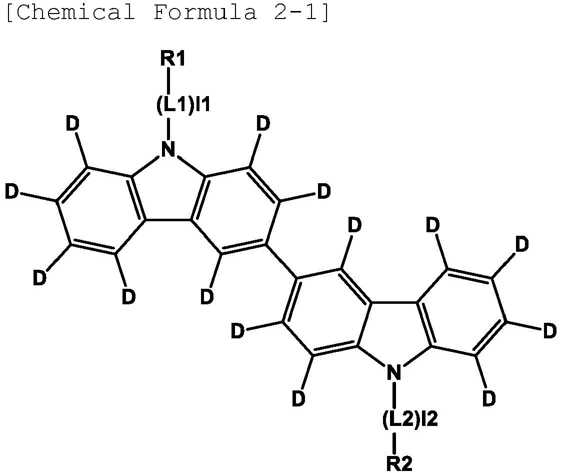

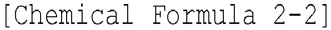

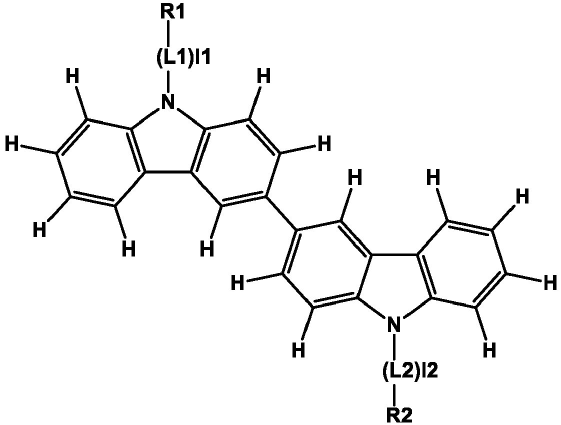

In Chemical Formulae 2-1 and 2-2, the definition of each substituent is the same as the definition in Chemical Formula 2.

In an exemplary embodiment of the present specification, the deuterium content of Chemical Formula 2 is 0% to 100%.

In an exemplary embodiment of the present specification, the deuterium content of Chemical Formula 2 is 0% or 10% to 100%.

In an exemplary embodiment of the present specification, the deuterium content of Chemical Formula 2 is 0% or 30% to 100%.

In an exemplary embodiment of the present specification, the deuterium content of Chemical Formula 2 may be 0%.

In an exemplary embodiment of the present specification, the deuterium content of Chemical Formula 2 may be more than 0% and 100% or less.

In an exemplary embodiment of the present specification, the deuterium content of Chemical Formula 2 may be 10% to 100%.

An organic light emitting device according to an exemplary embodiment of the present specification may include a heterocyclic compound of Chemical Formula 1 with a deuterium content of more than 0% and a compound of Chemical Formula 2 with a deuterium content of 0% in the organic material layer.

An organic light emitting device according to another exemplary embodiment may include a heterocyclic compound of Chemical Formula 1 with a deuterium content of more than 0% and a compound of Chemical Formula 2 with a deuterium content of more than 0% in the organic material layer.

In an exemplary embodiment of the present specification, when Chemical Formula 2 includes deuterium, there are effects of lowering the driving voltage and increasing the light emitting efficiency and service life compared to the case where Chemical Formula 2 does not include deuterium.

Likewise, in the case of the compound of Chemical Formula 2, in an electron transport moiety and a hole transport moiety, which directly exchange electrons, the vibrational frequency of interatomic bond in molecules changes continuously as electrons move, affecting the stability of interatomic bond in molecules and the stability of the molecular structure. By substituting the compound with deuterium, which has a higher molecular weight than hydrogen, the energy of the molecule is lowered by reducing the change of the vibrational frequency, and thus the stability of molecules is enhanced.

In addition, since the single bond dissociation energy of carbon and deuterium is higher than the single bond dissociation energy of carbon and hydrogen, the thermal stability of molecule is increased, so that there is an effect of improving the service life of the device.

In an exemplary embodiment of the present specification, Chemical Formula 2 may be represented by any one of the following compounds.

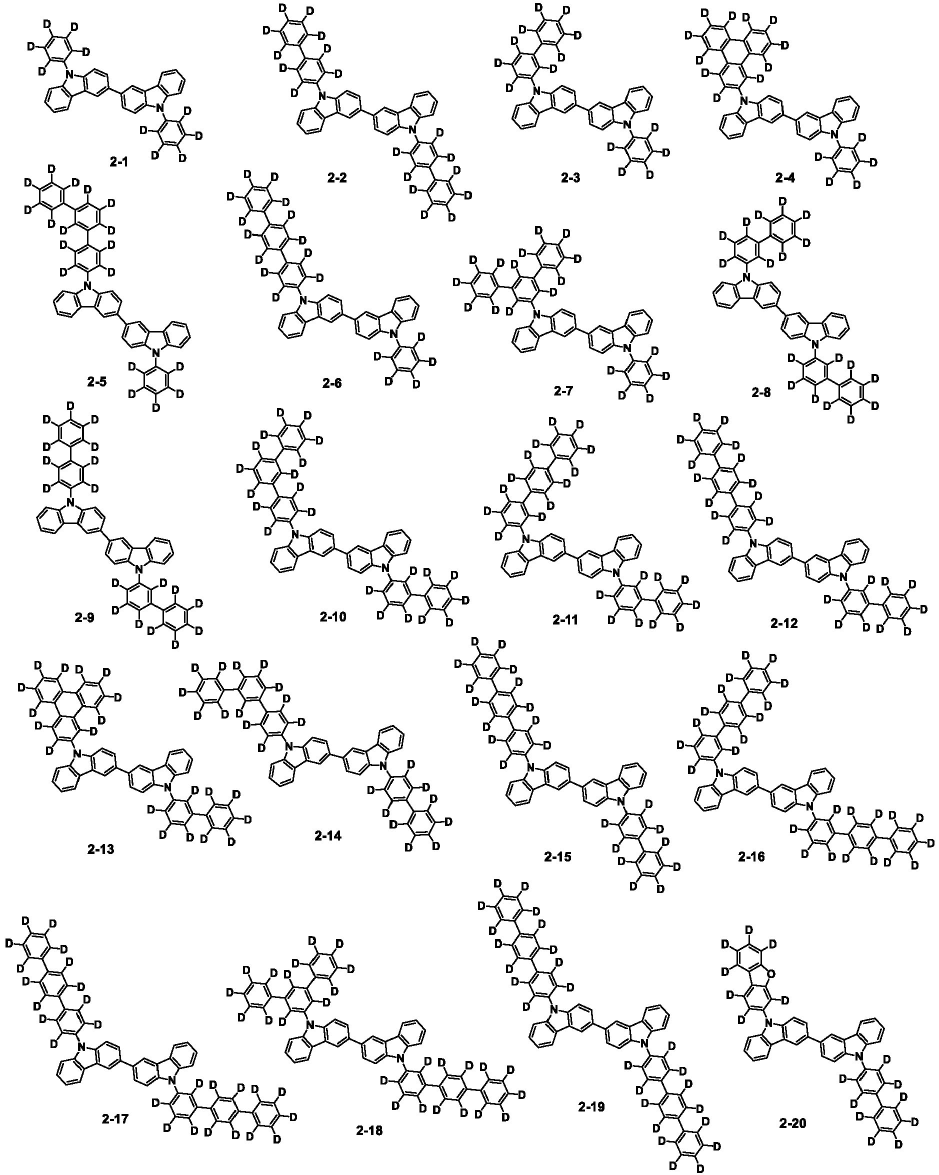

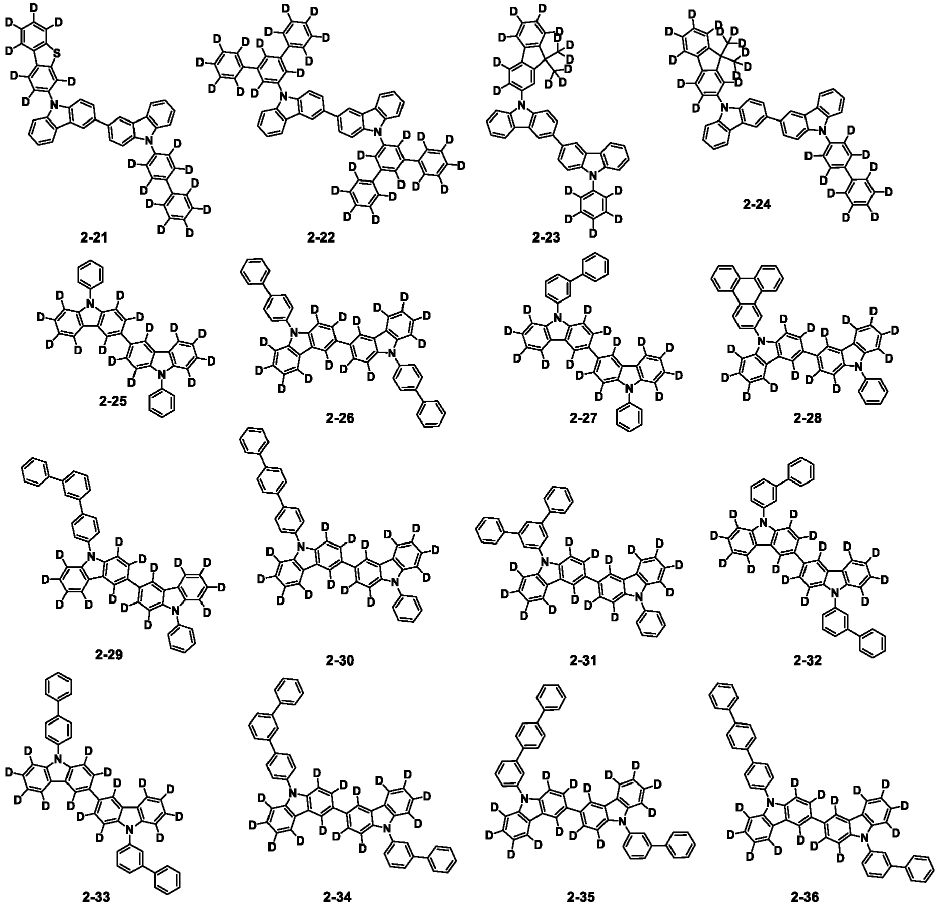

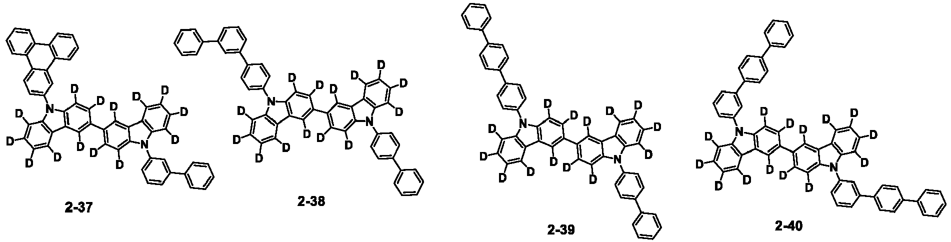

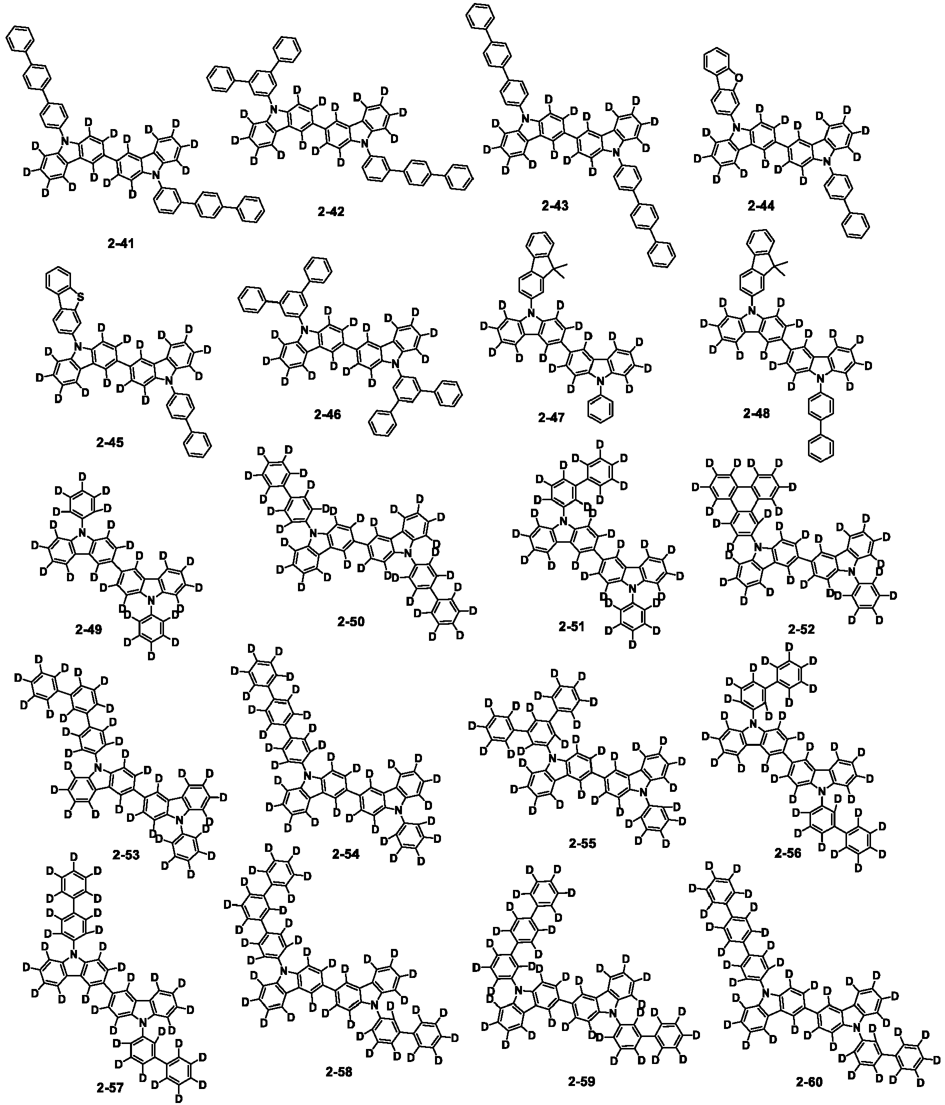

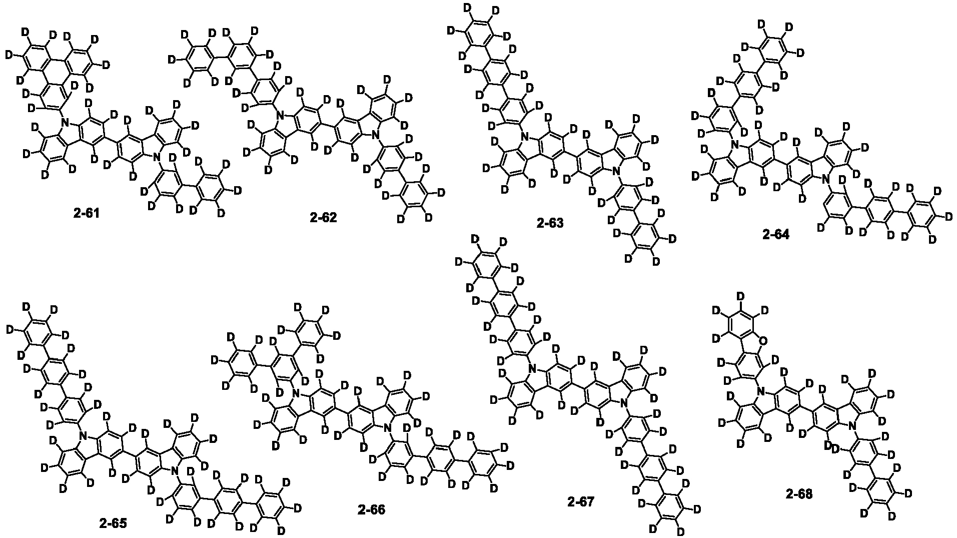

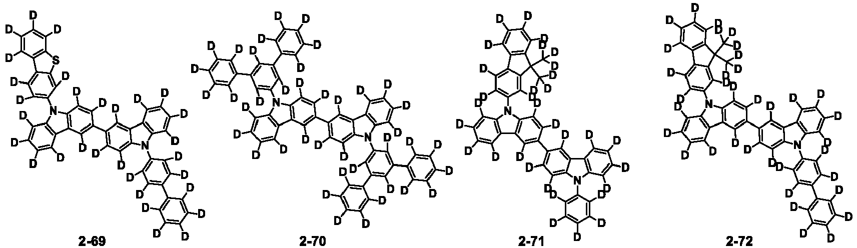

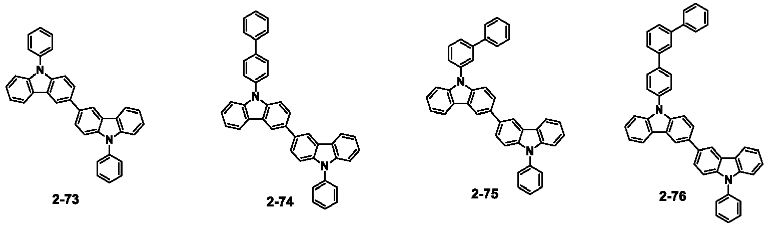

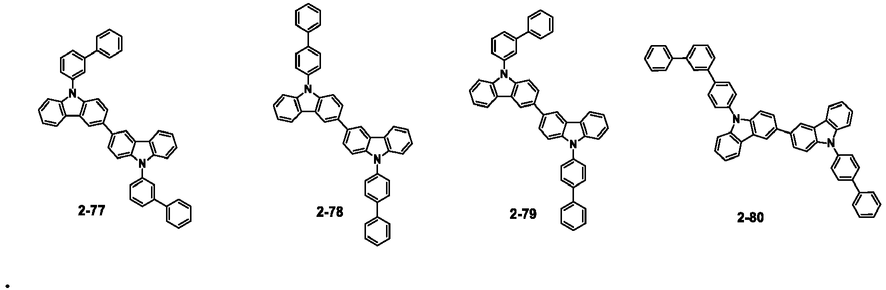

In an exemplary embodiment of the present specification, the organic material layer may include the heterocyclic compound of Chemical Formula 1 and the compound of Chemical Formula 2 at a weight ratio of 1:10 to 10:1, preferably at a weight ratio of 1:8 to 8:1, 1:5 to 5:1 and 1:3 to 3:1.

In an exemplary embodiment of the present specification, the organic material layer may include the heterocyclic compound of Chemical Formula 1 and the compound of Chemical Formula 2 at a weight ratio of 1:1 to 1:3.

In an exemplary embodiment of the present specification, the organic material layer may further include a phosphorescent dopant.

In an exemplary embodiment of the present specification, the phosphorescent dopant may be a green, blue or red phosphorescent dopant.

In an exemplary embodiment of the present specification, the phosphorescent dopant may be a green phosphorescent dopant.

In another exemplary embodiment, the phosphorescent dopant may be a red phosphorescent dopant.

In an exemplary embodiment of the present specification, the phosphorescent dopant may be an iridium-based dopant.

As the phosphorescent dopant material, those known in the art may be used. For example, phosphorescent dopant materials represented by LL'MX', LL'L"M, LMX'X", L₂MX' and L₃M may be used, but the scope of the present invention is not limited by these examples.

Here, L, L', L", X' and X" are bidentate ligands different from each other, and M is a metal forming an octahedral complex.

M may be iridium, platinum, osmium, and the like.

L, L' and L" are anionic bidentate ligands coordinated to M with the iridium-based dopant by sp2 carbon and heteroatoms, and non-limiting examples of L, L' and L" include 1-phenylisoquinoline, 2-(1-naphthyl)benzoxazole, 2-phenylbenzoxazole, 2-phenylbenzothiazole, 2-phenylbenzothiazole, 7,8-benzoquinoline, theiophene group pyrizine, phenylpyridine, benzothiophene group pyrizine, 3-methoxy-2-phenylpyridine, thiophene group pyrizine, tolylpyridine, and the like.

X' and X" may function to trap electrons or holes, and non-limiting examples of X' and X" include acetylacetonate (acac), hexafluoroacetylacetonate, salicylidene, picolinate, 8-hydroxyquinolinate and the like.

In an exemplary embodiment of the present specification, as the iridium-based dopant, Ir(ppy)₃ as a green phosphorescent dopant may be used.

In an exemplary embodiment of the present specification, as the iridium-based dopant, Ir(piq)2(acac) as a red phosphorescent dopant may be used.

In an exemplary embodiment of the present specification, the content of the dopant may be 1% to 15%, preferably 1% to 10% based on the entire light emitting layer.

The organic light emitting device according to an exemplary embodiment of the present specification may be manufactured by typical methods and materials for manufacturing an organic light emitting device, except that an organic material layer having one or more layers is formed by using the heterocyclic compound of the above-described Chemical Formula 1 alone or using the heterocyclic compound of Chemical Formula 1 together with the compound of Chemical Formula 2.

The heterocyclic compound of Chemical Formula 1 and the compound of Chemical Formula 2 may be formed as an organic material layer by not only a vacuum deposition method, but also a solution application method when an organic light emitting device is manufactured. Here, the solution application method means spin coating, dip coating, inkjet printing, screen printing, a spray method, roll coating, and the like, but is not limited thereto.

The organic material layer of the organic light emitting device of the present invention may be composed of a single-layered structure, but may be composed of a multilayered structure in which two or more organic material layers are stacked. For example, the organic light emitting device of the present invention may have a structure including a hole injection layer, a hole transport layer, a light emitting layer, an electron transport layer, an electron injection layer, and the like as organic material layers. However, the structure of the organic light emitting device is not limited thereto, and may include a fewer number of organic material layers.

In an exemplary embodiment of the present specification, the first electrode may be a positive electrode, and the second electrode may be a negative electrode.

In another exemplary embodiment of the present specification, the first electrode may be a negative electrode, and the second electrode may be a positive electrode.

In the organic light emitting device of the present invention, the organic material layer includes a light emitting layer, and the light emitting layer may include the heterocyclic compound of Chemical Formula 1.

In the organic light emitting device of the present specification, the organic material layer includes a light emitting layer, and the light emitting layer may include the heterocyclic compound of Chemical Formula 1 and the compound of Chemical Formula 2.

In the organic light emitting device of the present specification, the organic material layer includes a light emitting layer, the light emitting layer includes a host, and the host may include the heterocyclic compound of Chemical Formula 1.

In the organic light emitting device of the present specification, the heterocyclic compound of Chemical Formula 1 may be used as a green host or a red host.

In the organic light emitting device of the present specification, the organic material layer includes a light emitting layer, the light emitting layer includes a host, and the host may include the heterocyclic compound of Chemical Formula 1 and the compound of Chemical Formula 2.

In the organic light emitting device of the present specification, the compound of Chemical Formula 2 may be used as a green host or a red host.

When the heterocyclic compound of Chemical Formula 1 and the compound of Chemical Formula 2 are combined to form a host, better efficiency and service life are exhibited than when one host is used.

In an exemplary embodiment of the present specification, the heterocyclic compound of Chemical Formula 1 may be used as an N-type host material, and the compound of Chemical Formula 2 may be used as a P-type host material.

In an exemplary embodiment of the present specification, the organic light emitting device may be a blue organic light emitting device, and the heterocyclic compound of Chemical Formula 1 may be used as a material for the blue organic light emitting device. For example, the heterocyclic compound of Chemical Formula 1 may be included in a host material of a light emitting layer of a blue organic light emitting device.

In another exemplary embodiment of the present specification, the organic light emitting device may be a green organic light emitting device, and the heterocyclic compound of Chemical Formula 1 may be used as a material for the green organic light emitting device. For example, the heterocyclic compound of Chemical Formula 1 may be included in a host material of a light emitting layer of a green organic light emitting device.

In still another exemplary embodiment of the present specification, the organic light emitting device may be a red organic light emitting device, and the heterocyclic compound of Chemical Formula 1 may be used as a material for the red organic light emitting device. For example, the heterocyclic compound of Chemical Formula 1 may be included in a host material of a light emitting layer of a red organic light emitting device.

The organic light emitting device of the present invention may further include one or more layers selected from the group consisting of a light emitting layer, a hole injection layer, a hole transport layer, an electron injection layer, an electron transport layer, an electron blocking layer, and a hole blocking layer.

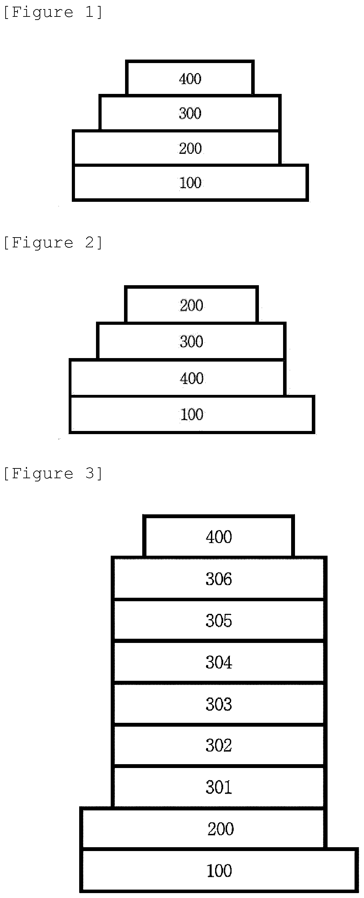

FIGS. 1 to 3 exemplify the stacking sequence of the electrodes and the organic material layer of the organic light emitting device according to an exemplary embodiment of the present specification. However, the scope of the present application is not intended to be limited by these drawings, and the structure of the organic light emitting device known in the art may also be applied to the present application.

According to FIG. 1, an organic light emitting device in which a positive electrode 200, an organic material layer 300, and a negative electrode 400 are sequentially stacked on a substrate 100 is illustrated. However, the organic light emitting device is not limited only to such a structure, and as in FIG. 2, an organic light emitting device in which a negative electrode, an organic material layer, and a positive electrode are sequentially stacked on a substrate may also be implemented.

FIG. 3 exemplifies a case where an organic material layer is a multilayer. An organic light emitting device according to FIG. 3 includes a hole injection layer 301, a hole transport layer 302, a light emitting layer 303, a hole blocking layer 304, an electron transport layer 305, and an electron injection layer 306. However, the scope of the present application is not limited by the stacking structure as described above, and if necessary, the other layers except for the light emitting layer may be omitted, and another necessary functional layer may be further added.

An organic material layer including the heterocyclic compound of Chemical Formula 1 may additionally include other materials, if necessary.

In the organic light emitting device according to an exemplary embodiment of the present specification, materials other than the heterocyclic compound of Chemical Formula 1 and the compound of Chemical Formula 2 will be exemplified below, but these materials are provided only for exemplification and are not for limiting the scope of the present application, and may be replaced with materials publicly known in the art.

As a positive electrode material, materials having a relatively high work function may be used, and a transparent conductive oxide, a metal or a conductive polymer, and the like may be used. Specific examples of the positive electrode material include: a metal such as vanadium, chromium, copper, zinc, and gold, or an alloy thereof; a metal oxide such as zinc oxide, indium oxide, indium tin oxide (ITO), and indium zinc oxide (IZO); a combination of a metal and an oxide, such as ZnO:Al or SnO₂:Sb; a conductive polymer such as poly(3-methylthiophene), poly[3,4-(ethylene-1,2-dioxy)thiophene] (PEDOT), polypyrrole, and polyaniline; and the like, but are not limited thereto.

As a negative electrode material, materials having a relatively low work function may be used, and a metal, a metal oxide, or a conductive polymer, and the like may be used. Specific examples of the negative electrode material include: a metal such as magnesium, calcium, sodium, potassium, titanium, indium, yttrium, lithium, gadolinium, aluminum, silver, tin, and lead, or an alloy thereof; a multi-layer structured material, such as LiF/Al or LiO₂/Al; and the like, but are not limited thereto.

As a hole injection material, a publicly-known hole injection material may also be used, and it is possible to use, for example, a phthalocyanine compound such as copper phthalocyanine disclosed in US Patent No. 4,356,429 or starburst-type amine derivatives described in the document [Advanced Material, 6, p. 677 (1994)], for example, tris(4-carbazoyl-9-ylphenyl)amine (TCTA), 4,4',4"-tri[phenyl(m-tolyl)amino]triphenylamine (m-MTDATA), 1,3,5-tris[4-(3-methylphenylphenylamino)phenyl]benzene (m-MTDAPB), polyaniline/dodecylbenzenesulfonic acid or poly(3,4-ethylenedioxythiophene)/poly(4-styrenesulfonate), which is a soluble conductive polymer, polyaniline/camphor sulfonic acid or polyaniline/poly(4-styrenesulfonate), and the like.

As a hole transporting material, a pyrazoline derivative, an arylamine-based derivative, a stilbene derivative, a triphenyldiamine derivative, and the like may be used, and a low-molecular weight or polymer material may also be used.

As an electron transporting material, it is possible to use an oxadiazole derivative, anthraquinodimethane and a derivative thereof, benzoquinone and a derivative thereof, naphthoquinone and a derivative thereof, anthraquinone and a derivative thereof, tetracyanoanthraquinodimethane and a derivative thereof, a fluorenone derivative, diphenyldicyanoethylene and a derivative thereof, a diphenoquinone derivative, a metal complex of 8-hydroxyquinoline and a derivative thereof, and the like, and a low-molecular weight material and a polymer material may also be used.

As an electron injection material, for example, LiF is representatively used in the art, but the present application is not limited thereto.

As a light emitting material, a red, green, or blue light emitting material may be used, and if necessary, two or more light emitting materials may be mixed and used. In this case, two or more light emitting materials are deposited or used as an individual supply source, or premixed to be deposited and used as one supply source. Further, a fluorescent material may also be used as the light emitting material, but may also be used as a phosphorescent material. As the light emitting material, it is also possible to use alone a material which emits light by combining holes and electrons each injected from a positive electrode and a negative electrode, but materials in which a host material and a dopant material are involved in light emission together may also be used.

When hosts of the light emitting material are mixed and used, the same series of hosts may also be mixed and used, and different series of hosts may also be mixed and used. For example, any two or more materials from N-type host materials or P-type host materials may be selected and used as a host material for a light emitting layer.

The organic light emitting device according to an exemplary embodiment of the present specification may be a top emission type, a bottom emission type, or a dual emission type according to the material to be used.

The compound according to an exemplary embodiment of the present specification may act even in organic electronic devices including organic solar cells, organic photoconductors, organic transistors, and the like, based on the principle similar to those applied to organic light emitting devices.

In addition, it is possible to finely adjust an energy band-gap by introducing various substituents into the structure of Chemical Formula 1, and meanwhile, it is possible to improve characteristics at the interface between organic materials and diversify the use of the material.

In another exemplary embodiment of the present specification, provided is a composition for an organic material layer of an organic light emitting device, including the heterocyclic compound of Chemical Formula 1.

Furthermore, another exemplary embodiment of the present specification provides a composition for an organic material layer of an organic light emitting device, which includes the heterocyclic compound of Chemical Formula 1 and the compound of Chemical Formula 2.

The specific contents on the heterocyclic compound of Chemical Formula 1 and the compound of Chemical Formula 2 are the same as those described above.

The weight ratio of the heterocyclic compound represented by Chemical Formula 1 and the compound of Chemical Formula 2 in the composition may be 1:10 to 10:1, 1:8 to 8:1, 1:5 to 5:1, and 1:3 to 3:1, but is not limited thereto.

In an exemplary embodiment of the present specification, the composition is in a form in which two or more compounds are simply mixed, materials in a powder state may also be mixed before an organic material layer of an organic light emitting device is formed, and it is possible to mix compounds in a liquid state at a temperature which is equal to or more than a suitable temperature. The composition is in a solid state at a temperature which is equal to or less than the melting point of each material, and may be maintained as a liquid if the temperature is adjusted.

In an exemplary embodiment of the present specification, the composition may be in a form in which the heterocyclic compound of Chemical Formula 1 and the compound of Chemical Formula 2 are simply mixed.

The composition may additionally include materials publicly known in the art such as solvents and additives.

The composition may be used when an organic material for an organic light emitting device is formed, and particularly, may be more preferably used when a host of a light emitting layer is formed.

In an exemplary embodiment of the present specification, provided is a method for manufacturing an organic light emitting device, the method including: preparing a substrate; forming a first electrode on the substrate; forming an organic material layer having one or more layers on the first electrode; and forming a second electrode on the organic material layer, in which the forming of the organic material layer includes forming the organic material layer having one or more layers by using the composition for an organic material layer, including heterocyclic compound of Chemical Formula 1.

The method for manufacturing an organic light emitting device according to another exemplary embodiment includes: preparing a substrate; forming a first electrode on the substrate; forming an organic material layer having one or more layers on the first electrode; and forming a second electrode on the organic material layer, in which the forming of the organic material layer includes forming an organic material layer having one or more layers by using a composition for an organic material layer, which includes the heterocyclic compound of Chemical Formula 1 and the compound of Chemical Formula 2, and the forming of the organic material layer may form the organic material layer by pre-mixing the heterocyclic compound of Chemical Formula 1; and the compound of Chemical Formula 2 and using a thermal vacuum deposition method.

The pre-mixing means that before the heterocyclic compound of Chemical Formula 1 and the compound of Chemical Formula 2 are deposited onto an organic material layer, the materials are first mixed and the mixture is contained in one common container and mixed.

The pre-mixed material may be referred to as a composition for an organic material layer according to an exemplary embodiment of the present specification.

Hereinafter, the present specification will be described in more detail through Examples, but these Examples are provided only for exemplifying the present application, and are not intended to limit the scope of the present application.

### <Preparation Examples>

### [Preparation Example 1] Preparation of Compound 1-1

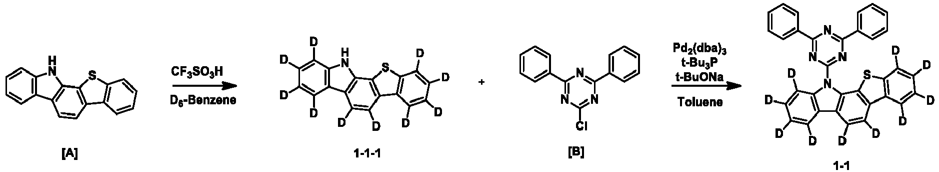

### 1) Preparation of Intermediate 1-1-1

A mixture of 12H-benzo[4,5]thieno[2,3-a]carbazole **[A]** (10 g, 0.037 mol), triflic acid (34 g, 0.23 mol) and D₆-benzene (200 mL) was refluxed at 70°C in a one-neck round bottom flask. The resulting product was quenched and extracted with dichloromethane (DCM) and H₂O, concentrated, and then filtered with silica gel. The filtered product was concentrated, and then treated with methanol to obtain Intermediate 1-1-1 (7.65 g, yield 73%).

### 2) Preparation of Compound 1-1

A mixture of Intermediate 1-1-1 (7.65 g, 0.027 mol), 2-chloro-4,6-diphenyl-1,3,5-triazine **[B]** (7.95 g, 0.030 mol), tris(dibenzylideneacetone)dipalladium(0) (Pd₂(dba)₃) (2.47 g, 0.0027 mol), tri-tert-butylphosphine (t-Bu₃P) (7.00 g, 0.035 mol), sodium tert-butoxide (t-BuONa) (5.19 g, 0.054 mol) and toluene (70 mL) was put into a one-neck round bottom flask and refluxed at 100°C. After the resulting product was extracted with DCM and concentrated, silica gel filtration was performed, and then the filtered product was concentrated, and then treated with methanol to obtain target Compound 1-1 (8.62 g, yield 62%).

Target Compound D1 of the following Table 1 was synthesized in the same manner as in Preparation Example 1, except that Compounds A1 and B1 in the following Table 1 were used instead of 12H-benzo[4,5]thieno[2,3-a]carbazole **[A]** and 2-chloro-4,6-diphenyl-1,3,5-triazine **[B]** in Preparation Example 1.

**[Table 1]**

| Compound | Compound A1 | Compound B1 | Compound D1 | Yield |
|---|---|---|---|---|
| 1-1 | 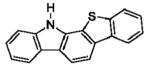 | 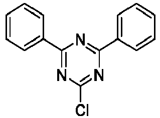 | 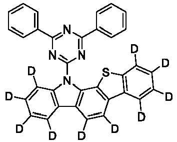 | 62% |
| 1-2 | 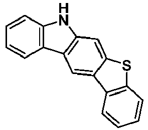 | 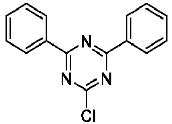 | 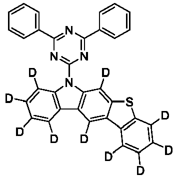 | 95% |
| 1-3 | 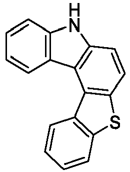 | 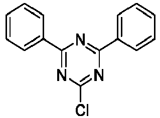 | 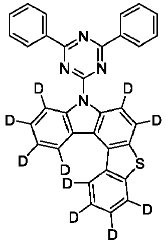 | 83% |
| 1-4 | 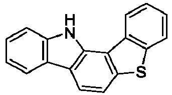 | 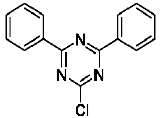 | 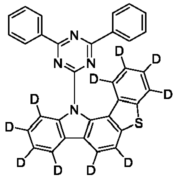 | 96% |
| 1-7 | 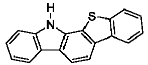 | 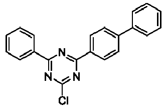 | 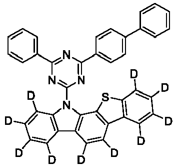 | 83% |
| 1-10 | 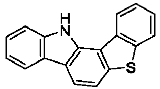 | 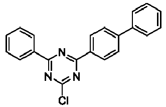 | 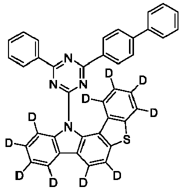 | 77% |
| 1-13 | 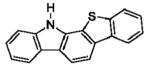 | 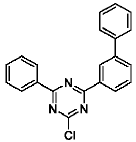 | 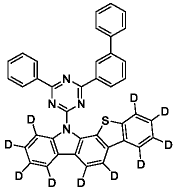 | 75% |
| 1-19 | 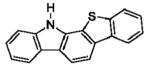 | 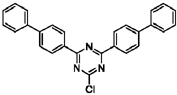 | 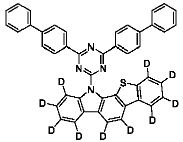 | 72% |
| 1-22 | 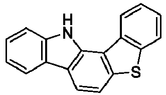 | 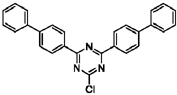 | 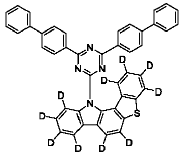 | 93% |
| 1-31 | 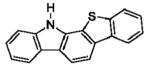 | 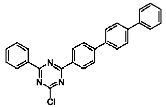 | 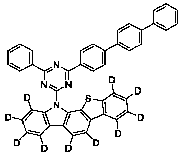 | 66% |
| 1-37 | 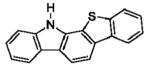 | 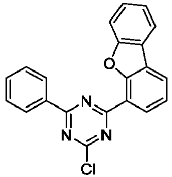 | 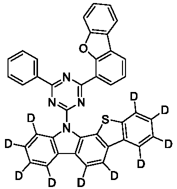 | 62% |
| 1-40 | 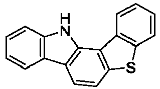 | 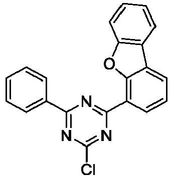 | 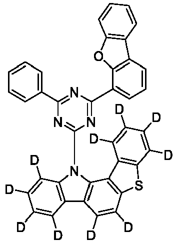 | 71% |
| 1-44 | 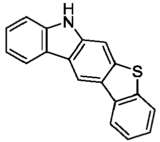 | 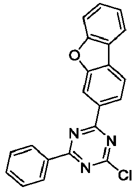 | 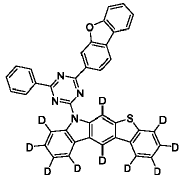 | 92% |
| 1-53 | 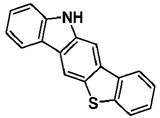 | 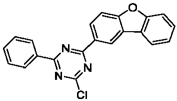 | 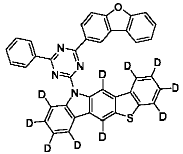 | 63% |
| 1-57 | 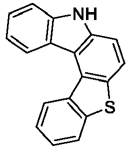 | 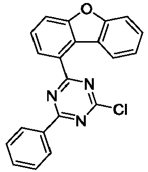 | 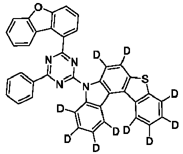 | 65% |
| 1-63 | 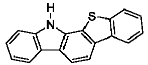 | 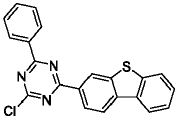 | 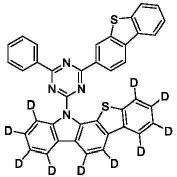 | 82% |
| 1-71 | 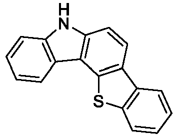 | 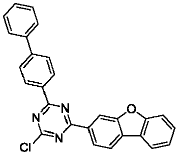 | 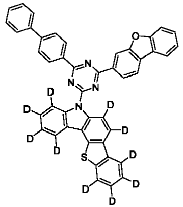 | 78% |
| 1-80 | 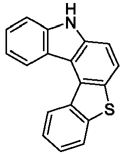 | 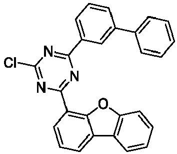 | 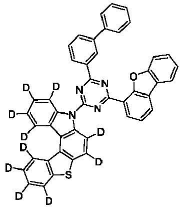 | 90% |
| 1-83 | 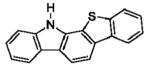 | 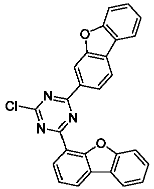 | 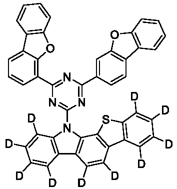 | 68% |
| 1-87 | 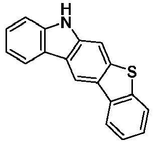 | 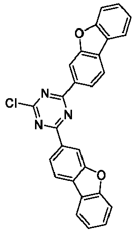 | 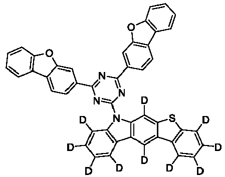 | 91% |
| 1-93 | 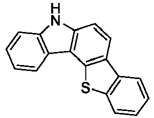 | 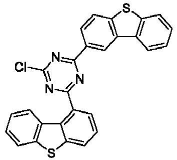 | 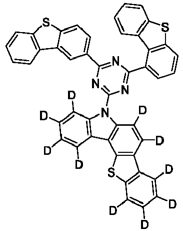 | 58% |
| 1-94 | 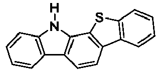 | 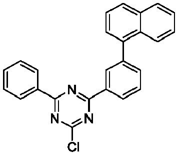 | 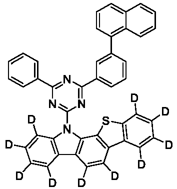 | 73% |
| 1-95 | 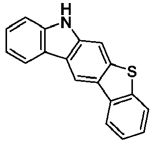 | 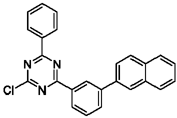 | 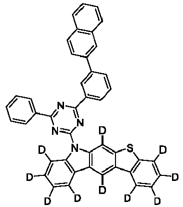 | 75% |
| 1-99 | 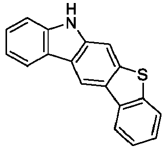 | 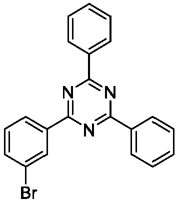 | 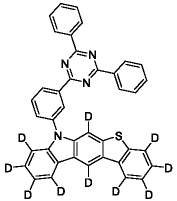 | 69% |
| 1-103 | 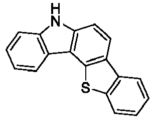 | 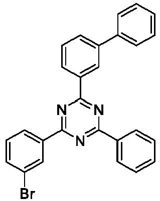 | 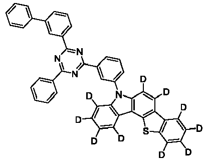 | 59% |
| 1-105 | 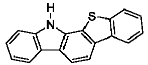 | 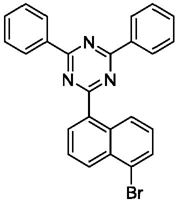 | 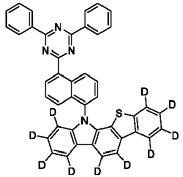 | 73% |
| 1-106 | 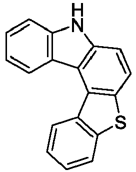 | 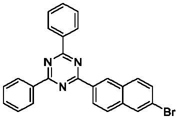 | 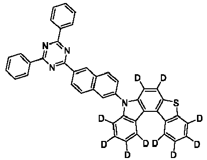 | 69% |
| 1-107 | 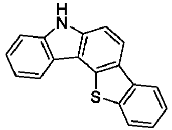 | 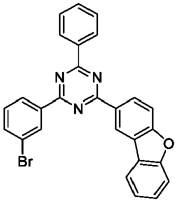 | 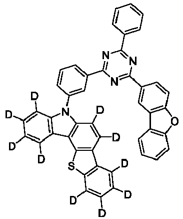 | 72% |
| 1-112 | 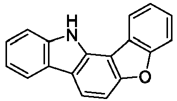 | 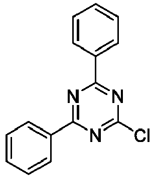 | 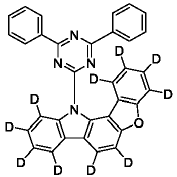 | 63% |
| 1-115 | 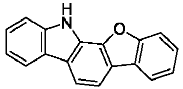 | 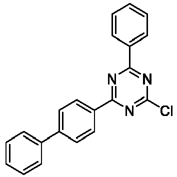 | 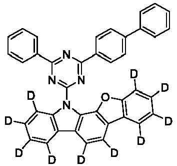 | 59% |
| 1-121 | 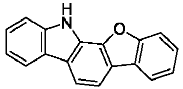 | 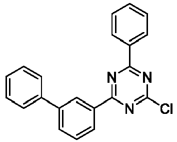 | 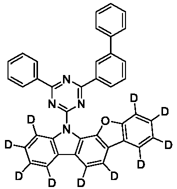 | 76% |
| 1-130 | 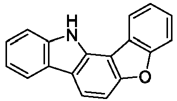 | 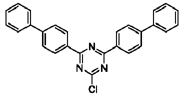 | 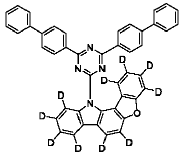 | 85% |
| 1-142 | 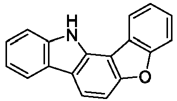 | 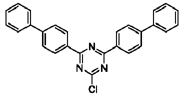 | 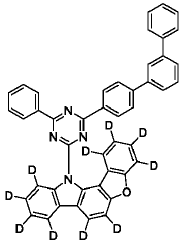 | 82% |
| 1-145 | 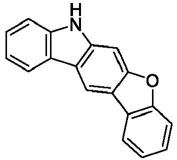 | 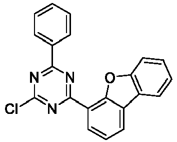 | 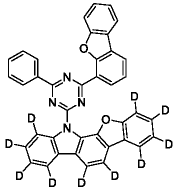 | 73% |
| 1-156 | 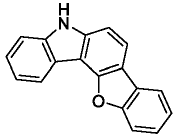 | 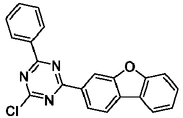 | 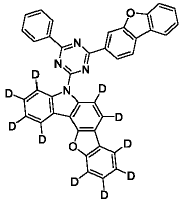 | 77% |
| 1-164 | 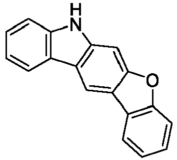 | 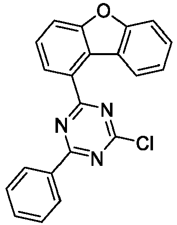 | 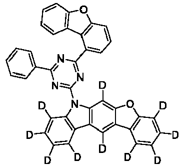 | 92% |
| 1-172 | 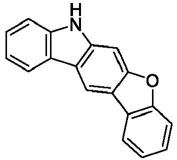 | 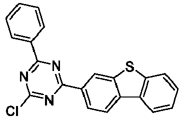 | 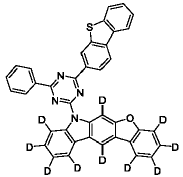 | 81% |
| 1-176 | 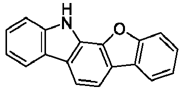 | 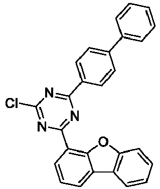 | 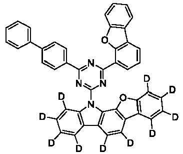 | 69% |
| 1-187 | 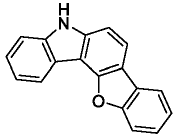 | 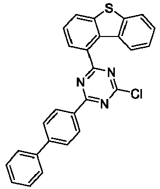 | 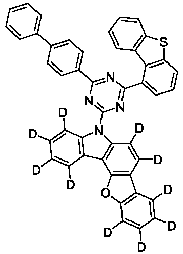 | 65% |
| 1-200 | 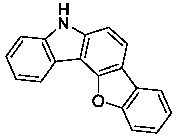 | 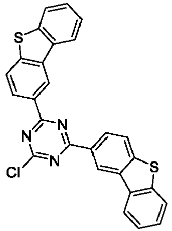 | 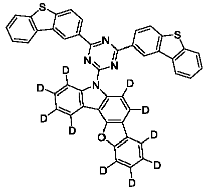 | 70% |
| 1-203 | 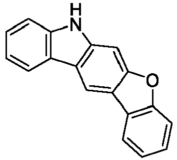 | 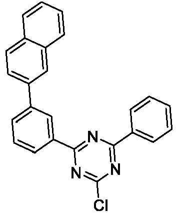 | 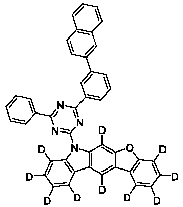 | 76% |
| 1-212 | 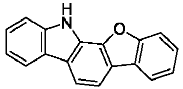 | 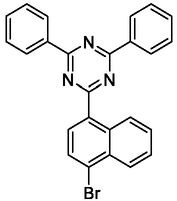 | 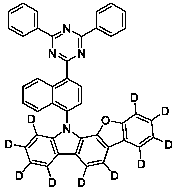 | 81% |
| 1-226 | 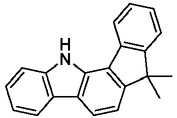 | 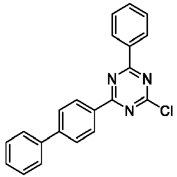 | 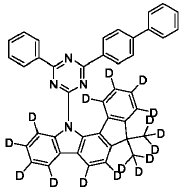 | 88% |
| 1-236 | 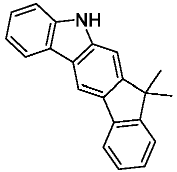 | 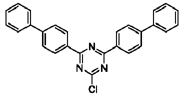 | 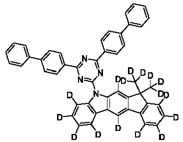 | 79% |
| 1-259 | 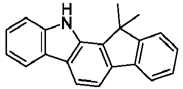 | 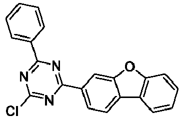 | 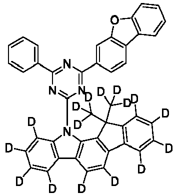 | 63% |
| 1-272 | 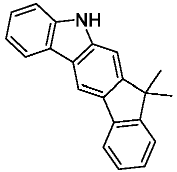 | 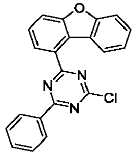 | 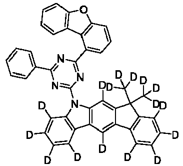 | 91% |
| 1-285 | 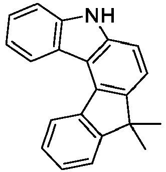 | 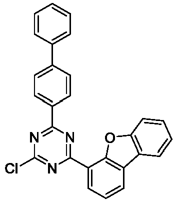 | 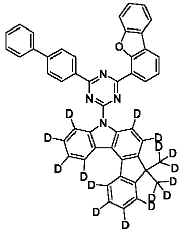 | 59% |
| 1-311 | 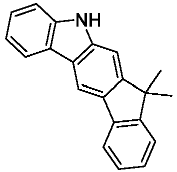 | 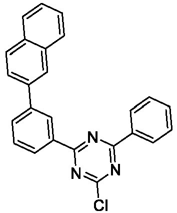 | 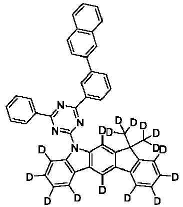 | 64% |
| 1-325 | 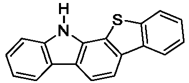 | 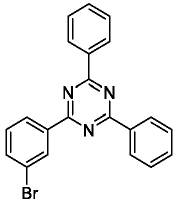 | 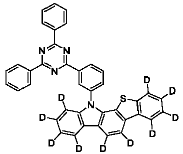 | 91% |
| 1-327 | 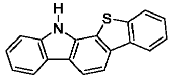 | 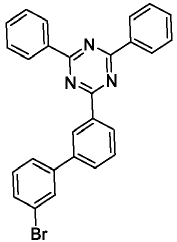 | 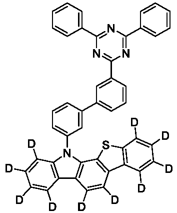 | 77% |
| 1-332 | 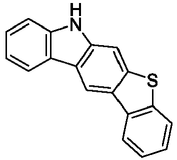 | 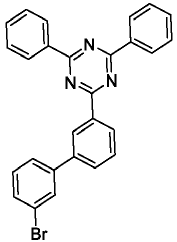 | 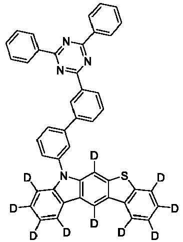 | 83% |
| 1-334 | 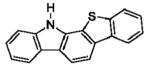 | 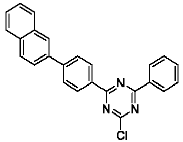 | 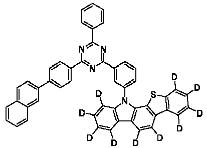 | 69% |
| 1-337 | 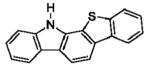 | 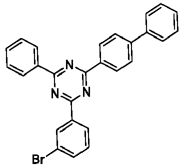 | 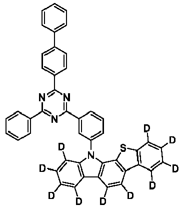 | 75% |
| 1-339 | 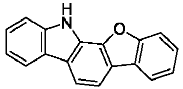 | 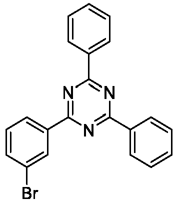 | 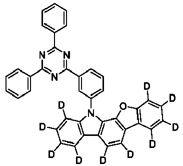 | 66% |
| 1-341 | 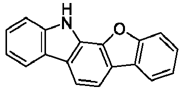 | 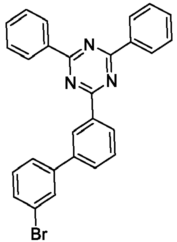 | 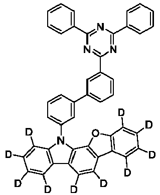 | 57% |
| 1-351 | 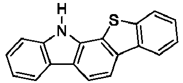 | 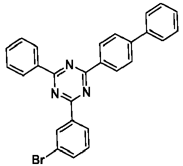 | 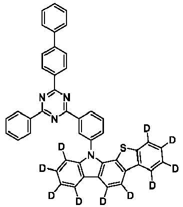 | 92% |

For reference, in the preparation of Intermediate Compound 1-1-1 in Preparation Example 1, as a result of conducting a reaction condition test experiment under the conditions as shown in Experiments 1 to 4 in the following Table 2, it could be confirmed that Compound 1-1-1 was not synthesized.

**[Table 2]**

| Experiment | Compound (g, eq) | Solvent (mL) | Catalyst (mol%) | Temperature /time (°C, d) | Condition | Obtained amount, Yield |
|---|---|---|---|---|---|---|
| 1 | 12H-benzo[4, 5]thieno [2,3-a]carbaz ole (1 g, 1 eq) | D₂O (100 mL) | Pt/C (10 mol%) | 150°C, 4d | Round flask Under Ar bag | NO reaction |
| 2 | 12H-benzo[4, 5] thieno [2,3-a]carbaz ole (1 g, 1 eq) | D₂O, i-PrOH, cyclohex ane (100 mL, 50 mL, 50 mL) | Pt/C, Pd/C (10 mol%) | 150°C, 4d | Round flask Under Ar bag | NO reaction |
| 3 | 12H-benzo[4, 5]thieno [2,3-a]carbaz ole (1 g, 1 eq) | D₂O, i-PrOH, cyclohex ane (100 mL, 50 mL, 50 mL) | Pt/C, Pd/C (10 mol%) | 200°C, 2d | Sealed tube | NO reaction |
| 4 | 12H-benzo[4, 5] thieno [2,3-a]carbaz ole (1 g, 1 eq) | D₂O, i-PrOH, cyclohex ane (100 mL, 50 mL, 50 mL) | Pt/C, Pd/C (10 mol%) | 150°C, 4d | Sealed tube | NO reaction |

Therefore, as shown in the following Table 3, Intermediate Compound 1-1-1 was synthesized under the reaction condition of Experiment 7 having the highest substitution rate.

**[Table 3]**

| Experiment | Compound (g, eq) | Solvent (mL) | Acid (g, eq) | Temperature | Yield | Substitution rate |
|---|---|---|---|---|---|---|
| 5 | 12H-benzo[4,5 ]thieno[2 ,3-a]carbazo le (1 g, 1 eq) | Benzene-D6 (50 mL) | CF3SO3H (13.7 g, 25 eq) | RT | 96% | 43% |
| 6 | 12H-benzo[4,5 ]thieno[2 ,3-a]carbazo le (1 g, 1 eq) | Benzene-D6 (50 mL) | CF3SO3H (13.7 g, 25 eq) | 30°C | 88% | 69% |
| 7 | 12H-benzo[4,5 ]thieno[2 ,3-a]carbazo le (1 g, 1 eq) | Benzene-D6 (50 mL) | CF3SO3H (13.7 g, 25 eq) | 40°C | 71% | 94% |
| 8 | 12H-benzo[4,5 ]thieno[2 ,3-a]carbazo le (1 g, 1 eq) | DMSO-D6 (50 mL) | CF3SO3H (13.7 g, 25 eq) | 40°C | 80% | 55% |
| 9 | 12H-benzo[4,5 ]thieno[2 ,3-a]carbazo le (1 g, 1 eq) | DMF-D6 (50 mL) | CF3SO3H (13.7 g, 25 eq) | 40°C | 760 | 60% |

In the reaction of substituting hydrogen in an organic compound with deuterium, there is a tendency that the higher the reaction temperature, the higher the substitution rate from hydrogen to deuterium and the lower the yield. Here, the substitution rate is calculated by [(the number of deuteriums substituted after the chemical reaction)/(the number of hydrogens in the compound before the chemical reaction)]*100.

### [Preparation Example 2] Preparation of Compound 2-2

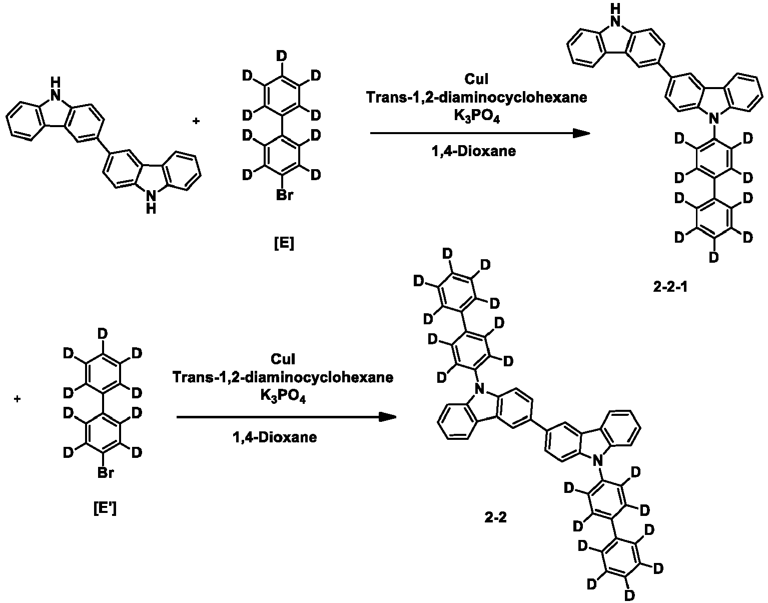

### 1) Preparation of Intermediate 2-2-1

After 9H,9'H-3,3'-bicarbazole (10 g, 0.030 mol), 4-bromo-1,1'-biphenyl-2,2',3,3',4',5,5',6,6'-D₉ [E] (7.26 g, 0.030 mol), CuI (0.57 g, 0.003 mol), trans-1,2-diaminocyclohexane (0.34 g, 0.003 mol), and K₃PO₄ (12.74 g, 0.06 mol) were dissolved in 100 mL of 1,4-dioxane in a one-neck round bottom flask, the resulting solution was refluxed at 125°C for 8 hours. After the reaction was completed, distilled water and DCM were added thereto at room temperature, extraction was performed, the organic layer was dried over MgSO₄, and then the solvent was removed by a rotary evaporator. The reactant was purified by column chromatography (DCM:hexane = 1:3) and recrystallized with methanol to obtain Intermediate 2-2-1 (13.92 g, yield 94%).

### 2) Preparation of Compound 2-2

After Intermediate 2-2-1 (13.92 g, 0.028 mol), 4-bromo-1,1'-biphenyl-2,2',3,3',4',5,5',6,6'-D₉ [E'] (6.83 g, 0.028 mol), CuI (0.53 g, 0.0028 mol), trans-1,2-diaminocyclohexane (0.32 g, 0.0028 mol), and K₃PO₄ (11.89 g, 0.056 mol) were dissolved in 140 mL of 1,4-dioxane in a one-neck round bottom flask, the resulting solution was refluxed at 125°C for 8 hours. After the reaction was completed, distilled water and DCM were added thereto at room temperature, extraction was performed, the organic layer was dried over MgSO₄, and then the solvent was removed by a rotary evaporator. The reactant was purified by column chromatography (DCM:hexane = 1:3) and recrystallized with methanol to obtain target Compound 2-2 (16.14 g, yield 88%).

When Compound E and Compound E' are the same, the target compound may be immediately synthesized by adding 2 equivalents of Compound E in Preparation Example 2-1. That is, when Compound E and Compound E' are the same, the aforementioned Preparation Example 2-2 may be omitted.

The following target Compound G1 was synthesized in the same manner as in the synthesis in Preparation Example 2, except that Compounds E1 and E'1 in the following Table 4 were used instead of 4-bromo-1,1'-biphenyl-2,2',3,3',4',5,5',6,6'-D₉ **[E]** and 4-bromo-1,1'-biphenyl-2,2',3,3',4',5,5',6,6'-D₉ **[E']** in Preparation Example 2.

**[Table 4]**

| Compound | Compound E1 | Compound E'1 | Compound G1 | Yield |
|---|---|---|---|---|
| 2-2 | 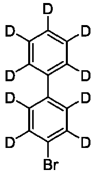 | 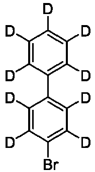 | 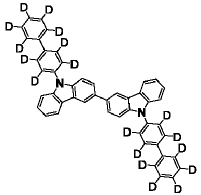 | 88% |
| 2-3 | 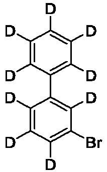 | 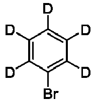 | 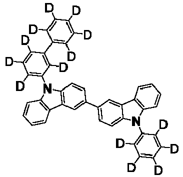 | 95% |
| 2-5 | 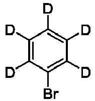 | 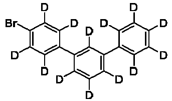 | 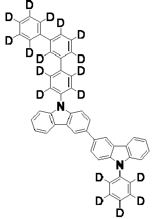 | 84% |
| 2-6 | 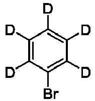 | 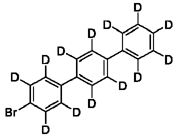 | 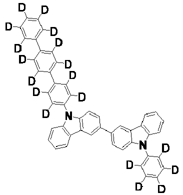 | 67% |
| 2-8 | 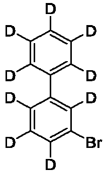 | 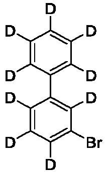 | 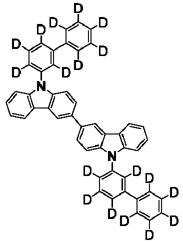 | 83% |
| 2-9 | 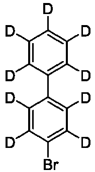 | 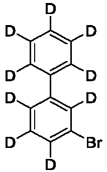 | 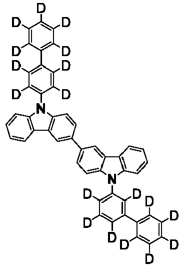 | 74% |
| 2-12 | 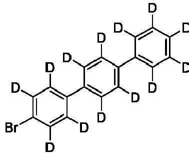 | 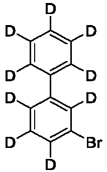 | 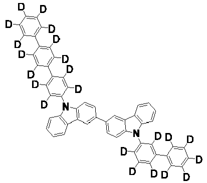 | 69% |
| 2-14 | 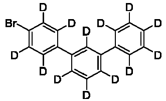 | 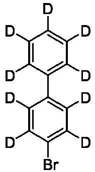 | 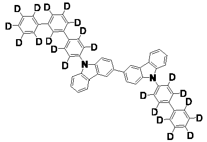 | 57% |
| 2-75 | 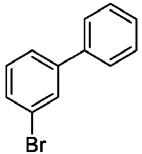 | 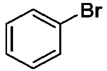 | 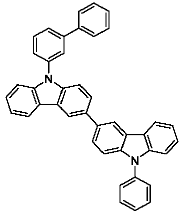 | 68% |
| 2-76 | 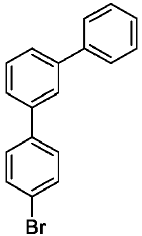 | 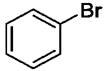 | 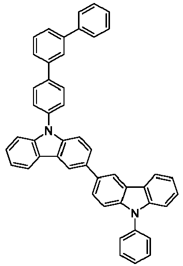 | 72% |
| 2-77 | 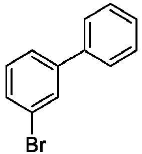 | 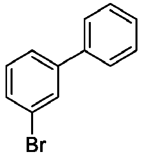 | 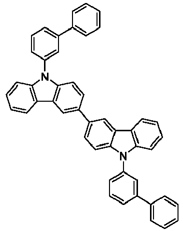 | 83% |
| 2-78 | 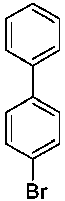 | 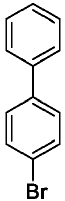 | 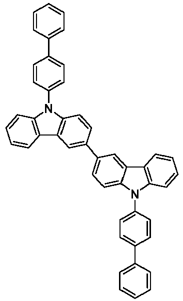 | 88% |

### [Preparation Example 3] Preparation of Compound 2-26

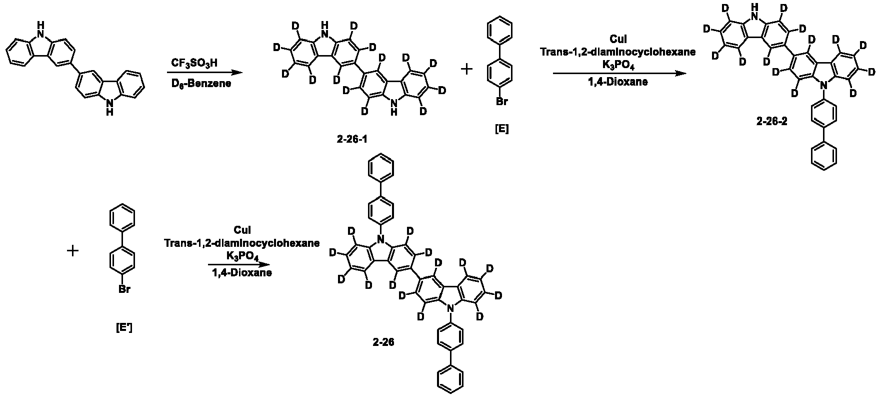

### 1) Preparation of Intermediate 2-26-1

A mixture of 9H,9'H-3,3'-bicarbazole (10 g, 0.030 mol), triflic acid (112.56 g, 0.75 mol) and D₆-benzene (500 mL) was refluxed at 40°C in a one-neck round bottom flask. The resulting product was quenched and extracted with DMC and H₂O and concentrated, and then filtered with silica gel. The filtered product was concentrated, and then treated with methanol to obtain Intermediate 2-26-1 (7.07 g, yield 68%) .

### 2) Preparation of Intermediate 2-26-2

After Intermediate 2-26-1 (7.07 g, 0.02 mol), CuI (0.38 g, 0.002 mol), 4-bromo-1,1'-biphenyl **[E]** (4.66 g, 0.02 mol), trans-1,2-diaminocyclohexane (0.23 g, 0.002 mol), and K₃PO₄ (8.49 g, 0.04 mol) were dissolved in 70 mL of 1,4-dioxane in a one-neck round bottom flask, the resulting solution was refluxed at 125°C for 8 hours. After the reaction was completed, distilled water and DCM were added thereto at room temperature, extraction was performed, the organic layer was dried over MgSO₄, and then the solvent was removed by a rotary evaporator. The reactant was purified by column chromatography (DCM:hexane = 1:3) and recrystallized with methanol to obtain Intermediate 2-26-2 (8.28 g, yield 83%).

### 3) Preparation of Compound 2-26

After Intermediate 2-26-2 (8.28 g, 0.017 mol), CuI (0.32 g, 0.0017 mol), 4-bromo-1,1'-biphenyl **[E']** (3.96 g, 0.017 mol), trans-1,2-diaminocyclohexane (0.19 g, 0.0017 mol), and K₃PO₄ (7.22 g, 0.034 mol) were dissolved in 80 mL of 1,4-dioxane in a one-neck round bottom flask, the resulting solution was refluxed at 125°C for 8 hours. After the reaction was completed, distilled water and DCM were added thereto at room temperature, extraction was performed, the organic layer was dried over MgSO₄, and then the solvent was removed by a rotary evaporator. The reactant was purified by column chromatography (DCM:hexane = 1:3) and recrystallized with methanol to obtain target Compound 2-26 (8.63 g, yield 78%).

When Compound E and Compound E' are the same, the target compound may be immediately synthesized by adding 2 equivalents of Compound E in Preparation Example 3-2. That is, when Compound E and Compound E' are the same, the aforementioned Preparation Example 3-3 may be omitted.

The following target Compound G2 was synthesized in the same manner as in the synthesis in Preparation Example 3, except that Compounds E2 and E'2 in the following Table 5 were used instead of 4-bromo-1,1'-biphenyl **[E]** and 4-bromo-1,1'-biphenyl **[E']** in Preparation Example 3.

**[Table 5]**

| Compound | Compound E | Compound E' | Compound G2 | Yield |
|---|---|---|---|---|
| 2-26 | 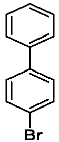 | 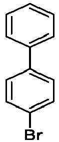 | 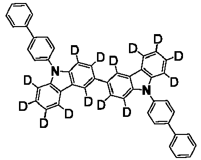 | 78% |
| 2-27 | 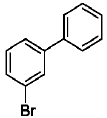 | 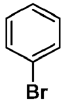 | 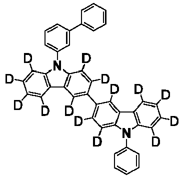 | 90% |
| 2-29 | 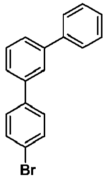 | 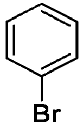 | 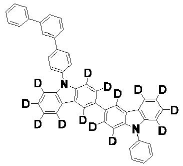 | 74% |
| 2-32 | 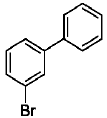 | 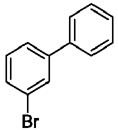 | 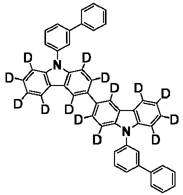 | 69% |
| 2-33 | 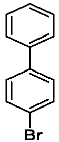 | 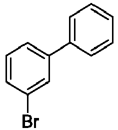 | 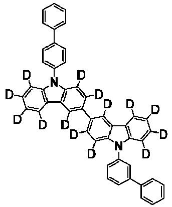 | 93% |

For reference, in the preparation of Intermediate Compound 2-26-1 in Preparation Example 3, as a result of conducting a reaction condition test experiment under the conditions as shown in Experiments 1 to 4 in the following Table 6, it could be confirmed that Compound 2-26-1 was not synthesized.

**[Table 6]**

| Experiment | Compound (g, eq) | Solvent (mL) | Catalyst (mol%) | Temperature/time (°C, d) | Condition | Obtained amount, Yield |
|---|---|---|---|---|---|---|
| 1 | 9H,9'H-3,3'-bicarbazole (1 g, 1 eq) | D₂O (100 mL) | Pt/C (10 mol%) | 150°C, 4d | Round flask Under Ar bag | NO reaction |
| 2 | 9H,9'H-3,3'-bicarbazole (1 g, 1 eq) | D₂O, i-PrOH, cyclohexane (100 mL, 50 mL, 50 mL) | Pt/C, Pd/C (10 mol%) | 150°C, 4d | Round flask Under Ar bag | NO reaction |
| 3 | 9H,9'H-3,3'-bicarbazole (1 g, 1 eq) | D₂O, i-PrOH, cyclohexane (100 mL, 50 mL, 50 mL) | Pt/C, Pd/C (10 mol%) | 200°C, 2d | Sealed tube | NO reaction |
| 4 | 9H,9'H-3,3'-bicarbazole (1 g, 1 eq) | D₂O, i-PrOH, cyclohexane (100 mL, 50 mL, 50 mL) | Pt/C, Pd/C (10 mol%) | 150°C, 4d | Sealed tube | NO reaction |

Therefore, as shown in the following Table 7, Intermediate Compound 2-26-1 was synthesized under the reaction condition of Experiment 7 having the highest substitution rate.

**[Table 7]**

| Experiment | Compound (g, eq) | Solvent (mL) | Acid (g, eq) | Temperature | Yield | Substitution rate |
|---|---|---|---|---|---|---|
| 5 | 9H,9'H-3,3'-bicarbazole (1 g, 1 eq) | Benzene-D6 (50 mL) | CF3SO3H (11.29 g, 25 eq) | RT | 93% | 44% |
| 6 | 9H,9'H-3,3'-bicarbazole (1 g, 1 eq) | Benzene-D6 (50 mL) | CF3SO3H (11.29g, 25 eq) | 30°C | 82% | 70% |
| 7 | 9H,9'H-3,3'-bicarbazole (1 g, 1 eq) | Benzene-D6 (50 mL) | CF3SO3H (11.29g, 25 eq) | 40°C | 68% | 94% |
| 8 | 9H,9'H-3,3'-bicarbazole (1 g, 1 eq) | DMSO-D6 (50 mL) | CF3SO3H (11.29 g, 25 eq) | 40°C | 760 | 61% |
| 9 | 9H,9'H-3,3'-bicarbazole (1 g, 1 eq) | DMF-D6 (50mL) | CF3SO3H (11.29g, 25 eq) | 40°C | 78% | 59% |

In the reaction of substituting hydrogen in an organic compound with deuterium, there is a tendency that the higher the reaction temperature, the higher the substitution rate from hydrogen to deuterium and the lower the yield. Here, the substitution rate is calculated by [(the number of deuteriums substituted after the chemical reaction)/(the number of hydrogens in the compound before the chemical reaction)]*100.

### [Preparation Example 4] Preparation of Compound 2-50

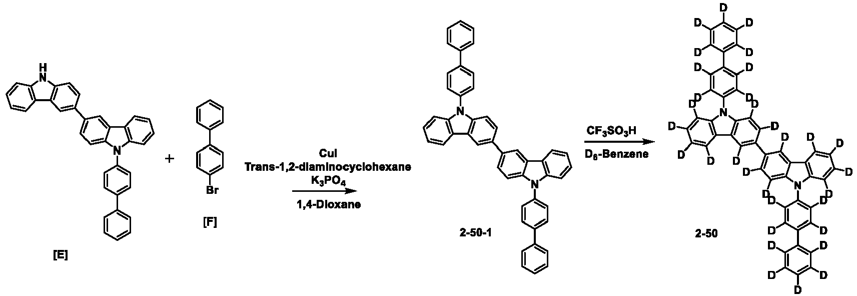

### 1) Preparation of Intermediate 2-50-1

After 9-([1,1'-biphenyl]-4-yl)-9H,9'H-3,3'-bicarbazole **[E]** (10 g, 0.021 mol), 4-bromo-1,1'-biphenyl **[F]** (4.90 g, 0.021 mol), CuI (0.40 g, 0.0021 mol), trans-1,2-diaminocyclohexane (0.024 g, 0.0021 mol), and K₃PO₄ (8.92 g, 0.042 mol) were dissolved in 100 mL of 1,4-dioxane in a one-neck round bottom flask, the resulting solution was refluxed at 125°C for 8 hours. After the reaction was completed, distilled water and DCM were added thereto at room temperature, extraction was performed, the organic layer was dried over MgSO₄, and then the solvent was removed by a rotary evaporator. The reactant was purified by column chromatography (DCM:hexane = 1:3) and recrystallized with methanol to obtain Intermediate 2-50-1 (12.17 g, yield 91%).

### 2) Preparation of Compound 2-50

A mixture of Intermediate 1-50-1 (12.17 g, 0.017 mol), triflic acid (63.78 g, 0.43 mol) and D₆-benzene (600 mL) was refluxed in a one-neck round bottom flask at 40°C. The resulting product was quenched and extracted with DMC and H₂O and concentrated, and then filtered with silica gel. The filtered product was concentrated, and then treated with methanol to obtain target Compound 2-50 (7.62 g, yield 67%) .

The following target Compound G3 was synthesized in the same manner as in the synthesis in Preparation Example 4, except that Compounds E3 and F3 in the following Table 8 were used instead of 9-([1,1'-biphenyl]-4-yl)-9H,9'H-3,3'-bicarbazole **[E]** and 4-bromo-1,1'-biphenyl **[F]** in Preparation Example 4.

**[Table 8]**

| Compound | Compound E3 | Compound F3 | Compound G3 | Yield |
|---|---|---|---|---|
| 2-50 | 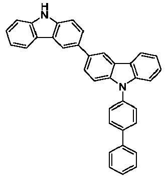 | 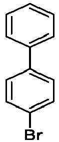 | 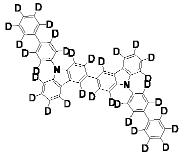 | 78% |
| 2-51 | 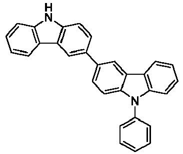 | 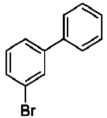 | 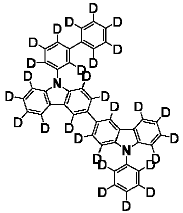 | 91% |
| 2-53 | 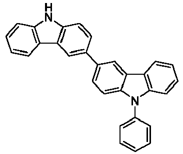 | 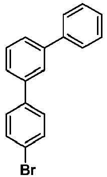 | 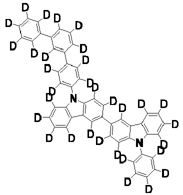 | 93% |
| 2-56 | 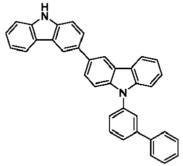 | 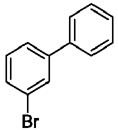 | 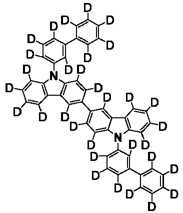 | 76% |
| 2-60 | 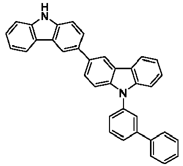 | 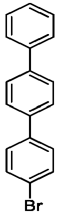 | 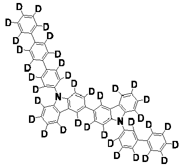 | 72% |
| 2-62 | 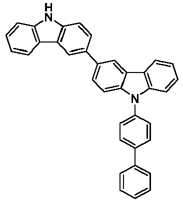 | 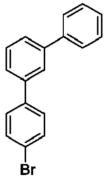 | 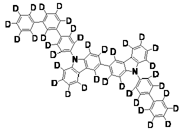 | 88% |
| 2-68 | 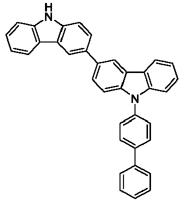 | 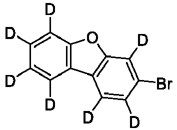 | 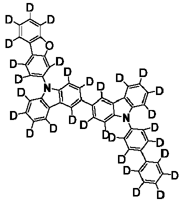 | 76% |

As a result of conducting a reaction condition test experiment under the conditions as shown in Experiments 1 to 4 in the following Table 9, it could be confirmed that Compound 2-50 was not synthesized.

**[Table 9]**

| Experiment | Compound (g, eq) | Solvent (mL) | Catalyst (mol%) | Temperature/time (°C, d) | Condition | Obtained amount, Yield |
|---|---|---|---|---|---|---|
| 1 | 2-50-1 (1 g, 1 eq) | D₂O (100 mL) | Pt/C (10 mol%) | 150°C, 4d | Round flask Under Ar bag | NO reaction |
| 2 | 2-50-1 (1 g, 1 eq) | D₂O, i-PrOH, cyclohexane (100 mL, 50 mL, 50 mL) | Pt/C, Pd/C (10 mol%) | 150°C, 4d | Round flask Under Ar bag | NO reaction |
| 3 | 2-50-1 (1 g, 1 eq) | D₂O, i-PrOH, cyclohexane (100 mL, 50 mL, 50 mL) | Pt/C, Pd/C (10 mol%) | 200 °C, 2d | Sealed tube | NO reaction |
| 4 | 2-50-1 (1 g, 1 eq) | D₂O, i-PrOH, cyclohexane (100 mL, 50 mL, 50 mL) | Pt/C, Pd/C (10 mol%) | 150°C, 4d | Sealed tube | NO reaction |

Therefore, as shown in the following Table 10, Compound 2-50 was synthesized under the reaction condition of Experiment 7 having the highest substitution rate.

**[Table 10]**

| Experime nt | Compound (g, eq) | Solvent (mL) | Acid (g, eq) | Temperat ure | Yield | Substitution rate |
|---|---|---|---|---|---|---|
| 5 | 2-50-1 (1 g, 1 eq) | Benzene-D6 (50 mL) | CF3SO3H (5.89 g, 25 eq) | RT | 88% | 39% |
| 6 | 2-50-1 (1 g, 1 eq) | Benzene-D6 (50 mL) | CF3SO3H (5.89 g, 25 eq) | 30°C | 760 | 53% |
| 7 | 2-50-1 (1 g, 1 eq) | Benzene-D6 (50 mL) | CF3SO3H (5.89g, 25 eq) | 40°C | 67% | 95% |
| 8 | 2-50-1 (1 g, 1 eq) | DMSO-D6 (50 mL) | CF3SO3H (5.89g, 25 eq) | 40°C | 73% | 62% |
| 9 | 2-50-1 (1 g, 1 eq) | DMF-D6 (50 mL) | CF3SO3H (5.89g, 25 eq) | 40°C | 77% | 60% |

Compounds were prepared in the same manner as in the Preparation Examples, and the synthesis confirmation results thereof are shown in the following Tables 11 and 12. Table 11 is about measured values of field desorption mass spectrometry (FD-MS), and Table 12 is about ¹H NMR (DMSO, 300 Mz) values.

**[Table 11]**

| Compound | FD-Mass | Compound | FD-Mass |
|---|---|---|---|
| 1-1 | m/z= 514.68 (C₃₃H₁₀D₁₀N₄S=514.20) | 1-2 | m/z= 514.68 (C₃₃H₁₀D₁₀N₄S=514.20) |
| 1-3 | m/z= 514.68 (C₃₃H₁₀D₁₀N₄S=514.20) | 1-4 | m/z= 514.68 (C₃₃H₁₀D₁₀N₄S=514.20) |
| 1-7 | m/z= 590.77 (C₃₉H₁₄D₁₀N₄S=590.23) | 1-10 | m/z= 590.77 (C₃₉H₁₄D₁₀N₄S=590. 23) |
| 1-13 | m/z= 590.77 (C₃₉H₁₄D₁₀N₄S=590. 23) | 1-19 | m/z= 666.87 (C₄₅H₁₈D₁₀N₄S=666.27) |
| 1-22 | m/z= 666.87 (C₄₅H₁₈D₁₀N₄S=666.27) | 1-31 | m/z= 666.87 (C₄₅H₁₈D₁₀N₄S=666.27) |
| 1-37 | m/z= 604.75 (C₃₉H₁₂D₁₀N₄OS=604.21) | 1-40 | m/z= 604.75 (C₃₉H₁₂D₁₀N₄OS=604.21) |
| 1-44 | m/z= 604.75 (C₃₉H₁₂D₁₀N₄OS=604.21) | 1-53 | m/z= 604.75 (C₃₉H₁₂D₁₀N₄OS=604.21) |
| 1-57 | m/z= 604.75 (C₃₉H₁₂D₁₀N₄OS=604.21) | 1-63 | m/z= 620.81 (C₃₉H₁₂D₁₀N₄S₂=620.19) |
| 1-71 | m/z= 680.85 (C₄₅H₁₆D₁₀N₄OS=680.25) | 1-80 | m/z= 680.85 (C₄₅H₁₆D₁₀N₄OS=680.25) |
| 1-83 | m/z= 694.83 (C₄₅H₁₄D₁₀N₄O₂S=694.22) | 1-87 | m/z= 694.83 (C₄₅H₁₄D₁₀N₄O₂S=694.22) |
| 1-93 | m/z= 726.96 (C₄₅H₁₄D₁₀N₄S₃=726.18) | 1-94 | m/z= 640.83 (C₄₅H₁₆D₁₀N₄S=640.25) |
| 1-95 | m/z= 640.83 (C₄₅H₁₆D₁₀N₄S=640.25) | 1-99 | m/z= 590.77 (C₃₉H₁₄D₁₀N₄S=590.23] |
| 1-103 | m/z= 666.87 (C₄₅H₁₈D₁₀N₄S=666.27) | 1-105 | m/z= 640.83 (C₄₅H₁₆D₁₀N₄S=640.25) |
| 1-106 | m/z= 640.83 (C₄₅H₁₆D₁₀N₄S=640.25) | 1-107 | m/z= 680.85 (C₄₅H₁₆D₁0N₄OS=680.25) |
| 1-112 | m/z= 498.61 (C₃₃H₁₀D₁₀N₄O=498.23) | 1-115 | m/z= 574.71 (C₃₉H₁₄D₁₀N₄O=574.26) |
| 1-121 | m/z= 574.71 (C₃₉H₁₄D₁₀N₄O=574.26) | 1-130 | m/z= 650.81 (C₄₅H₁₈D₁₀N₄O=650.29) |
| 1-142 | m/z= 650.81 (C₄₅H₁₈D₁₀N₄O=650.29) | 1-145 | m/z= 588.69 (C₃₉H₁₂D₁₀N₄O₂=588.24) |
| 1-156 | m/z= 588.69 (C₃₉H₁₂D₁₀N₄O₂=588. 24) | 1-164 | m/z= 588.69 (C₃₉H₁₂D₁₀N₄O₂=588. 24) |
| 1-172 | m/z= 604.75 (C₃₉H₁₂D₁₀N₄OS=604.21) | 1-176 | m/z= 664.79 (C₄₅H₁₆D₁₀N₄O₂=664.27) |
| 1-187 | m/z= 680.85 (C₄₅H₁₅D₁₀N₄OS=680.25) | 1-200 | m/z= 710.90 (C₄₅H₁₄D₁₀N₄OS₂=710.20) |
| 1-203 | m/z= 624.77 (C₄₃H₁₆D₁₀N₄O=624.27) | 1-212 | m/z= 624.77 (C₄₃H₁₆D₁₀N₄O=624.27) |
| 1-226 | m/z= 606.83 (C₄₂H₁₄D₁₆N₄=606.35) | 1-236 | m/z= 682.93 (C₄₈H₁₈D₁₆N₄=682.38) |
| 1-259 | m/z= 620.81 (C₄₂H₁₂D₁₆N₄O=620.33) | 1-272 | m/z= 620.81 (C₄₂H₁₂D₁₈N₄O=620.33) |
| 1-285 | m/z= 696.91 (C₄₈H₁₆D₁₆N₄O=696.36) | 1-311 | m/z= 656.89 (C₄₆H₁₆D₁₆N₄=656.36) |
| 1-325 | m/z= 590.77 (C₃₉H₁₄D₁₀N₄S=690.23) | 1-327 | m/z= 666.87 (C₄₅H₁₈D₁₀N₄S=666.87) |
| 1-332 | m/z= 666.87 (C₄₅H₁₈D₁₀N₄S=666.87) | 1-334 | m/z= 716.93 (C₄₉H₂₀D₁₀N₄S=716.28) |
| 1-337 | m/z= 666.87 (C₄₅H₁₈D₁₀N₄S=666.87) | 1-339 | m/z= 574.71 (C39H14D10N4O=574.26) |
| 1-341 | m/z= 650.81 (C₄₅H₁₈D₁₀N₄O=650.29) | 1-351 | m/z= 666.87 (C₄₅H₁₈D₁₀N₄S=666.27) |
| 2-2 | m/z= 654.91 (C48H14D18N2=654.37) | 2-3 | m/z= 574.79 (C42H14D14N2=574.31) |
| 2-6 | m/z= 654.91 (C48H14D18N2=654.37) | 2-8 | m/z= 654.91 (C48H14D18N2=654.37) |
| 2-9 | m/z= 654.91 (C48H14D18N2=654.37) | 2-12 | m/z= 735.03 (C54H14D22N2=734.43) |
| 2-14 | m/z= 735.03 (C54H14D22N2=734.43) | 2-26 | m/z= 650.88 (C48H18D14N2=650.34) |
| 2-27 | m/z= 574.79 (C42H14D14N2=574.31) | 2-29 | m/z= 650.88 (C48H18D14N2=650.34) |
| 2-32 | m/z= 650.88 (C48H18D14N2=650.34) | 2-33 | m/z= 650.88 (C48H18D14N2=650.34) |
| 2-50 | m/z= 668.99 (C48D32N2=668.46) | 2-51 | m/z= 588.87 (C42D28N2=588.40) |
| 2-53 | m/z= 668.99 (C48D32N2=668.46) | 2-56 | m/z= 668.99 (C48D32N2=668.46) |
| 2-60 | m/z= 749.12 (C54D32N2=748.51) | 2-62 | m/z= 749.12 (C54D36N2=748.51) |
| 2-68 | m/z= 680.96 (C48D30N2O=680.42) | 2-75 | m/z= 560.70 (C42H28N2=560.23) |
| 2-76 | m/z= 636.80 (C48H32N2=636.26) | 2-77 | m/z= 636.80 (C48H32N2=636.26) |
| 2-78 | m/z= 636.80 (C48H32N2=636.26) | | |

**[Table 12]**

| Compound | ¹H NMR(DMSO, 300 Mz) |
|---|---|
| 1-1 | δ = 8.36 (4H, m), 7.50 (6H, m) |
| 1-2 | δ = 8.36 (4H, m), 7.50 (6H, m) |
| 1-3 | δ = 8.36 (4H, m), 7.50 (6H, m) |
| 1-4 | δ = 8.36 (4H, m), 7.50 (6H, m) |
| 1-7 | δ = 8.36 (2H, m), 7.96 (2H, d), 7.75 (2H, d), 7.41∼7.50 (6H, m), 7.25 (2H, d) |
| 1-10 | δ = 8.36 (2H, m), 7.96 (2H, d), 7.75 (2H, d), 7.41∼7.50 (6H, m), 7.25 (2H, d) |
| 1-13 | δ = 8.36∼8.38 (3H, m), 7.94 (1H, s), 7.73∼7.75 (3H, m), 7.61 (1H, d), 7.41∼7.50 (6H, m) |
| 1-19 | δ = 7.96 (4H, d), 7.75 (4H, d), 7.41∼7.49 (6H, m), 7.25 (4H, d) |
| 1-22 | δ = 7.96 (4H, d), 7.75 (4H, d), 7.41∼7.49 (6H, m), 7.25 (4H, d) |
| 1-31 | δ = 8.36 (2H, m), 7.96 (2H, d), 7.75 (2H, d), 7.41∼7.50 (6H, m), 7.25 (6H, m) |
| 1-37 | δ = 8.36 (2H, m), 8.08 (1H, d), 7.98 (1H, d), 7.88 (1H, d), 7.50∼7.54 (5H, m), 7.31∼7.39 (2H, m) |
| 1-40 | δ = 8.36 (2H, m), 8.08 (1H, d), 7.98 (1H, d), 7.88 (1H, d), 7.50∼7.54 (5H, m), 7.31∼7.39 (2H, m) |
| 1-44 | δ = 8.36 (2H, m), 7.98~8.03 (2H, m), 7.76∼7.82 (2H, m), 7.50∼7.54 (4H, m), 7.31∼7.39 (2H, m) |
| 1-53 | δ = 8.36 (2H, m), 7.98 (1H, d), 7.79∼7.88 (3H, m), 7.50∼7.54 (4H, m), 7.31∼7.39 (2H, m) |
| 1-57 | δ = 8.36 (2H, m), 7.98 (1H, d), 7.82 (1H, d), 7.69 (1H, d), 7.50∼7.57 (5H, m), 7.31∼7.39 (2H, m) |
| 1-63 | δ = 8.45 (1H, d), 8.36 (2H, m), 8.20∼8.24 (2H, m), 7.93∼7.94 (2H, d), 7.49∼7.56 (5H, m) |
| 1-71 | δ = 7.96~8.03 (4H, m), 7.75∼7.82 (4H, m), 7.25∼7.54 (8H, m) |
| 1-80 | δ = 8.38 (1H, d), 8.08 (1H, d), 7.94∼7.98 (3H, m), 7.73∼7.75 (3H, m), 7.31∼7.61 (8H, m) |
| 1-83 | δ = 7.98 (2H, m), 7.76∼7.88 (3H, m), 7.51∼7.54 (3H, m), 7.31∼7.39 (4H, m) |
| 1-87 | δ = 7.98~8.03 (4H, m), 7.76∼7.82 (4H, m), 7.54 (2H, d), 7.31∼7.39 (4H, m) |
| 1-93 | δ = 8.45 (2H, d), 8.12 (1H, s), 7.93∼8.03 (6H, m), 7.68 (1H, t), 7.49∼7.56 (4H, m) |
| 1-94 | δ = 8.95 (1H, d), 8.50 (1H, d), 8.36∼8.38 (3H, m), 8.20 (1H, d), 8.09 (1H, d), 7.94 (1H, s), 7.73∼7.77 (2H, m), 7.50∼7.61 (5H, m), 7.39 (1H, t) |
| 1-95 | δ = 8.36∼8.38 (3H, m), 7.94~8.09 (4H, m), 7.73 (1H, t), 7.50∼7.63 (7H, m), 7.38 (1H, d) |
| 1-99 | δ = 8.36 (4H, m), 8.21∼8.24 (2H, m), 7.60∼7.68 (2H, m), 7.50 (6H, m) |
| 1-103 | δ = 8.36∼8.38 (3H, m), 8.21∼8.24 (2H, m), 7.94 (1H, s), 7.60∼7.75 (6H, m), 7.41∼7.50 (6H, m) |
| 1-105 | δ = 8.93 (1H, d), 8.36 (4H, m), 8.22 (1H, d), 8.10 (1H, d), 8.00 (1H, t), 7.79 (1H, t), 7.60 (1H, d), 7.50 (6H, m) |
| 1-106 | δ = 9.09 (1H, s), 8.49 (1H, d), 8.36 (4H, m), 8.03 (1H, d), 7.93 (1H, d), 7.83 (1H, s), 7.50 (6H, m), 7.36 (1H, d) |
| 1-107 | δ = 8.36 (4H, m), 8.21∼8.24 (2H, m), 7.98 (1H, d), 7.79∼7.88 (3H, m), 7.50∼7.68 (6H, m), 7.31∼7.39 (2H, m) |
| 1-112 | δ = 8.36 (4H, m), 7.50 (6H, m) |
| 1-115 | δ = 8.36 (2H, m), 7.96 (2H, d), 7.75 (2H, d), 7.41∼7.50 (6H, m), 7.25 (2H, d) |
| 1-121 | δ = 8.36∼8.38 (3H, m), 7.94 (1H, s), 7.73∼7.75 (3H, m), 7.61 (1H, d), 7.41∼7.50 (6H, m) |
| 1-130 | δ = 7.96 (4H, d), 7.75 (4H, d), 7.41∼7.49 (6H, m), 7.25 (4H, d) |
| 1-142 | δ = 7.96 (4H, d), 7.75 (4H, d), 7.41∼7.49 (6H, m), 7.25 (4H, d) |
| 1-145 | δ = 8.36 (2H, m), 8.08 (1H, d), 7.98 (1H, d), 7.88 (1H, d), 7.50∼7.54 (5H, m), 7.31∼7.39 (2H, m) |
| 1-156 | δ = 8.36 (2H, m), 7.98~8.03 (2H, m), 7.76∼7.82 (2H, m), 7.50∼7.54 (4H, m), 7.31∼7.39 (2H, m) |
| 1-164 | δ = 8.36 (2H, m), 7.98 (1H, d), 7.82 (1H, d), 7.69 (1H, d), 7.50∼7.57 (5H, m), 7.31∼7.39 (2H, m) |
| 1-172 | δ = 8.45 (1H, d), 8.36 (2H, m), 8.20∼8.24 (2H, m), 7.93∼7.94 (2H, d), 7.49∼7.56 (5H, m) |
| 1-176 | δ = 8.08 (1H, d), 7.88∼7.98 (4H, m), 7.75 (2H, d), 7.25∼7.54 (9H, m) |
| 1-187 | δ = 8.45 (1H, d), 7.93~8.03 (5H, m), 7.68∼7.75 (3H, m), 7.41∼7.56 (5H, m), 7.25 (2H, d) |
| 1-200 | δ = 8.45 (1H, d), 8.12 (2H, s), 7.92∼7.99 (6H, m), 7.49∼7.56 (4H, m) |
| 1-203 | δ = 8.36∼8.38 (3H, m), 7.94~8.09 (4H, m), 7.73 (1H, t), 7.50∼7.63 (7H, m), 7.38 (1H, d) |
| 1-212 | δ = 8.97 (1H, d), 8.36 (4H, m), 8.24 (1H, d), 8.12 (1H, d), 7.79 (1H, d), 7.50∼7.59 (8H, m) |
| 1-226 | δ = 8.36 (2H, m), 7.96 (2H, d), 7.75 (2H, d), 7.41∼7.50 (6H, m), 7.25 (2H, d) |
| 1-236 | δ = 7.96 (4H, d), 7.75 (4H, d), 7.41∼7.49 (6H, m), 7.25 (4H, d) |
| 1-259 | δ = 8.36 (2H, m), 7.98~8.03 (2H, m), 7.76∼7.82 (2H, m), 7.50∼7.54 (4H, m), 7.31∼7.39 (2H, m) |
| 1-272 | δ = 8.36 (2H, m), 7.98 (1H, d), 7.82 (1H, d), 7.69 (1H, d), 7.50∼7.57 (5H, m), 7.31∼7.39 (2H, m) |
| 1-285 | δ = 8.08 (1H, d), 7.88∼7.98 (4H, m), 7.75 (2H, d), 7.25∼7.54 (9H, m) |
| 1-311 | δ = 8.36∼8.38 (3H, m), 7.94~8.09 (4H, m), 7.73 (1H, t), 7.50∼7.63 (7H, m), 7.38 (1H, d) |
| 1-325 | δ = 8.36 (4H, m), 8.21∼8.24 (2H, m), 7.60∼7.68 (2H, m), 7.50 (6H, m) |
| 1-327 | δ = 8.36∼8.38 (5H, m), 8.21 (1H, s), 7.94 (1H, s), 7.60∼7.73 (4H, m), 7.47∼7.50 (7H, m) |
| 1-332 | δ = 8.36∼8.38 (5H, m), 8.21 (1H, s), 7.94 (1H, s), 7.60∼7.73 (4H, m), 7.47∼7.50 (7H, m) |
| 1-334 | δ = 8.36 (2H, m), 8.21∼8.24 (2H, m), 7.96~8.09 (5H, m), 7.50∼7.68 (8H, m), 7.38 (1H, d), 7.25 (2H, d) |
| 1-337 | δ = 8.36 (2H, m), 8.21∼8.24 (2H, m), 7.96 (2H, d), 7.60∼7.75 (4H, m), 7.41∼7.50 (6H, m), 7.25 (2H, d) |
| 1-339 | δ = 8.36 (4H, m), 8.21∼8.24 (2H, m), 7.60∼7.68 (2H, m), 7.50 (6H, m) |
| 1-341 | δ = 8.36∼8.38 (3H, m), 8.21 (1H, s), 7.94 (1H, s), 7.60∼7.73 (4H, m), 7.47∼7.50 (7H, m) |
| 1-351 | δ = 8.36 (2H, m), 8.21∼8.24 (2H, m), 7.96 (2H, d), 7.60∼7.75 (4H, m), 7.41∼7.50 (6H, m), 7.25 (2H, d) |
| 2-2 | δ = 8.55 (1H, d), 8.30 (1H, d), 8.13∼8.19 (2H, m), 7.89∼7.99 (4H, m), 7.77 (1H, d), 7.50∼7.58 (2H, m), 7.35 (1H, t), 7.16∼7.20 (2H, t) |
| 2-3 | δ = 8.55 (1H, d), 8.30 (1H, d), 8.13∼8.19 (2H, m), 7.89∼7.99 (4H, m), 7.77 (1H, d), 7.50∼7.58 (2H, m), 7.35 (1H, t), 7.16∼7.20 (2H, t) |
| 2-6 | δ = 8.55 (1H, d), 8.30 (1H, d), 8.13∼8.19 (2H, m), 7.89∼7.99 (4H, m), 7.77 (1H, d), 7.50∼7.58 (2H, m), 7.35 (1H, t), 7.16∼7.20 (2H, t) |
| 2-8 | δ = 8.55 (1H, d), 8.30 (1H, d), 8.13∼8.19 (2H, m), 7.89∼7.99 (4H, m), 7.77 (1H, d), 7.50∼7.58 (2H, m), 7.35 (1H, t), 7.16∼7.20 (2H, t) |
| 2-9 | δ = 8.55 (1H, d), 8.30 (1H, d), 8.13∼8.19 (2H, m), 7.89∼7.99 (4H, m), 7.77 (1H, d), 7.50∼7.58 (2H, m), 7.35 (1H, t), 7.16∼7.20 (2H, t) |
| 2-12 | δ = 8.55 (1H, d), 8.30 (1H, d), 8.13∼8.19 (2H, m), 7.89∼7.99 (4H, m), 7.77 (1H, d), 7.50∼7.58 (2H, m), 7.35 (1H, t), 7.16∼7.20 (2H, t) |
| 2-14 | δ = 8.55 (1H, d), 8.30 (1H, d), 8.13∼8.19 (2H, m), 7.89∼7.99 (4H, m), 7.77 (1H, d), 7.50∼7.58 (2H, m), 7.35 (1H, t), 7.16∼7.20 (2H, t) |
| 2-26 | δ = 7.91∼7.92 (8H, m), 7.75 (4H, d), 7.41∼7.49 (6H, m) |
| 2-27 | δ = 8.21 (1H, s), 7.41∼7.68 (13H, m) |
| 2-29 | δ = 7.91∼7.94 (5H, m), 7.73∼7.75 (3H, t), 7.41∼7.62 (10H, m) |
| 2-32 | δ = 8.21 (2H, s), 7.60∼7.75 (8H, m), 7.41∼7.49 (8H, m) |
| 2-33 | δ = 8.21 (1H, s), 7.91∼7.92 (4H, m), 7.75∼7.68 (6H, m), 7.41∼7.49 (7H, m) |
| 2-50 | δ = deuterium content of 100% with no ¹H NMR peak |
| 2-51 | δ = deuterium content of 100% with no ¹H NMR peak |
| 2-53 | δ = deuterium content of 100% with no ¹H NMR peak |
| 2-56 | δ = deuterium content of 100% with no ¹H NMR peak |
| 2-60 | δ = deuterium content of 100% with no ¹H NMR peak |
| 2-62 | δ = deuterium content of 100% with no ¹H NMR peak |
| 2-68 | δ = deuterium content of 100% with no ¹H NMR peak |
| 2-75 | δ = 8.55 (1H, d), 8.30 (1H, d), 8.13∼8.21 (3H, m), 7.89∼7.99 (4H, m), 7.35∼7.77 (17H, m), 7.16∼7.20 (2H, t) |
| 2-76 | δ = 8.55 (1H, d), 8.30 (1H, d), 8.13∼8.19 (2H, m), 7.89∼7.99 (9H, m), 7.73∼7.77 (4H, m), 7.35∼7.62 (13H, m), 7.16∼7.20 (2H, m) |
| 2-77 | δ = 8.55 (1H, d), 8.30 (1H, d), 8.13∼8.21 (4H, m), 7.89∼7.99 (4H, m), 7.35∼7.77 (20H, m), 7.16∼7.20 (2H, t) |
| 2-78 | δ = 8.55 (1H, d), 8.30 (1H, d), 8.13∼8.19 (2H, m), 7.89∼7.99 (12H, m), 7.75∼7.77 (5H, m), 7.58 (1H, d), 7.35∼7.50 (8H, m), 7.16∼7.20 (2H, m) |

### [Comparative Example Preparation Example 5]

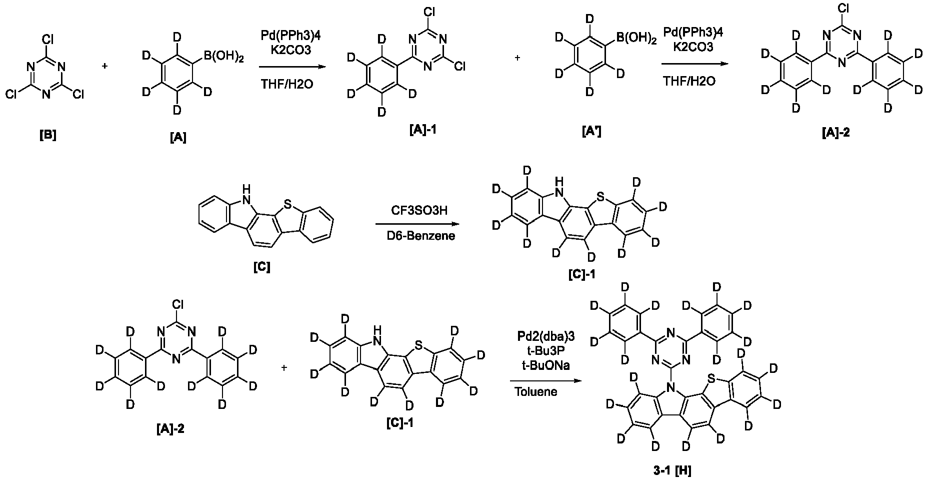

### Preparation of Compound [A]-1

A mixture of 2,4,6-trichloro-1,3,5-triazine (10 g, 0.054 mol), (phenyl-d5)boronic acid (7.49 g, 0.059 mol), Pd(PPh₃)₄ (3.12 g, 0.0027 mol), K₂CO₃ (14.93 g, 0.11 mol) and tetrahydrofuran (100 mL)/water (30 mL) was refluxed at 120°C in a one-neck round bottom flask. The resulting product was cooled, and then concentrated and filtered with silica gel to obtain Compound [A]-1 (6.99 g, 56%).

### Preparation of Compound [A]-2

A mixture of 2,4-dichloro-6-(phenyl-d5)-1,3,5-triazine (6.99 g, 0.030 mol), (phenyl-d5)boronic acid (4.19 g, 0.033 mol), Pd(PPh₃)₄ (1.73 g, 0.0015 mol), K₂CO₃ (8.29 g, 0.06 mol) and tetrahydrofuran (70 mL)/water (20 mL) was refluxed at 120°C in a one-neck round bottom flask. The resulting product was cooled, and then concentrated and filtered with silica gel to obtain Compound [A]-2 (5.58 g, 67%) .

### Preparation of Compound [C]-1

A mixture of 12H-benzo[4,5]thieno[2,3-a]carbazole (10 g, 0.037 mol), triflic acid) (34 g, 0.23 mol) and D6-benzene (200 mL) was refluxed at 70°C in a one-neck round bottom flask. The resulting product was quenched and extracted with dichloromethane (DCM) and H₂O, concentrated, and then filtered with silica gel. The filtered product was concentrated, and then treated with methanol to obtain Compound [C]-1 (7.65 g, 73%).

### Preparation of Compound 3-1[H]

A mixture of Compound 2-chloro-4,6-bis(phenyl-d5)-1,3,5-triazine (5.58 g, 0.020 mol), 12H-benzo[4,5]thieno[2,3-a]carbazole-1,2,3,4,5,6,7,8,9,10-d10 (6.24 g, 0.022 mol), tris(dibenzylideneacetone)dipalladium(0) (Pd₂(dba)₃) (0.92 g, 0.001 mol), tri-tert-butylphosphine (t-Bu₃P) (6.00 g, 0.030 mol), sodium tert-butoxide (t-BuONa) (5.77 g, 0.060 mol) and toluene (60mL) was put into a one-neck round bottom flask and refluxed at 100°C. After the resulting product was extracted with dichloromethane (DCM) and concentrated, silica gel filtration was performed, and then the filtered product was concentrated, and then treated with methanol to obtain target Compound 3-1[H] (8.19 g, yield 78%).

When Compounds A and A' are the same, A-2 may be immediately synthesized by adding 2 equivalents of Compound A in the first reaction. That is, when A and A' are the same, the second reaction step may be omitted.

Compound H in the following Table 13 was synthesized in the same manner as in Comparative Example Preparation Example 5, except that other Intermediates A, A', B, and C were used in Comparative Example Preparation Example 5.

**[Table 13]**

| Compound | A | A' | B | C | H | Yield |
|---|---|---|---|---|---|---|
| 3-1 | 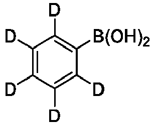 | 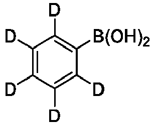 | 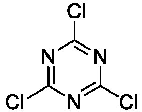 | 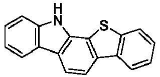 | 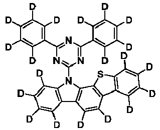 | 780 |
| 3-2 | 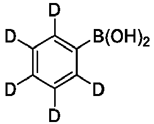 | 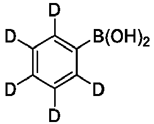 | 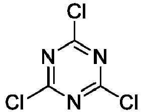 | 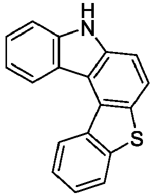 | 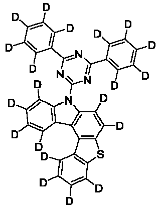 | 68% |
| 3-3 | 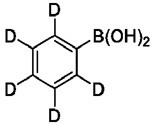 | 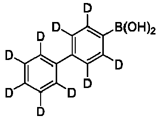 | 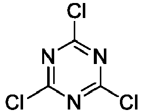 | 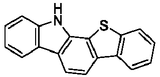 | 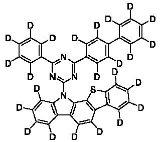 | 87% |
| 3-4 | 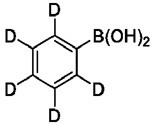 | 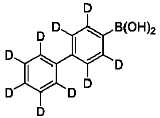 | 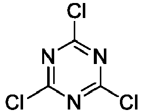 | 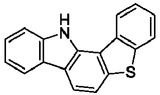 | 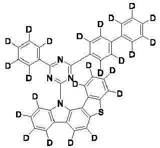 | 55% |
| 3-5 | 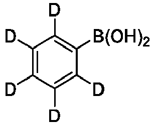 | 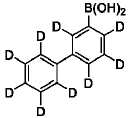 | 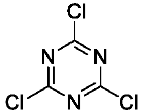 | 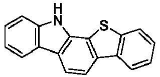 | 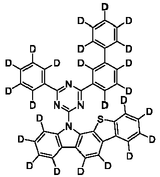 | 65% |
| 3-6 | 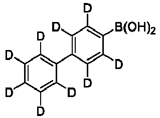 | 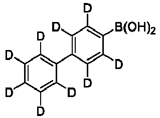 | 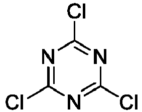 | 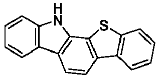 | 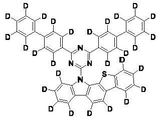 | 710 |
| 3-7 | 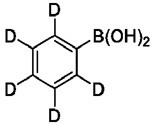 | 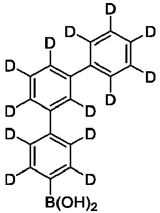 | 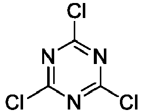 | 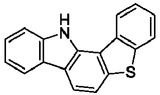 | 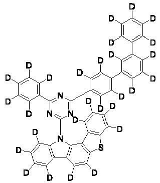 | 880 |
| 3-8 | 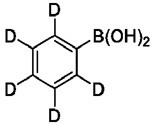 | 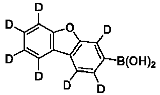 | 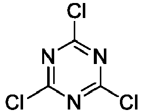 | 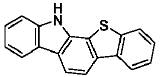 | 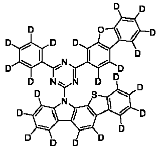 | 51% |
| 3-9 | 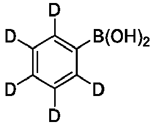 | 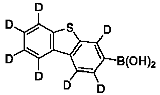 | 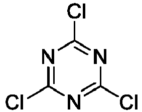 | 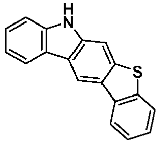 | 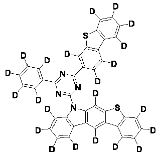 | 63% |
| 3-10 | 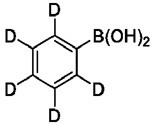 | 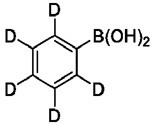 | 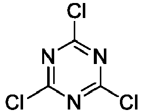 | 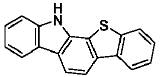 | 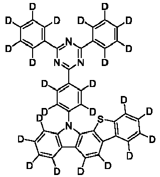 | 79% |
| 3-11 | 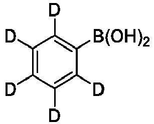 | 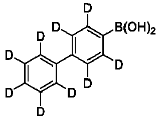 | 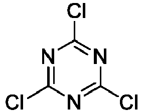 | 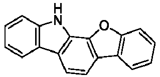 | 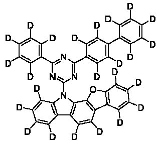 | 83% |
| 3-12 | 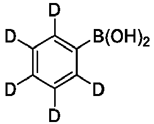 | 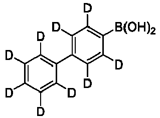 | 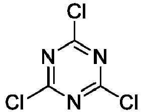 | 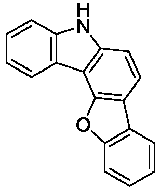 | 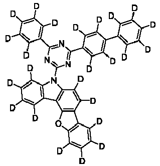 | 77% |
| 3-13 | 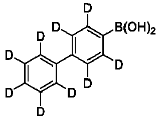 | 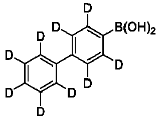 | 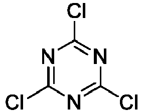 | 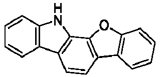 | 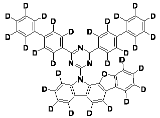 | 65% |
| 3-14 | 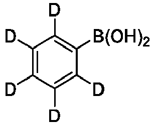 | 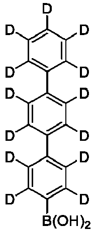 | 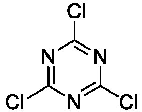 | 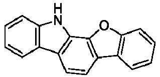 | 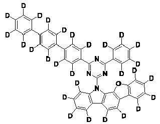 | 57% |
| 3-15 | 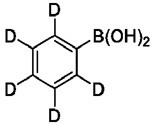 | 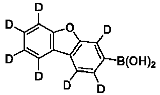 | 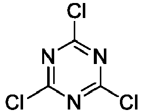 | 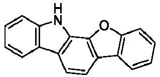 | 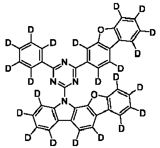 | 69% |
| 3-16 | 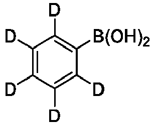 | 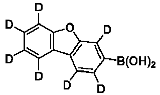 | 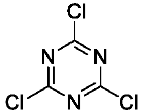 | 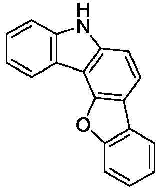 | 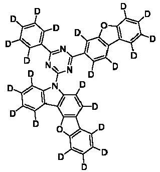 | 58% |

### [Comparative Example Preparation Example 6]

### Preparation of Compound 3-21(1)

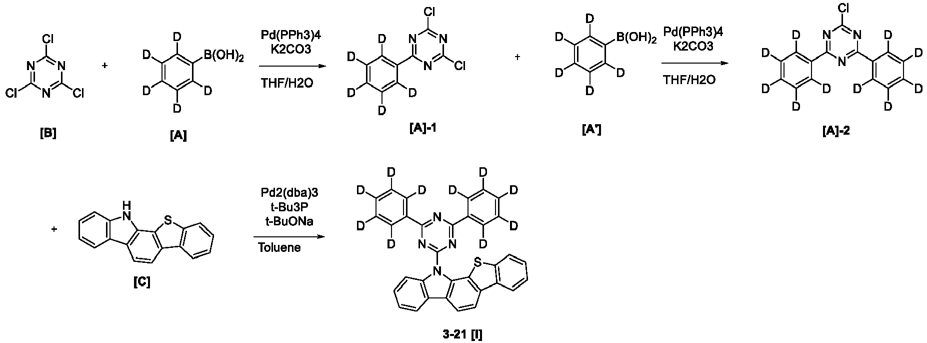

### Preparation of Compound [A]-1

A mixture of 2,4,6-trichloro-1,3,5-triazine (10 g, 0.054 mol), (phenyl-d5)boronic acid (7.49 g, 0.059 mol), Pd(PPh₃)₄ (3.12 g, 0.0027 mol), K₂CO₃ (14.93 g, 0.11 mol) and tetrahydrofuran (100 mL)/water (30 mL) was refluxed at 120°C in a one-neck round bottom flask. The resulting product was cooled, and then concentrated and filtered with silica gel to obtain Compound [A]-1 (6.99 g, 56%).

### Preparation of Compound [A]-2

A mixture of 2,4-dichloro-6-(phenyl-d5)-1,3,5-triazine (6.99 g, 0.030 mol), (phenyl-d5)boronic acid (4.19 g, 0.033 mol), Pd(PPh₃)₄ (1.73 g, 0.0015 mol), K₂CO₃ (8.29 g, 0.06 mol) and tetrahydrofuran (70 mL)/water (20 mL) was refluxed at 120°C in a one-neck round bottom flask. The resulting product was cooled, and then concentrated and filtered with silica gel to obtain Compound [A]-2 (5.58 g, 67%) .

### Preparation of Compound 3-21[I]

A mixture of Compound [A]-2 (5.58 g, 0.020 mol), 12H-benzo[4,5]thieno[2,3-a]carbazole [B] (6.01 g, 0.022 mol), tris(dibenzylideneacetone)dipalladium(0) (Pd₂(dba)₃) (0.92 g, 0.001 mol), tri-tert-butylphosphine (t-Bu₃P) (6.00 g, 0.030 mol), sodium tert-butoxide (t-BuONa) (5.77 g, 0.060 mol) and toluene (60 mL) was put into a one-neck round bottom flask and refluxed at 100°C. After the resulting product was extracted with dichloromethane (DCM) and concentrated, silica gel filtration was performed, and then the filtered product was concentrated, and then treated with methanol to obtain target Compound 3-21[I] (7.41 g, yield 72%).

When Compounds A and A' are the same, A-2 may be immediately synthesized by adding 2 equivalents of Compound A in the first reaction. That is, when A and A' are the same, the second reaction step may be omitted.

The following Compound I was synthesized in the same manner as in the synthesis in Comparative Example Preparation Example 6, except that Intermediates A, A', B, and C in the following Table 14 were used in Comparative Example Preparation Example 6.

**[Table 14]**

| Compound | A | A' | B | C | I | Yield |
|---|---|---|---|---|---|---|
| 3-21 | 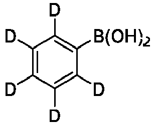 | 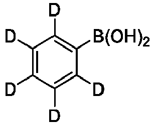 | 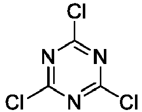 | 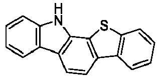 | 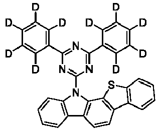 | 720 |
| 3-22 | 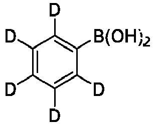 | 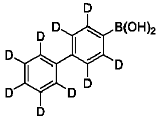 | 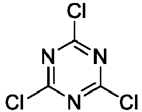 | 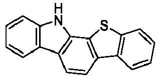 | 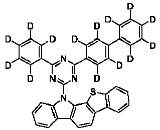 | 610 |
| 3-23 | 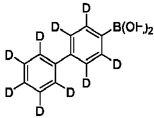 | 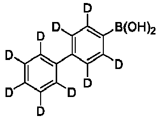 | 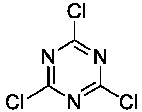 | 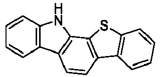 | 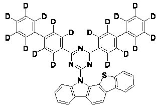 | 59% |
| 3-24 | 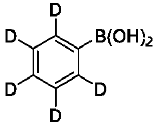 | 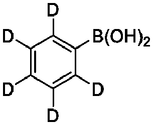 | 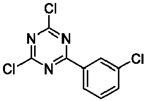 | 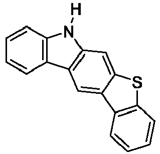 | 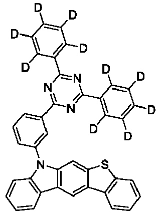 | 55% |

For reference, the above-described Preparation Examples 1 to 4 are easier to synthesize than Comparative Example Production Examples 5 and 6. In addition, the cost of the reagents used when [A]-1 and [A]-2 are synthesized in Comparative Example Preparation Examples 5 and 6 is very high. Furthermore, when [A]-1 and [A]-2 are synthesized, a substance in which Compound A or A' is bonded to all three substituents of 2,4,6-trichloro-1,3,5-triazine is synthesized in a large amount as impurities, so that the selectivity of the reaction also deteriorates. Considering the ease, cost and performance of synthesis, it could be confirmed that the heterocyclic compound structures in Chemical Formula 1 of the present invention are more advantageous in terms of process than the structures of Comparative Example Preparation Examples 5 and 6.

### <Experimental Example 1>

### 1) Manufacture of Organic Light Emitting Device

A glass substrate thinly coated with ITO to have a thickness of 1,500 Å was ultrasonically washed with distilled water. When the washing with distilled water is finished, the glass substrate was ultrasonically washed with a solvent such as acetone, methanol, and isopropyl alcohol, was dried and then was subjected to UVO treatment for 5 minutes by using UV in a UV washing machine. Thereafter, the substrate was transferred to a plasma washing machine (PT), and then was subjected to plasma treatment in a vacuum state for an ITO work function and in order to remove a residual film, and was transferred to a thermal deposition apparatus for organic deposition.

As the common layers, the hole injection layer 4,4',4''-tris[2-naphthyl(phenyl)amino]triphenylamine (2-TNATA) and the hole transporting layer N,N'-di(1-naphthyl)-N,N'-diphenyl-(1,1'-biphenyl)-4,4'-diamine (NPB) were formed on the ITO transparent electrode (positive electrode).

A light emitting layer was thermally vacuum deposited thereon as follows. The light emitting layer was deposited to have a thickness of 400 Å by using a compound described in the following Table 15 as a host and tris(2-phenylpyridine)iridium (Ir(ppy)3) as a green phosphorescent dopant to dope the host with Ir(ppy)₃ in an amount of 7%. Thereafter, bathocuproine (BCP) as a hole blocking layer was deposited to have a thickness of 60 Å, and Alq₃ as an electron transport layer was deposited to have a thickness of 200 Å thereon. Finally, lithium fluoride (LiF) was deposited to have a thickness of 10 Å on the electron transport layer to form an electron injection layer, and then aluminum (Al) negative electrode was deposited to have a thickness of 1200 Å on the electron injection layer to form a negative electrode, thereby manufacturing an organic electroluminescence device.

Meanwhile, all the organic compounds required for manufacturing an OLED device were subjected to vacuum sublimed purification under 10⁻⁸ to 10⁻⁶ torr for each material, and used for the manufacture of OLED.

### [Comparative

### Example]

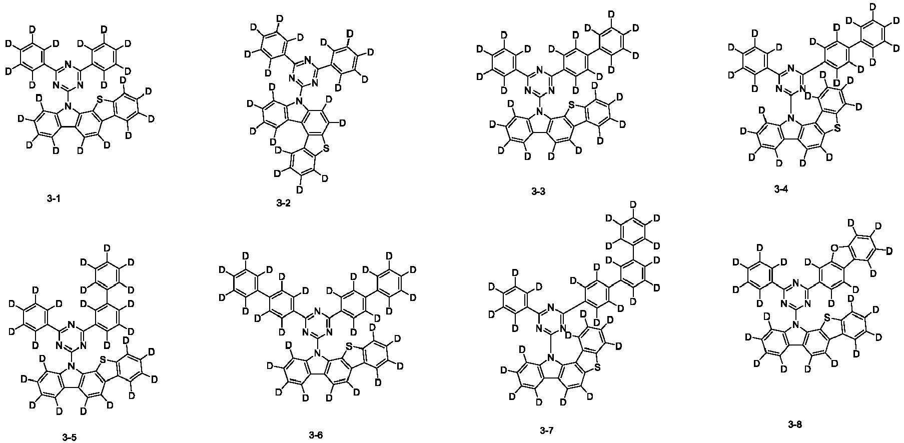

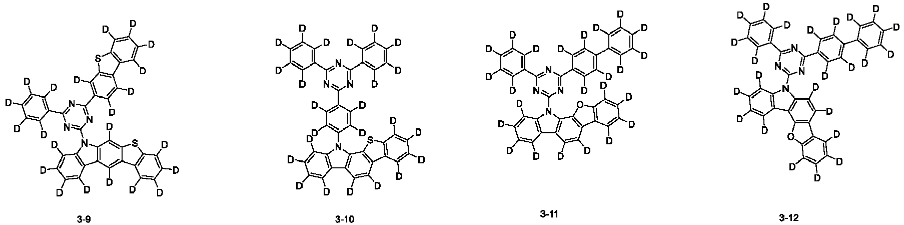

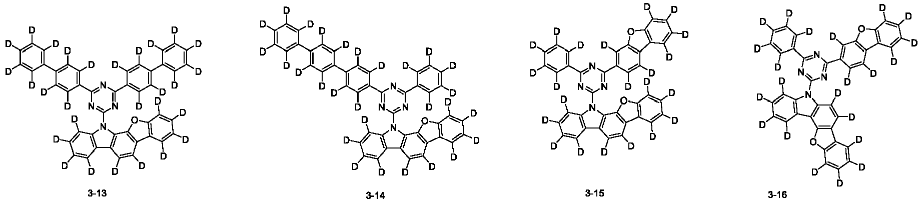

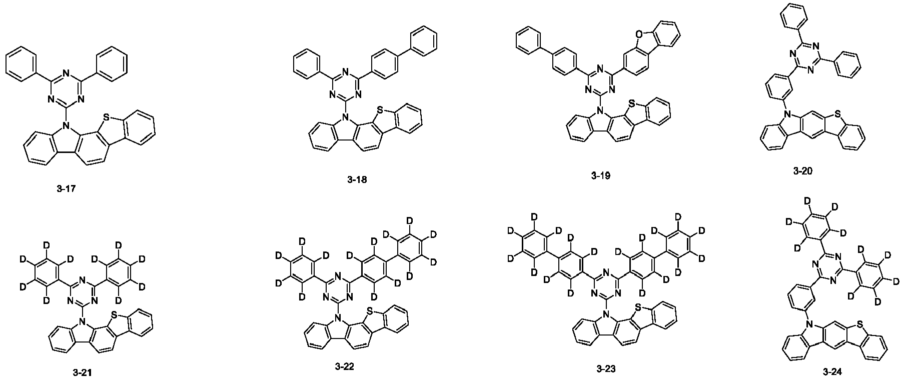

### 2) Driving Voltage and Light Emitting Efficiency of Organic Electroluminescence Device

For the organic electroluminescence device manufactured as described above, electroluminescence (EL) characteristics were measured by M7000 manufactured by McScience Inc., and based on the measurement result thereof, T₉₀ was measured by a service life measurement device (M6000) manufactured by McScience Inc., when the reference luminance was 6,000 cd/m².

The results of measuring the driving voltage, light emitting efficiency, color coordinate (CIE) and service life of the organic light emitting device manufactured according to the present invention are shown in the following Table 15.

**[Table 15]**

| | Compound | Driving voltage (V) | Efficiency (cd/A) | Color coordinate (x, y) | Service life (T₉₀) |
|---|---|---|---|---|---|
| Comparative Example 1 | 3-1 | 5.22 | 43.0 | (0.275, 0.671) | 72 |
| Comparative Example 2 | 3-2 | 5.13 | 43.7 | (0.276, 0.673) | 74 |
| Comparative Example 3 | 3-3 | 5.78 | 44.2 | (0.284, 0.668) | 75 |
| Comparative Example 4 | 3-4 | 5.66 | 44.1 | (0.280, 0.674) | 73 |
| Comparative Example 5 | 3-5 | 5.72 | 44.9 | (0.271, 0.673) | 71 |
| Comparative Example 6 | 3-6 | 5.83 | 43.8 | (0.284, 0.659) | 68 |
| Comparative Example 7 | 3-7 | 5.43 | 43.2 | (0.268, 0.651) | 66 |
| Comparative Example 8 | 3-8 | 5.28 | 44.6 | (0.284, 0.676) | 63 |
| Comparative Example 9 | 3-9 | 5.33 | 43.8 | (0.254, 0.656) | 68 |
| Comparative Example 10 | 3-10 | 5.35 | 42.6 | (0.275, 0.667) | 69 |
| Comparative Example 11 | 3-11 | 5.24 | 45.0 | (0.268, 0.681) | 75 |
| Comparative Example 12 | 3-12 | 5.03 | 44.2 | (0.274, 0.675) | 77 |
| Comparative Example 13 | 3-13 | 5.18 | 43.8 | (0.266, 0.671) | 65 |
| Comparative Example 14 | 3-14 | 5.23 | 42.3 | (0.268, 0.674) | 68 |
| Comparative Example 15 | 3-15 | 5.62 | 43.7 | (0.263, 0.676) | 74 |
| Comparative Example 16 | 3-16 | 5.46 | 45.2 | (0.284, 0.675) | 70 |
| Comparative Example 17 | 3-17 | 6.11 | 42.1 | (0.263, 0.677) | 65 |
| Comparative Example 18 | 3-18 | 6.42 | 42.6 | (0.285, 0.678) | 62 |
| Comparative Example 19 | 3-19 | 6.07 | 41.7 | (0.271, 0.675) | 69 |
| Comparative Example 20 | 3-20 | 6.23 | 43.2 | (0.282, 0.673) | 66 |
| Comparative Example 21 | 3-21 | 6.29 | 42.3 | (0.275, 0.657) | 63 |
| Comparative Example 22 | 3-22 | 6.40 | 40.9 | (0.665, 0.253) | 58 |
| Comparative Example 23 | 3-23 | 6.38 | 41.8 | (0.624, 0.269) | 63 |
| Comparative Example 24 | 3-24 | 6.22 | 42.5 | (0.265, 0.686) | 56 |
| Example 1 | 1-1 | 4.17 | 95.2 | (0.244, 0.637) | 145 |
| Example 2 | 1-2 | 4.12 | 90.5 | (0.269, 0.621) | 149 |
| Example 3 | 1-3 | 4.03 | 87.9 | (0.254, 0.643) | 155 |
| Example 4 | 1-4 | 3.68 | 83.2 | (0.245, 0.673) | 157 |
| Example 5 | 1-7 | 3.77 | 90.3 | (0.257, 0.645) | 168 |
| Example 6 | 1-10 | 3.89 | 91.5 | (0.264, 0.641) | 166 |
| Example 7 | 1-13 | 3.66 | 88.7 | (0.251, 0.614) | 162 |
| Example 8 | 1-19 | 3.59 | 89.2 | (0.264, 0.658) | 158 |
| Example 9 | 1-22 | 3.75 | 90.2 | (0.255, 0.645) | 152 |
| Example 10 | 1-31 | 3.84 | 91.3 | (0.259, 0.704) | 154 |
| Example 11 | 1-37 | 3.93 | 92.2 | (0.247, 0.656) | 159 |
| Example 12 | 1-40 | 3.72 | 94.7 | (0.256, 0.634) | 161 |
| Example 13 | 1-44 | 3.53 | 89.2 | (0.267, 0.625) | 163 |
| Example 14 | 1-53 | 3.62 | 87.2 | (0.258, 0.620) | 155 |
| Example 15 | 1-57 | 3.69 | 88.5 | (0.253, 0.723) | 158 |
| Example 16 | 1-63 | 3.56 | 90.2 | (0.243, 0.612) | 153 |
| Example 17 | 1-71 | 4.13 | 93.7 | (0.264, 0.667) | 172 |
| Example 18 | 1-80 | 4.02 | 91.2 | (0.252, 0.625) | 166 |
| Example 19 | 1-83 | 3.51 | 87.6 | (0.247, 0.656) | 162 |
| Example 20 | 1-87 | 3.62 | 89.3 | (0.244, 0.643) | 158 |
| Example 21 | 1-93 | 3.77 | 92.3 | (0.264, 0.652) | 145 |
| Example 22 | 1-94 | 4.20 | 94.2 | (0.264, 0.623) | 154 |
| Example 23 | 1-95 | 4.10 | 91.7 | (0.254, 0.654) | 166 |
| Example 24 | 1-99 | 3.63 | 94.7 | (0.254, 0.703) | 162 |
| Example 25 | 1-103 | 3.72 | 95.0 | (0.243, 0.627) | 159 |
| Example 26 | 1-105 | 4.11 | 88.7 | (0.264, 0.730) | 170 |
| Example 27 | 1-106 | 3.83 | 87.3 | (0.262, 0.645) | 168 |
| Example 28 | 1-107 | 3.54 | 86.4 | (0.253, 0.634) | 152 |
| Example 29 | 1-112 | 4.13 | 80.2 | (0.256, 0.632) | 137 |
| Example 30 | 1-115 | 4.05 | 87.6 | (0.258, 0.687) | 122 |
| Example 31 | 1-121 | 4.35 | 85.5 | (0.235, 0.641) | 138 |
| Example 32 | 1-130 | 4.22 | 84.9 | (0.237, 0.613) | 131 |
| Example 33 | 1-142 | 4.39 | 83.2 | (0.243, 0.613) | 135 |
| Example 34 | 1-145 | 4.47 | 81.6 | (0.253, 0.627) | 120 |
| Example 35 | 1-156 | 4.23 | 84.7 | (0.255, 0.712) | 128 |
| Example 36 | 1-164 | 4.12 | 89.2 | (0.242, 0.627) | 132 |
| Example 37 | 1-172 | 4.38 | 88.3 | (0.264, 0.744) | 136 |
| Example 38 | 1-176 | 4.25 | 85.4 | (0.234, 0.613) | 140 |
| Example 39 | 1-187 | 4.36 | 82.6 | (0.246, 0.643) | 134 |
| Example 40 | 1-200 | 4.42 | 83.7 | (0.257, 0.619) | 122 |
| Example 41 | 1-203 | 4.08 | 86.7 | (0.256, 0.724) | 132 |
| Example 42 | 1-212 | 4.23 | 87.2 | (0.245, 0.623) | 142 |
| Example 43 | 1-226 | 4.77 | 66.2 | (0.267, 0.731) | 95 |
| Example 44 | 1-236 | 4.83 | 68.7 | (0.251, 0.713) | 100 |
| Example 45 | 1-259 | 4.91 | 70.2 | (0.242, 0.665) | 103 |
| Example 46 | 1-272 | 4.66 | 69.3 | (0.257, 0.633) | 97 |
| Example 47 | 1-285 | 4.87 | 67.5 | (0.265, 0.642) | 91 |
| Example 48 | 1-311 | 4.56 | 69.3 | (0.251, 0.634) | 93 |
| Example 49 | 1-325 | 3.12 | 110.3 | (0.257, 0.671) | 175 |
| Example 50 | 1-327 | 3.54 | 100.6 | (0.265, 0.651) | 163 |
| Example 51 | 1-332 | 3.44 | 108.9 | (0.287, 0.674) | 152 |
| Example 52 | 1-334 | 3.05 | 105.5 | (0.271, 0.673) | 148 |
| Example 53 | 1-337 | 3.28 | 106.2 | (0.276, 0.663) | 166 |
| Example 54 | 1-339 | 3.26 | 95.6 | (0.244, 0.622) | 163 |
| Example 55 | 1-341 | 3.42 | 98.7 | (0.263, 0.625) | 158 |
| Example 56 | 1-351 | 3.33 | 100.5 | (0.253, 0.624) | 172 |

Referring to the results of Table 15, it can be seen that an organic light emitting device including the heterocyclic compound of the present invention has excellent driving voltage, light emitting efficiency and service life than the Comparative Examples. In particular, it can be confirmed that the higher the deuterium substitution rate, the lower the driving voltage and the better the service life characteristics.

Specifically, the case where deuterium is included only in a specific position as in the present invention provides an organic light emitting device having a lower driving voltage, higher light emitting efficiency, and longer service life than the comparative examples in which a compound that does not include deuterium is used. This is because the compound of the present invention is substituted with deuterium that has a higher molecular weight than hydrogen to reduce the change in vibrational frequency and lower the energy of the molecule, and thus the stability of molecule is enhanced. Further, since the single bond dissociation energy of carbon and deuterium is higher than the single bond dissociation energy of carbon and hydrogen, it can be confirmed that the service life of the device is improved as the thermal stability of the molecule is enhanced.

In the case of a compound included only in a specific position as in Chemical Formula 1 of the present invention, when the compound is deposited on a silicon wafer, a material including deuterium tends to be packed with a narrower intermolecular distance. Further, when the surface of a thin film is observed using an atomic force microscope (AFM), it could be confirmed that the thin film made of a compound including deuterium is deposited with a more uniform surface without any aggregated portion.

In addition, when the organic light emitting device is driven, the molecule is thermally damaged by the transfer of electrons. In particular, it is highly likely that defects occur in oxygen and sulfur, which are the most unstable sites of Chemical Formula 1 of the present invention. A compound corresponding to Chemical Formula 1 of the present invention reduces the change in vibrational frequency and lowers the energy of molecule by substituting the core structure of a heterocyclic compound with deuterium having a larger molecular weight than hydrogen to prevent the likelihood, and accordingly, it could be confirmed that the stability of molecule is enhanced, and thus the service life of the device is significantly enhanced compared to the Comparative Examples.

In other words, it can be confirmed that when the heterocyclic compound of Chemical Formula 1 of the present invention is used as a host of a light emitting layer, the driving voltage, light emitting efficiency and service life are remarkably excellent.

### <Experimental Example 2>

### 1) Manufacture of Organic Light Emitting Device

A glass substrate thinly coated with ITO to have a thickness of 1,500 Å was ultrasonically washed with distilled water. When the washing with distilled water was finished, the glass substrate was ultrasonically washed with a solvent such as acetone, methanol, and isopropyl alcohol, dried and then was subjected to UVO treatment for 5 minutes using UV in a UV cleaning machine. Thereafter, the substrate was transferred to a plasma washing machine (PT), and then was subjected to plasma treatment in a vacuum state for an ITO work function and in order to remove a residual film, and was transferred to a thermal deposition apparatus for organic deposition.

As the common layers, the hole injection layer 4,4',4''-tris[2-naphthyl(phenyl)amino]triphenylamine (2-TNATA) and the hole transporting layer N,N'-di(1-naphthyl)-N,N'-diphenyl-(1,1'-biphenyl)-4,4'-diamine (NPB) were formed on the ITO transparent electrode (positive electrode).

A light emitting layer was thermally vacuum deposited thereon as follows. The light emitting layer was deposited to have a thickness of 400 Å from one supply source after pre-mixing one type of the heterocyclic compound of Chemical Formula 1 and one type of compound of Chemical Formula 2 as hosts, and then was deposited by doping the host with Ir(ppy)₃ as a green phosphorescent dopant in an amount of 7% of the deposition thickness of the light emitting layer. Thereafter, BCP as a hole blocking layer was deposited to have a thickness of 60 Å, and Alq₃ as an electron transport layer was deposited to have a thickness of 200 Å thereon. Finally, lithium fluoride (LiF) was deposited to have a thickness of 10 Å on the electron transport layer to form an electron injection layer, and then aluminum (Al) negative electrode was deposited to have a thickness of 1,200 Å on the electron injection layer to form a negative electrode, thereby manufacturing an organic electroluminescence device.

Meanwhile, all the organic compounds required for manufacturing an OLED device were subjected to vacuum sublimed purification under 10⁻⁸ to 10⁻⁶ torr for each material, and used for the manufacture of OLED.

For the organic electroluminescence device manufactured as described above, electroluminescence (EL) characteristics were measured by M7000 manufactured by McScience Inc., and based on the measurement result thereof, T₉₀ was measured by a service life measurement device (M6000) manufactured by McScience Inc., when the reference luminance was 6,000 cd/m².

The results of measuring the driving voltage, light emitting efficiency, color coordinate (CIE) and service life of the organic light emitting device manufactured according to the present invention are shown in the following Table 16.

**[Table 16]**

| | Light emitting layer Compound | Ratio | Driving voltage (V) | Efficiency (cd/A) | Color coordinate (x, y) | Service life (T₉₀) |
|---|---|---|---|---|---|---|
| Comparative Example 25 | 3-1 : 2-53 | 1 : 1 | 4.03 | 63.2 | (0.245, 0.633) | 185 |
| Comparative Example 26 | | 1 : 2 | 4.15 | 62.5 | (0.266, 0.621) | 193 |
| Comparative Example 27 | | 1 : 3 | 4.22 | 60.3 | (0.253, 0.694) | 203 |
| Comparative Example 28 | 3-2 : 2-62 | 1 : 1 | 4.31 | 70.2 | (0.245, 0.675) | 163 |
| Comparative Example 29 | | 1 : 2 | 4.35 | 69.3 | (0.257, 0.643) | 172 |
| Comparative Example 30 | | 1 : 3 | 4.42 | 68.7 | (0.264, 0.642) | 183 |
| Comparative Example 31 | 3-3 : 2-56 | 1 : 1 | 4.25 | 71.3 | (0.257, 0.671) | 171 |
| Comparative Example 32 | | 1 : 2 | 4.36 | 70.5 | (0.237, 0.645) | 173 |
| Comparative Example 33 | | 1 : 3 | 4.39 | 69.8 | (0.236, 0.624) | 182 |
| Comparative Example 34 | 3-4 : 2-50 | 1 : 1 | 4.28 | 74.9 | (0.255, 0.627) | 204 |
| Comparative Example 35 | | 1 : 2 | 4.35 | 74.1 | (0.252, 0.623) | 213 |
| Comparative Example 36 | | 1 : 3 | 4.41 | 73.2 | (0.257, 0.714) | 224 |
| Comparative Example 37 | 3-5 : 2-53 | 1 : 1 | 4.15 | 70.1 | (0.244, 0.645) | 175 |
| Comparative Example 38 | | 1 : 2 | 4.26 | 69.2 | (0.261, 0.762) | 183 |
| Comparative Example 39 | | 1 : 3 | 4.33 | 68.3 | (0.254, 0.623) | 191 |
| Comparative Example 40 | 3-6 : 2-62 | 1 : 1 | 4.22 | 66.2 | (0.254, 0.657) | 230 |
| Comparative Example 41 | | 1 : 2 | 4.28 | 65.5 | (0.277, 0.655) | 237 |
| Comparative Example 42 | | 1 : 3 | 4.31 | 64.7 | (0.263, 0.642) | 241 |
| Comparative Example 43 | 3-7 : 2-51 | 1 : 1 | 4.02 | 72.2 | (0.256, 0.633) | 236 |
| Comparative Example 44 | | 1 : 2 | 4.11 | 71.3 | (0.258, 0.632) | 244 |
| Comparative Example 45 | | 1 : 3 | 4.19 | 70.5 | (0.251, 0.684) | 251 |
| Comparative Example 46 | 3-7 : 2-75 | 1 : 1 | 4.13 | 67.0 | (0.235, 0.653) | 193 |
| Comparative Example 47 | | 1 : 2 | 4.23 | 66.2 | (0.236, 0.628) | 201 |
| Comparative Example 48 | | 1 : 3 | 4.32 | 65.9 | (0.258, 0.691) | 213 |
| Comparative Example 49 | 3-8 : 2-62 | 1 : 1 | 4.05 | 71.3 | (0.261, 0.740) | 189 |
| Comparative Example 50 | | 1 : 2 | 4.13 | 70.2 | (0.233, 0.623) | 199 |
| Comparative Example 51 | | 1 : 3 | 4.22 | 69.3 | (0.248, 0.644) | 203 |
| Comparative Example 52 | 3-9 : 2-53 | 1 : 1 | 4.32 | 73.9 | (0.257, 0.629) | 215 |
| Comparative Example 53 | | 1 : 2 | 4.39 | 73.5 | (0.254, 0.726) | 223 |
| Comparative Example 54 | | 1 : 3 | 4.41 | 72.2 | (0.245, 0.625) | 233 |
| Comparative Example 55 | 3-10 : 2-62 | 1 : 1 | 4.55 | 73.6 | (0.263, 0.732) | 228 |
| Comparative Example 56 | | 1 : 2 | 4.59 | 72.8 | (0.257, 0.653) | 231 |
| Comparative Example 57 | | 1 : 3 | 4.62 | 71.3 | (0.285, 0.659) | 235 |
| Comparative Example 58 | 3-11 : 2-60 | 1 : 1 | 4.37 | 71.3 | (0.268, 0.655) | 246 |
| Comparative Example 59 | | 1 : 2 | 4.41 | 70.1 | (0.287, 0.673) | 249 |
| Comparative Example 60 | | 1 : 3 | 4.49 | 69.5 | (0.254, 0.626) | 256 |
| Comparative Example 61 | 3-12 : 2-51 | 1 : 1 | 4.33 | 69.3 | (0.279, 0.657) | 189 |
| Comparative Example 62 | | 1 : 2 | 4.38 | 68.8 | (0.268, 0.686) | 193 |
| Comparative Example 63 | | 1 : 3 | 4.42 | 68.2 | (0.274, 0.665) | 211 |
| Comparative Example 64 | 3-13 : 2-68 | 1 : 1 | 4.11 | 67.5 | (0.263, 0.672) | 173 |
| Comparative Example 65 | | 1 : 2 | 4.25 | 66.3 | (0.262, 0.654) | 182 |
| Comparative Example 66 | | 1 : 3 | 4.29 | 65.2 | (0.263, 0.676) | 195 |
| Comparative Example 67 | 3-14 : 2-68 | 1 : 1 | 4.13 | 66.5 | (0.257, 0.645) | 177 |
| Comparative Example 68 | | 1 : 2 | 4.21 | 65.2 | (0.263, 0.625) | 182 |
| Comparative Example 69 | | 1 : 3 | 4.25 | 64.2 | (0.256, 0.625) | 188 |
| Comparative Example 70 | 3-15 : 2-51 | 1 : 1 | 4.52 | 70.5 | (0.254, 0.723) | 177 |
| Comparative Example 71 | | 1 : 2 | 4.55 | 69.2 | (0.247, 0.632) | 182 |
| Comparative Example 72 | | 1 : 3 | 4.60 | 68.3 | (0.246, 0.623) | 188 |
| Comparative Example 73 | 3-16 : 2-53 | 1 : 1 | 4.26 | 75.0 | (0.245, 0.655) | 169 |
| Comparative Example 74 | | 1 : 2 | 4.29 | 74.2 | (0.258, 0.613) | 172 |
| Comparative Example 75 | | 1 : 3 | 4.30 | 73.7 | (0.255, 0.694) | 183 |
| Comparative Example 76 | 3-16 : 2-76 | 1 : 1 | 4.33 | 71.3 | (0.247, 0.625) | 165 |
| Comparative Example 77 | | 1 : 2 | 4.41 | 70.5 | (0.248, 0.625) | 171 |
| Comparative Example 78 | | 1 : 3 | 4.45 | 69.3 | (0.254, 0.623) | 177 |
| Comparative Example 79 | 3-17 : 2-62 | 1 : 1 | 5.29 | 57.3 | (0.255, 0.693) | 155 |
| Comparative Example 80 | | 1 : 2 | 5.31 | 56.7 | (0.247, 0.629) | 159 |
| Comparative Example 81 | | 1 : 3 | 5.33 | 55.6 | (0.258, 0.703) | 162 |
| Comparative Example 82 | 3-18 : 3-53 | 1 : 1 | 5.36 | 56.9 | (0.247, 0.652) | 128 |
| Comparative Example 83 | | 1 : 2 | 5.44 | 55.8 | (0.246, 0.623) | 135 |
| Comparative Example 84 | | 1 : 3 | 5.58 | 54.2 | (0.247, 0.665) | 144 |
| Comparative Example 85 | 3-19 : 2-51 | 1 : 1 | 5.63 | 59.1 | (0.251, 0.623) | 138 |
| Comparative Example 86 | | 1 : 2 | 5.71 | 58.3 | (0.245, 0.659) | 142 |
| Comparative Example 87 | | 1 : 3 | 5.77 | 57.6 | (0.254, 0.623) | 159 |
| Comparative Example 88 | 3-20 : 2-53 | 1 : 1 | 5.13 | 55.7 | (0.248, 0.651) | 129 |
| Comparative Example 89 | | 1 : 2 | 5.26 | 54.3 | (0.244, 0.654) | 137 |
| Comparative Example 90 | | 1 : 3 | 5.31 | 53.8 | (0.257, 0.693) | 140 |
| Comparative Example 91 | 3-21 : 2-51 | 1 : 1 | 5.66 | 50.3 | (0.248, 0.657) | 133 |
| Comparative Example 92 | | 1 : 2 | 5.71 | 49.5 | (0.253, 0.667) | 146 |
| Comparative Example 93 | | 1 : 3 | 5.83 | 48.7 | (0.266, 0.655) | 150 |
| Comparative Example 94 | 3-22 : 2-56 | 1 : 1 | 5.72 | 49.5 | (0.257, 0.638) | 126 |
| Comparative Example 95 | | 1 : 2 | 5.83 | 48.7 | (0.257, 0.633) | 132 |
| Comparative Example 96 | | 1 : 3 | 5.91 | 47.6 | (0.251, 0.673) | 139 |
| Comparative Example 97 | 3-23 : 2-68 | 1 : 1 | 5.50 | 48.3 | (0.255, 0.724) | 122 |
| Comparative Example 98 | | 1 : 2 | 5.63 | 47.6 | (0.249, 0.662) | 139 |
| Comparative Example 99 | | 1 : 3 | 5.71 | 47.2 | (0.257, 0.633) | 143 |
| Comparative Example 100 | 3-23 : 2-53 | 1 : 1 | 5.63 | 46.5 | (0.268, 0.617) | 118 |
| Comparative Example 101 | | 1 : 2 | 5.68 | 45.2 | (0.252, 0.622) | 120 |
| Comparative Example 102 | | 1 : 3 | 5.77 | 44.8 | (0.252, 0.613) | 123 |
| Comparative Example 103 | 3-24 : 2-60 | 1 : 1 | 5.46 | 49.7 | (0.255, 0.622) | 139 |
| Comparative Example 104 | | 1 : 2 | 5.53 | 48.3 | (0.258, 0.734) | 143 |
| Comparative Example 105 | | 1 : 3 | 5.59 | 47.6 | (0.241, 0.625) | 147 |
| Example 57 | 1-1 : 2-51 | 1 : 1 | 2.13 | 145.3 | (0.251, 0.723) | 487 |
| Example 58 | | 1 : 2 | 2.23 | 144.3 | (0.247, 0.645) | 491 |
| Example 59 | | 1 : 3 | 2.31 | 143.1 | (0.263, 0.625) | 499 |
| Example 60 | | 1 : 4 | 2.44 | 140.6 | (0.258, 0.637) | 501 |
| Example 61 | | 1 : 5 | 2.53 | 138.2 | (0.262, 0.635) | 503 |
| Example 62 | 1-1 : 2-75 | 1 : 1 | 2.42 | 140.3 | (0.257, 0.624) | 473 |
| Example 63 | | 1 : 2 | 2.45 | 139.8 | (0.255, 0.657) | 482 |
| Example 64 | | 1 : 3 | 2.53 | 138.4 | (0.256, 0.645) | 489 |
| Example 65 | 1-2 : 2-51 | 1 : 1 | 2.37 | 138.8 | (0.255, 0.674) | 453 |
| Example 66 | | 1 : 2 | 2.44 | 137.2 | (0.244, 0.656) | 469 |
| Example 67 | | 1 : 3 | 2.53 | 136.4 | (0.257, 0.633) | 477 |
| Example 68 | | 1 : 4 | 2.66 | 133.2 | (0.268, 0.651) | 478 |
| Example 69 | | 1 : 5 | 2.71 | 130.1 | (0.243, 0.657) | 480 |
| Example 70 | 1-3 : 2-56 | 1 : 1 | 2.33 | 140.6 | (0.268, 0.625) | 477 |
| Example 71 | | 1 : 2 | 2.41 | 139.7 | (0.257, 0.628) | 483 |
| Example 72 | | 1 : 3 | 2.43 | 138.2 | (0.256, 0.723) | 492 |
| Example 73 | | 1 : 4 | 2.56 | 132.6 | (0.244, 0.637) | 495 |
| Example 74 | | 1 : 5 | 2.57 | 130.2 | (0.256, 0.658) | 497 |
| Example 75 | 1-3 : 2-77 | 1 : 1 | 2.13 | 133.2 | (0.243, 0.652) | 413 |
| Example 76 | | 1 : 2 | 2.25 | 132.7 | (0.267, 0.669) | 429 |
| Example 77 | | 1 : 3 | 2.37 | 131.4 | (0.259, 0.627) | 440 |
| Example 78 | 1-4 : 2-68 | 1 : 1 | 2.33 | 142.5 | (0.248, 0.654) | 423 |
| Example 79 | | 1 : 2 | 2.42 | 141.3 | (0.241, 0.654) | 438 |
| Example 80 | | 1 : 3 | 2.53 | 140.2 | (0.274, 0.658) | 446 |
| Example 81 | | 1 : 4 | 2.66 | 137.2 | (0.267, 0.623) | 448 |
| Example 82 | | 1 : 5 | 2.70 | 136.6 | (0.251, 0.656) | 449 |
| Example 83 | 1-7 : 2-62 | 1 : 1 | 2.41 | 143.7 | (0.263, 0.623) | 406 |
| Example 84 | | 1 : 2 | 2.53 | 142.5 | (0.254, 0.673) | 413 |
| Example 85 | | 1 : 3 | 2.64 | 141.8 | (0.245, 0.647) | 432 |
| Example 86 | | 1 : 4 | 2.65 | 138.5 | (0.264, 0.658) | 435 |
| Example 87 | | 1 : 5 | 2.68 | 137.2 | (0.267, 0.625) | 439 |
| Example 88 | 1-10 : 2-56 | 1 : 1 | 2.78 | 138.7 | (0.245, 0.623) | 419 |
| Example 89 | | 1 : 2 | 2.83 | 137.5 | (0.257, 0.657) | 428 |
| Example 90 | | 1 : 3 | 2.88 | 136.4 | (0.258, 0.622) | 436 |
| Example 91 | | 1 : 4 | 2.92 | 133.4 | (0.265, 0.623) | 438 |
| Example 92 | | 1 : 5 | 2.95 | 132.8 | (0.253, 0.656) | 440 |
| Example 93 | 1-10 : 2-77 | 1 : 1 | 2.63 | 138.8 | (0.258, 0.714) | 400 |
| Example 94 | | 1 : 2 | 2.69 | 137.1 | (0.244, 0.627) | 405 |
| Example 95 | | 1 : 3 | 2.71 | 136.0 | (0.269, 0.613) | 411 |
| Example 96 | 1-13 : 2-60 | 1 : 1 | 2.16 | 135.7 | (0.250, 0.653) | 436 |
| Example 97 | | 1 : 2 | 2.23 | 134.1 | (0.242, 0.667) | 448 |
| Example 98 | | 1 : 3 | 2.28 | 133.9 | (0.258, 0.632) | 456 |
| Example 99 | | 1 : 4 | 2.30 | 130.8 | (0.236, 0.621) | 458 |
| Example 100 | | 1 : 5 | 2.31 | 130.1 | (0.245, 0.622) | 461 |
| Example 101 | 1-19 : 2-60 | 1 : 1 | 2.26 | 138.6 | (0.254, 0.646) | 463 |
| Example 102 | | 1 : 2 | 2.38 | 137.1 | (0.256, 0.634) | 471 |
| Example 103 | | 1 : 3 | 2.48 | 135.5 | (0.253, 0.641) | 483 |
| Example 104 | | 1 : 4 | 2.55 | 132.6 | (0.253, 0.652) | 485 |
| Example 105 | | 1 : 5 | 2.57 | 130.7 | (0.247, 0.665) | 488 |
| Example 106 | 1-22 : 2-62 | 1 : 1 | 2.13 | 141.2 | (0.258, 0.673) | 429 |
| Example 107 | | 1 : 2 | 2.28 | 140.3 | (0.235, 0.645) | 433 |
| Example 108 | | 1 : 3 | 2.36 | 138.9 | (0.237, 0.624) | 448 |
| Example 109 | | 1 : 4 | 2.38 | 135.2 | (0.255, 0.622) | 450 |
| Example 110 | | 1 : 5 | 2.40 | 134.7 | (0.257, 0.642) | 451 |
| Example 111 | 1-31 : 2-60 | 1 : 1 | 2.43 | 142.6 | (0.244, 0.603) | 403 |
| Example 112 | | 1 : 2 | 2.46 | 141.0 | (0.253, 0.620) | 419 |
| Example 113 | | 1 : 3 | 2.55 | 138.1 | (0.255, 0.613) | 432 |
| Example 114 | | 1 : 4 | 2.56 | 135.6 | (0.256, 0.625) | 435 |
| Example 115 | | 1 : 5 | 2.58 | 132.7 | (0.257, 0.647) | 436 |
| Example 116 | 1-37 : 2-53 | 1 : 1 | 2.16 | 143.5 | (0.242, 0.624) | 422 |
| Example 117 | | 1 : 2 | 2.28 | 141.8 | (0.267, 0.734) | 439 |
| Example 118 | | 1 : 3 | 2.33 | 140.7 | (0.253, 0.627) | 442 |
| Example 119 | | 1 : 4 | 2.40 | 138.2 | (0.265, 0.623) | 445 |
| Example 120 | | 1 : 5 | 2.42 | 137.9 | (0.258, 0.627) | 447 |
| Example 121 | 1-37 : 2-76 | 1 : 1 | 2.38 | 139.5 | (0.256, 0.659) | 419 |
| Example 122 | | 1 : 2 | 2.41 | 138.2 | (0.275, 0.666) | 426 |
| Example 123 | | 1 : 3 | 2.48 | 137.1 | (0.266, 0.628) | 438 |
| Example 124 | 1-40 : 2-33 | 1 : 1 | 2.22 | 143.2 | (0.255, 0.625) | 416 |
| Example 125 | | 1 : 2 | 2.31 | 142.8 | (0.258, 0.617) | 428 |
| Example 126 | | 1 : 3 | 2.48 | 141.3 | (0.257, 0.623) | 433 |
| Example 127 | | 1 : 4 | 2.50 | 138.7 | (0.268, 0.627) | 435 |
| Example 128 | | 1 : 5 | 2.51 | 137.2 | (0.256, 0.657) | 436 |
| Example 129 | 1-44 : 2-60 | 1 : 1 | 2.38 | 145.5 | (0.247, 0.625) | 423 |
| Example 130 | | 1 : 2 | 2.43 | 143.2 | (0.265, 0.724) | 442 |
| Example 131 | | 1 : 3 | 2.49 | 142.1 | (0.255, 0.623) | 453 |
| Example 132 | | 1 : 4 | 2.50 | 140.6 | (0.254, 0.663) | 455 |
| Example 133 | | 1 : 5 | 2.52 | 138.7 | (0.265, 0.650) | 456 |
| Example 134 | 1-53 : 2-68 | 1 : 1 | 2.56 | 144.5 | (0.685, 0.652) | 429 |
| Example 135 | | 1 : 2 | 2.63 | 143.2 | (0.257, 0.673) | 433 |
| Example 136 | | 1 : 3 | 2.73 | 142.3 | (0.269, 0.651) | 442 |
| Example 137 | | 1 : 4 | 2.74 | 140.2 | (0.256, 0.623) | 445 |
| Example 138 | | 1 : 5 | 2.77 | 138.3 | (0.257, 0.663) | 446 |
| Example 139 | 1-57 : 2-50 | 1 : 1 | 2.46 | 138.5 | (0.286, 0.674) | 468 |
| Example 140 | | 1 : 2 | 2.53 | 137.4 | (0.271, 0.675) | 479 |
| Example 141 | | 1 : 3 | 2.63 | 135.5 | (0.274, 0.673) | 481 |
| Example 142 | | 1 : 4 | 2.66 | 130.3 | (0.267, 0.663) | 483 |
| Example 143 | | 1 : 5 | 2.67 | 130.0 | (0.286, 0.654) | 484 |
| Example 144 | 1-57 : 2-78 | 1 : 1 | 2.80 | 142.2 | (0.266, 0.671) | 446 |
| Example 145 | | 1 : 2 | 2.83 | 141.3 | (0.263, 0.655) | 453 |
| Example 146 | | 1 : 3 | 2.91 | 140.9 | (0.258, 0.665) | 462 |
| Example 147 | 1-63 : 2-56 | 1 : 1 | 2.34 | 143.7 | (0.273, 0.672) | 477 |
| Example 148 | | 1 : 2 | 2.49 | 141.9 | (0.270, 0.676) | 483 |
| Example 149 | | 1 : 3 | 2.53 | 140.2 | (0.286, 0.674) | 498 |
| Example 150 | | 1 : 4 | 2.55 | 138.1 | (0.257, 0.624) | 500 |
| Example 151 | | 1 : 5 | 2.56 | 137.6 | (0.254, 0.628) | 503 |
| Example 152 | 1-63 : 2-77 | 1 : 1 | 2.61 | 137.6 | (0.255, 0.633) | 423 |
| Example 153 | | 1 : 2 | 2.68 | 136.4 | (0.256, 0.683) | 436 |
| Example 154 | | 1 : 3 | 2.72 | 135.2 | (0.235, 0.644) | 449 |
| Example 155 | 1-71 : 2-51 | 1 : 1 | 2.41 | 133.9 | (0.235, 0.654) | 478 |
| Example 156 | | 1 : 2 | 2.53 | 132.1 | (0.245, 0.639) | 483 |
| Example 157 | | 1 : 3 | 2.68 | 131.7 | (0.258, 0.614) | 491 |
| Example 158 | | 1 : 4 | 2.70 | 128.6 | (0.256, 0.624) | 493 |
| Example 159 | | 1 : 5 | 2.71 | 128.3 | (0.255, 0.630) | 499 |
| Example 160 | 1-71 : 2-75 | 1 : 1 | 2.33 | 130.3 | (0.253, 0.713) | 453 |
| Example 161 | | 1 : 2 | 2.47 | 128.7 | (0.247, 0.623) | 462 |
| Example 162 | | 1 : 3 | 2.53 | 124.5 | (0.258, 0.629) | 473 |
| Example 163 | 1-80 : 2-53 | 1 : 1 | 2.06 | 133.9 | (0.254, 0.624) | 438 |
| Example 164 | | 1 : 2 | 2.18 | 132.1 | (0.244, 0.624) | 442 |
| Example 165 | | 1 : 3 | 2.23 | 131.7 | (0.268, 0.623) | 456 |
| Example 166 | | 1 : 4 | 2.35 | 129.3 | (0.245, 0.641) | 457 |
| Example 167 | | 1 : 5 | 2.36 | 128.7 | (0.234, 0.653) | 459 |
| Example 168 | 1-83 : 2-60 | 1 : 1 | 2.11 | 144.9 | (0.259, 0.623) | 426 |
| Example 169 | | 1 : 2 | 2.23 | 143.1 | (0.645, 0.648) | 438 |
| Example 170 | | 1 : 3 | 2.38 | 142.2 | (0.253, 0.683) | 447 |
| Example 171 | | 1 : 4 | 2.40 | 140.5 | (0.254, 0.624) | 449 |
| Example 172 | | 1 : 5 | 2.42 | 139.8 | (0.252, 0.623) | 450 |
| Example 173 | 1-87 : 2-60 | 1 : 1 | 2.26 | 145.0 | (0.269, 0.659) | 453 |
| Example 174 | | 1 : 2 | 2.37 | 144.6 | (0.289, 0.674) | 462 |
| Example 175 | | 1 : 3 | 2.44 | 142.8 | (0.251, 0.675) | 478 |
| Example 176 | | 1 : 4 | 2.45 | 140.6 | (0.264, 0.624) | 479 |
| Example 177 | | 1 : 5 | 2.47 | 139.9 | (0.252, 0.627) | 483 |
| Example 178 | 1-93 : 2-62 | 1 : 1 | 2.39 | 140.9 | (0.264, 0.675) | 468 |
| Example 179 | | 1 : 2 | 2.41 | 138.7 | (0.268, 0.657) | 473 |
| Example 180 | | 1 : 3 | 2.46 | 136.1 | (0.265, 0.643) | 482 |
| Example 181 | | 1 : 4 | 2.47 | 135.5 | (0.253, 0.676) | 485 |
| Example 182 | | 1 : 5 | 2.49 | 135.2 | (0.264, 0.623) | 488 |
| Example 183 | 1-94 : 2-53 | 1 : 1 | 2.33 | 133.8 | (0.258, 0.645) | 458 |
| Example 184 | | 1 : 2 | 2.49 | 132.5 | (0.253, 0.661) | 462 |
| Example 185 | | 1 : 3 | 2.57 | 131.1 | (0.257, 0.688) | 474 |
| Example 186 | | 1 : 4 | 2.58 | 130.6 | (0.264, 0.643) | 475 |
| Example 187 | | 1 : 5 | 2.60 | 129.3 | (0.259, 0.676) | 477 |
| Example 188 | 1-94 : 2-76 | 1 : 1 | 2.13 | 132.4 | (0.253, 0.627) | 413 |
| Example 189 | | 1 : 2 | 2.28 | 130.8 | (0.235, 0.618) | 426 |
| Example 190 | | 1 : 3 | 2.33 | 129.1 | (0.247, 0.623) | 438 |
| Example 191 | 1-95 : 2-56 | 1 : 1 | 2.26 | 136.5 | (0.258, 0.639) | 469 |
| Example 192 | | 1 : 2 | 2.37 | 134.8 | (0.253, 0.733) | 472 |
| Example 193 | | 1 : 3 | 2.43 | 133.2 | (0.243, | 483 |
| | | | | | 0.629) | |
| Example 194 | | 1 : 4 | 2.44 | 130.3 | (0.255, 0.627) | 485 |
| Example 195 | | 1 : 5 | 2.48 | 129.7 | (0.235, 0.628) | 488 |
| Example 196 | 1-95 : 2-77 | 1 : 1 | 2.36 | 136.5 | (0.263, 0.634) | 423 |
| Example 197 | | 1 : 2 | 2.41 | 135.1 | (0.255, 0.625) | 436 |
| Example 198 | | 1 : 3 | 2.59 | 134.8 | (0.254, 0.669) | 442 |
| Example 199 | 1-99 : 2-33 | 1 : 1 | 2.46 | 139.5 | (0.277, 0.686) | 436 |
| Example 200 | | 1 : 2 | 2.53 | 138.4 | (0.264, 0.624) | 448 |
| Example 201 | | 1 : 3 | 2.61 | 137.1 | (0.254, 0.623) | 453 |
| Example 202 | | 1 : 4 | 2.66 | 135.7 | (0.258, 0.659) | 455 |
| Example 203 | | 1 : 5 | 2.73 | 132.6 | (0.274, 0.689) | 459 |
| Example 204 | 1-103 : 2-51 | 1 : 1 | 2.37 | 135.9 | (0.257, 0.625) | 432 |
| Example 205 | | 1 : 2 | 2.41 | 134.1 | (0.243, 0.635) | 444 |
| Example 206 | | 1 : 3 | 2.53 | 133.6 | (0.247, 0.627) | 459 |
| Example 207 | | 1 : 4 | 2.55 | 132.8 | (0.255, 0.667) | 460 |
| Example 208 | | 1 : 5 | 2.59 | 132.1 | (0.273, 0.684) | 462 |
| Example 209 | 1-105 : 2-60 | 1 : 1 | 2.08 | 143.2 | (0.655, 0.254) | 413 |
| Example 210 | | 1 : 2 | 2.13 | 141.5 | (0.579, 0.358) | 426 |
| Example 211 | | 1 : 3 | 2.29 | 140.6 | (0.612, 0.376) | 438 |
| Example 212 | | 1 : 4 | 2.30 | 139.6 | (0.650, 0.256) | 440 |
| Example 213 | | 1 : 5 | 2.33 | 138.8 | (0.578, 0.354) | 442 |
| Example 214 | 1-107 : 2-62 | 1 : 1 | 2.13 | 144.7 | (0.255, 0.677) | 435 |
| Example 215 | | 1 : 2 | 2.23 | 143.1 | (0.266, 0.653) | 449 |
| Example 216 | | 1 : 3 | 2.34 | 141.8 | (0.265, 0.739) | 451 |
| Example 217 | | 1 : 4 | 4.36 | 140.6 | (0.576, 0.354) | 453 |
| Example 218 | | 1 : 5 | 4.38 | 139.2 | (0.255, 0.673) | 455 |
| Example 219 | 1-112 : 2-50 | 1 : 1 | 3.12 | 132.5 | (0.257, 0.654) | 387 |
| Example 220 | | 1 : 2 | 3.26 | 131.7 | (0.683, 0.643) | 392 |
| Example 221 | | 1 : 3 | 3.37 | 130.5 | (0.267, 0.625) | 399 |
| Example 222 | | 1 : 4 | 3.44 | 129.6 | (0.243, 0.624) | 401 |
| Example 223 | | 1 : 5 | 3.46 | 129.2 | (0.264, 0.708) | 403 |
| Example 224 | 1-112 : 2-76 | 1 : 1 | 3.23 | 130.8 | (0.267, 0.624) | 354 |
| Example 225 | | 1 : 2 | 3.31 | 129.7 | (0.276, 0.613) | 362 |
| Example 226 | | 1 : 3 | 3.35 | 129.1 | (0.254, 0.626) | 366 |
| Example 227 | 1-115 : 2-60 | 1 : 1 | 3.16 | 126.5 | (0.249, 0.628) | 374 |
| Example 228 | | 1 : 2 | 3.27 | 125.3 | (0.266, 0.758) | 382 |
| Example 229 | | 1 : 3 | 3.33 | 124.8 | (0.253, 0.635) | 388 |
| Example 230 | | 1 : 4 | 3.40 | 123.6 | (0.275, 0.659) | 390 |
| Example 231 | | 1 : 5 | 3.42 | 121.7 | (0.261, 0.627) | 391 |
| Example 232 | 1-121 : 2-62 | 1 : 1 | 3.11 | 131.6 | (0.642, 0.645) | 364 |
| Example 233 | | 1 : 2 | 3.23 | 130.4 | (0.256, 0.674) | 372 |
| Example 234 | | 1 : 3 | 3.34 | 128.6 | (0.268, 0.666) | 384 |
| Example 235 | | 1 : 4 | 3.40 | 128.3 | (0.263, 0.627) | 388 |
| Example 236 | | 1 : 5 | 3.42 | 127.7 | (0.642, 0.644) | 391 |
| Example 237 | 1-130 : 2-56 | 1 : 1 | 3.26 | 133.5 | (0.281, 0.674) | 355 |
| Example 238 | | 1 : 2 | 3.31 | 132.7 | (0.274, 0.645) | 361 |
| Example 239 | | 1 : 3 | 3.33 | 131.2 | (0.267, 0.677) | 369 |
| Example 240 | | 1 : 4 | 3.39 | 130.6 | (0.644, 0.647) | 371 |
| Example 241 | | 1 : 5 | 3.40 | 129.7 | (0.273, 0.670) | 373 |
| Example 242 | 1-142 : 2-68 | 1 : 1 | 3.29 | 134.5 | (0.268, 0.657) | 374 |
| Example 243 | | 1 : 2 | 3.35 | 133.7 | (0.265, 0.644) | 382 |
| Example 244 | | 1 : 3 | 3.39 | 132.5 | (0.259, 0.647) | 388 |
| Example 245 | | 1 : 4 | 3.41 | 132.0 | (0.254, 0.675) | 390 |
| Example 246 | | 1 : 5 | 3.44 | 131.9 | (0.257, 0.644) | 392 |
| Example 247 | 1-145 : 2-60 | 1 : 1 | 2.63 | 128.6 | (0.245, 0.645) | 365 |
| Example 248 | | 1 : 2 | 2.71 | 127.1 | (0.274, 0.659) | 371 |
| Example 249 | | 1 : 3 | 2.77 | 126.5 | (0.261, 0.623) | 378 |
| Example 250 | | 1 : 4 | 2.79 | 125.7 | (0.256, 0.645) | 380 |
| Example 251 | | 1 : 5 | 2.80 | 125.5 | (0.253, 0.665) | 383 |
| Example 252 | 1-145 : 2-77 | 1 : 1 | 2.51 | 123.4 | (0.253, 0.675) | 360 |
| Example 253 | | 1 : 2 | 2.55 | 122.8 | (0.247, 0.644) | 362 |
| Example 254 | | 1 : 3 | 2.69 | 121.6 | (0.248, 0.612) | 366 |
| Example 255 | 1-156 : 2-62 | 1 : 1 | 2.63 | 130.8 | (0.257, 0.654) | 379 |
| Example 256 | | 1 : 2 | 2.71 | 129.4 | (0.258, 0.612) | 382 |
| Example 257 | | 1 : 3 | 2.83 | 128.7 | (0.256, 0.675) | 390 |
| Example 258 | | 1 : 4 | 2.88 | 127.3 | (0.255, 0.674) | 393 |
| Example 259 | | 1 : 5 | 2.91 | 125.6 | (0.246, 0.643) | 395 |
| Example 260 | 1-164 : 2-56 | 1 : 1 | 2.70 | 127.4 | (0.259, 0.663) | 387 |
| Example 261 | | 1 : 2 | 2.76 | 126.2 | (0.256, 0.684) | 392 |
| Example 262 | | 1 : 3 | 2.83 | 125.5 | (0.263, 0.648) | 400 |
| Example 263 | | 1 : 4 | 2.84 | 124.9 | (0.243, 0.621) | 402 |
| Example 264 | | 1 : 5 | 2.86 | 124.1 | (0.255, 0.623) | 404 |
| Example 265 | 1-172 : 2-56 | 1 : 1 | 3.12 | 131.7 | (0.264, 0.625) | 366 |
| Example 266 | | 1 : 2 | 3.26 | 130.5 | (0.245, 0.623) | 372 |
| Example 267 | | 1 : 3 | 3.37 | 129.4 | (0.255, 0.624) | 383 |
| Example 268 | | 1 : 4 | 3.39 | 128.2 | (0.259, 0.675) | 388 |
| Example 269 | | 1 : 5 | 3.41 | 127.7 | (0.243, 0.647) | 390 |
| Example 270 | 1-172 : 2-77 | 1 : 1 | 3.28 | 132.4 | (0.262, 0.721) | 361 |
| Example 271 | | 1 : 2 | 3.33 | 131.2 | (0.245, 0.628) | 366 |
| Example 272 | | 1 : 3 | 3.41 | 130.8 | (0.248, 0.623) | 372 |
| Example 273 | 1-176 : 2-62 | 1 : 1 | 3.36 | 134.7 | (0.264, 0.693) | 384 |
| Example 274 | | 1 : 2 | 3.44 | 133.5 | (0.253, 0.629) | 388 |
| Example 275 | | 1 : 3 | 3.52 | 132.4 | (0.685, 0.623) | 394 |
| Example 276 | | 1 : 4 | 3.55 | 131.6 | (0.258, 0.675) | 395 |
| Example 277 | | 1 : 5 | 3.56 | 130.7 | (0.243, 0.645) | 399 |
| Example 278 | 1-187 : 2-56 | 1 : 1 | 3.28 | 135.6 | (0.264, 0.665) | 370 |
| Example 279 | | 1 : 2 | 3.37 | 134.2 | (0.269, 0.645) | 382 |
| Example 280 | | 1 : 3 | 3.42 | 133.8 | (0.284, 0.669) | 393 |
| Example 281 | | 1 : 4 | 3.44 | 133.7 | (0.256, 0.675) | 399 |
| Example 282 | | 1 : 5 | 3.46 | 132.6 | (0.243, 0.643) | 402 |
| Example 283 | 1-200 : 2-62 | 1 : 1 | 2.89 | 132.4 | (0.273, 0.661) | 364 |
| Example 284 | | 1 : 2 | 2.93 | 131.6 | (0.248, 0.623) | 378 |
| Example 285 | | 1 : 3 | 3.05 | 130.7 | (0.645, 0.658) | 385 |
| Example 286 | | 1 : 4 | 3.11 | 129.6 | (0.267, 0.691) | 388 |
| Example 287 | | 1 : 5 | 3.13 | 128.8 | (0.258, 0.624) | 390 |
| Example 288 | 1-203 : 2-53 | 1 : 1 | 2.77 | 135.4 | (0.257, 0.675) | 372 |
| Example 289 | | 1 : 2 | 2.83 | 134.6 | (0.259, 0.657) | 384 |
| Example 290 | | 1 : 3 | 2.95 | 133.7 | (0.284, 0.674) | 390 |
| Example 291 | | 1 : 4 | 2.99 | 133.1 | (0.255, 0.653) | 393 |
| Example 292 | | 1 : 5 | 3.01 | 132.6 | (0.257, 0.647) | 395 |
| Example 293 | 1-203 : 2-76 | 1 : 1 | 2.63 | 126.5 | (0.273, 0.671) | 362 |
| Example 294 | | 1 : 2 | 2.75 | 125.4 | (0.265, 0.674) | 366 |
| Example 295 | | 1 : 3 | 2.84 | 124.6 | (0.258, 0.663) | 374 |
| Example 296 | 1-212 : 2-56 | 1 : 1 | 2.13 | 133.9 | (0.256, 0.653) | 387 |
| Example 297 | | 1 : 2 | 2.26 | 132.7 | (0.257, 0.645) | 394 |
| Example 298 | | 1 : 3 | 2.39 | 131.8 | (0.235, 0.618) | 400 |
| Example 299 | | 1 : 4 | 3.41 | 130.6 | (0.266, 0.674) | 402 |
| Example 300 | | 1 : 5 | 3.44 | 130.0 | (0.254, 0.653) | 404 |
| Example 301 | 1-212 : 2-77 | 1 : 1 | 2.63 | 126.8 | (0.247, 0.653) | 354 |
| Example 302 | | 1 : 2 | 2.66 | 125.4 | (0.257, 0.675) | 366 |
| Example 303 | | 1 : 3 | 2.71 | 124.1 | (0.257, 0.724) | 378 |
| Example 304 | 1-216 : 2-50 | 1 : 1 | 3.56 | 115.0 | (0.243, 0.639) | 342 |
| Example 305 | | 1 : 2 | 3.64 | 113.2 | (0.263, 0.436) | 350 |
| Example 306 | | 1 : 3 | 3.69 | 110.9 | (0.255, 0.625) | 355 |
| Example 307 | | 1 : 4 | 3.71 | 110.5 | (0.276, 0.651) | 357 |
| Example 308 | | 1 : 5 | 3.73 | 109.7 | (0.264, 0.624) | 359 |
| Example 309 | 1-216 : 2-75 | 1 : 1 | 3.62 | 113.6 | (0.255, 0.669) | 326 |
| Example 310 | | 1 : 2 | 3.74 | 112.0 | (0.245, 0.651) | 337 |
| Example 311 | | 1 : 3 | 3.82 | 110.7 | (0.247, 0.667) | 349 |
| Example 312 | 1-236 : 2-56 | 1 : 1 | 3.77 | 112.5 | (0.277, 0.652) | 348 |
| Example 313 | | 1 : 2 | 3.80 | 111.4 | (0.265, 0.623) | 352 |
| Example 314 | | 1 : 3 | 3.83 | 110.9 | (0.254, 0.678) | 369 |
| Example 315 | | 1 : 4 | 3.85 | 109.7 | (0.265, 0.652) | 371 |
| Example 316 | | 1 : 5 | 3.88 | 108.8 | (0.246, 0.657) | 377 |
| Example 317 | 1-259 : 2-60 | 1 : 1 | 3.55 | 96.4 | (0.245, 0.634) | 318 |
| Example 318 | | 1 : 2 | 3.64 | 95.1 | (0.241, 0.627) | 332 |
| Example 319 | | 1 : 3 | 3.76 | 94.7 | (0.256, 0.654) | 340 |
| Example 320 | | 1 : 4 | 3.79 | 94.2 | (0.261, 0.653) | 342 |
| Example 321 | | 1 : 5 | 3.81 | 93.8 | (0.257, 0.661) | 344 |
| Example 322 | 1-272 : 2-51 | 1 : 1 | 3.62 | 98.4 | (0.263, 0.625) | 302 |
| Example 323 | | 1 : 2 | 3.74 | 97.2 | (0.263, 0.704) | 318 |
| Example 324 | | 1 : 3 | 3.88 | 96.1 | (0.273, 0.669) | 326 |
| Example 325 | | 1 : 4 | 3.91 | 93.2 | (0.262, 0.674) | 328 |
| Example 326 | | 1 : 5 | 3.94 | 92.6 | (0.259, 0.651) | 332 |
| Example 327 | 1-285 : 2-62 | 1 : 1 | 3.69 | 100.4 | (0.268, 0.625) | 329 |
| Example 328 | | 1 : 2 | 3.70 | 98.7 | (0.254, 0.678) | 341 |
| Example 329 | | 1 : 3 | 3.78 | 97.0 | (0.245, 0.646) | 356 |
| Example 330 | | 1 : 4 | 3.82 | 94.5 | (0.257, 0.674) | 352 |
| Example 331 | | 1 : 5 | 3.86 | 94.1 | (0.255, 0.675) | 350 |
| Example 332 | 1-311 : 2-62 | 1 : 1 | 3.77 | 105.4 | (0.676, 0.629) | 321 |
| Example 333 | | 1 : 2 | 3.84 | 103.2 | (0.660, 0.618) | 339 |
| Example 334 | | 1 : 3 | 3.89 | 99.4 | (0.559, 0.568) | 348 |
| Example 335 | | 1 : 4 | 3.90 | 98.3 | (0.257, 0.664) | 346 |
| Example 336 | | 1 : 5 | 3.95 | 97.6 | (0.249, 0.648) | 344 |
| Example 337 | 1-325 : 2-53 | 1 : 1 | 1.89 | 155.0 | (0.268, 0.654) | 587 |
| Example 338 | | 1 : 2 | 1.93 | 154.1 | (0.253, 0.675) | 593 |
| Example 339 | | 1 : 3 | 2.05 | 152.5 | (0.254, 0.685) | 600 |
| Example 340 | | 1 : 4 | 2.13 | 153.2 | (0.256, 0.663) | 603 |
| Example 341 | | 1 : 5 | 2.19 | 153.9 | (0.245, 0.646) | 606 |
| Example 342 | 1-327 : 2-51 | 1 : 1 | 1.79 | 150.4 | (0.248, 0.651) | 574 |
| Example 343 | | 1 : 2 | 1.83 | 149.3 | (0.243, 0.654) | 583 |
| Example 344 | | 1 : 3 | 1.98 | 146.8 | (0.277, 0.641) | 591 |
| Example 345 | | 1 : 4 | 2.03 | 145.3 | (0.265, 0.644) | 593 |
| Example 346 | | 1 : 5 | 2.12 | 144.1 | (0.243, 0.651) | 599 |
| Example 347 | 1-332 : 2-62 | 1 : 1 | 2.03 | 152.6 | (0.268, 0.635) | 532 |
| Example 348 | | 1 : 2 | 2.16 | 150.7 | (0.258, 0.667) | 544 |
| Example 349 | | 1 : 3 | 2.27 | 149.4 | (0.243, 0.645) | 559 |
| Example 350 | | 1 : 4 | 2.34 | 146.3 | (0.253, 0.648) | 563 |
| Example 351 | | 1 : 5 | 2.49 | 144.2 | (0.269, 0.623) | 569 |
| Example 352 | 1-334 : 2-53 | 1 : 1 | 1.98 | 152.6 | (0.249, 0.627) | 562 |
| Example 353 | | 1 : 2 | 2.07 | 150.3 | (0.256, 0.644) | 577 |
| Example 354 | | 1 : 3 | 2.16 | 149.4 | (0.261, 0.627) | 583 |
| Example 355 | | 1 : 4 | 2.23 | 143.2 | (0.256, 0.664) | 588 |
| Example 356 | | 1 : 5 | 2.34 | 140.6 | (0.247, 0.658) | 590 |
| Example 357 | 1-337 : 2-62 | 1 : 1 | 2.13 | 146.7 | (0.259, 0.714) | 489 |
| Example 358 | | 1 : 2 | 2.25 | 145.1 | (0.261, 0.649) | 492 |
| Example 359 | | 1 : 3 | 2.36 | 143.6 | (0.267, 0.635) | 507 |
| Example 360 | | 1 : 4 | 2.41 | 142.7 | (0.272, 0.641) | 513 |
| Example 361 | | 1 : 5 | 2.44 | 140.3 | (0.268, 0.643) | 522 |
| Example 362 | 1-339 : 2-68 | 1 : 1 | 2.03 | 149.5 | (0.265, 0.623) | 513 |
| Example 363 | | 1 : 2 | 2.23 | 147.3 | (0.264, 0.655) | 528 |
| Example 364 | | 1 : 3 | 2.39 | 146.2 | (0.243, 0.643) | 534 |
| Example 365 | | 1 : 4 | 2.41 | 145.3 | (0.265, 0.675) | 539 |
| Example 366 | | 1 : 5 | 2.44 | 144.0 | (0.259, 0.657) | 542 |
| Example 367 | 1-341 : 2-53 | 1 : 1 | 2.32 | 152.6 | (0.241, 0.658) | 562 |
| Example 368 | | 1 : 2 | 2.49 | 151.2 | (0.253, 0.625) | 571 |
| Example 369 | | 1 : 3 | 2.55 | 149.5 | (0.258, 0.651) | 588 |
| Example 370 | | 1 : 4 | 2.57 | 148.3 | (0.268, 0.655) | 591 |
| Example 371 | | 1 : 5 | 2.59 | 147.7 | (0.253, 0.645) | 594 |
| Example 372 | 1-351 : 2-51 | 1 : 1 | 2.18 | 152.6 | (0.256, 0.674) | 567 |
| Example 373 | | 1 : 2 | 2.26 | 150.0 | (0.258, 0.663) | 579 |
| Example 374 | | 1 : 3 | 2.39 | 148.4 | (0.275, 0.671) | 593 |
| Example 375 | | 1 : 4 | 2.41 | 147.6 | (0.257, 0.658) | 595 |
| Example 376 | | 1 : 5 | 2.44 | 146.6 | (0.243, 0.651) | 598 |
| Example 377 | 1-351 : 2-75 | 1 : 1 | 2.32 | 146.2 | (0.264, 0.675) | 531 |
| Example 378 | | 1 : 2 | 2.41 | 144.6 | (0.257, 0.665) | 548 |
| Example 379 | | 1 : 3 | 2.44 | 142.7 | (0.256, 0.653) | 563 |

When comparing the results in Table 16 with the results in Table 15, it can be confirmed that when the heterocyclic compound of Chemical Formula 1 and the compound of Chemical Formula 2 are simultaneously used as the light emitting layer host, the service life is improved about 3-fold, and the driving voltage and the light emitting efficiency are improved about 40% and about 50%, respectively.

In contrast, it can be seen that when a compound not included in the scope of the present invention is used in combination with the compound of Chemical Formula 2, the service life is similar to that when the heterocyclic compound of the present invention is used alone, and the performance deteriorates in terms of driving voltage and light emitting efficiency.

That is, it can be confirmed that when the heterocyclic compound of Chemical Formula 1 and the compound of Chemical Formula 2 of the present invention are simultaneously used as hosts of a light emitting layer, the driving voltage, light emitting efficiency and service life are remarkably excellent.

In particular, when comparing Examples in which the same heterocyclic compound of Chemical Formula 1 is used and the compounds of Chemical Formula 2 have the same structure but differ only in whether or not the compounds are substituted with deuterium, it can be confirmed that when the compound of Chemical Formula 2 substituted with deuterium is used, the light emitting device are all excellent in terms of driving voltage, light emitting efficiency, and service life.

This is because the case where even the compound of Chemical Formula 2 includes deuterium is even more effective in improving the performance of the organic light emitting device as the stability and thermal stability of the molecule are enhanced in the compound of Chemical Formula 2 along with the heterocyclic compound of Chemical Formula 1.

Further, in the case of a combined device of Chemical Formula 1 and Chemical Formula 2, the optimum driving, efficiency, and service life results are shown particularly when the combination ratio of a N-type host and a P-type host is 1:3. This is a result according to the tendency for driving and service life to increase and for efficiency to decrease as the proportion of the N-type host decreases. Particularly when the proportion of the N-type host is less than 23%, that is, from the ratio of 1:4, the driving and service life tend to be similar or gradually increase. In contrast, it could be confirmed through experiments that efficiency tends to sharply decrease. As a result, particularly when a device is made by combining the compounds of the present invention, good results may be obtained in terms of driving, efficiency, and service life when the proportion of the N-type host is 30% or more. Through this, it could be confirmed that Chemical Formula 1 plays an important role in increasing the efficiency of the device.

### <Experimental Example 3>- HOMO/LUMO/SOMO

In the case of the material of Compound 1-1 of the present invention, as can be confirmed in Table 17, when the electron cloud distribution in the HOMO and LUMO states is observed, LUMO is distributed in the triazine moiety and HOMO is distributed in the heterocyclic moiety. In the organic compound molecule, holes move through the HOMO and electrons move through the LUMO. A moiety substituted with deuterium has a higher packing density than a moiety substituted with hydrogen. Therefore, in the moiety substituted with deuterium, the distance between molecules becomes closer, so that holes or electrons move faster, and in the present invention, hole characters were enhanced by substituting the HOMO moiety that transmits holes with deuterium.

It can be confirmed that when an OLED device is made using Compound 1-1 with enhanced role of the hole character of the molecule and a new dopant with enhanced efficiency characteristics, a recombination zone (RZ) is located in the center of the light emitting layer compared to Comparative Example 3-17, and thus the efficiency and the service life are improved.

In addition, when triazines responsible for LUMO are substituted with deuterium as in Comparative Example 3-21, electrons move even faster, so that the recombination zone (RZ) is further biased toward the HTL side compared to Comparative Example 3-17 in which triazines are not substituted with deuterium.

Accordingly, a result that both efficiency and service life were reduced occurred. When the entire compound is substituted with deuterium as in Comparative Example 3-1, both electrons and holes become faster, and thus, the RZ is not changed, showing a level of efficiency similar to that of Comparative Example 3-17. In other words, it can be confirmed that when the heterocyclic compound of Chemical Formula 1 of the present invention is used as a host of a light emitting layer, the light emitting efficiency and service life are remarkably excellent.

For reference, the radical cation singly occupied molecular orbital (SOMO) distribution of Compound 1-1 can be confirmed in the following Table 17. When the SOMO moiety is substituted with deuterium, the number of neutrons in the atom nucleus increases compared to when the SOMO moiety is substituted with hydrogen, so that it is possible to improve the stability of radical cation that may occur when a device is driven. Therefore, when Chemical Formula 1 of the present invention is used as the host of the light emitting layer, the stability of the molecule is excellent during the driving of the device.

**[Table 17]**

| HOMO Electronic Distribution | LUMO Electronic Distribution | SOMO Electronic Distribution |
|---|---|---|
| 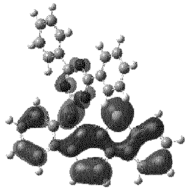 | 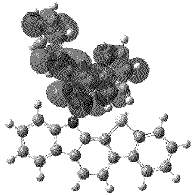 | 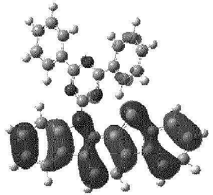 |

### <Experimental Example 4>- Confirmation of recombination zone

A recombination zone (RZ) confirmation experiment was conducted by the same organic light emitting device manufacturing method as in Experimental Example 1. The difference from Experimental Example 1 is the doping position of a green phosphorescent in the light emitting layer.

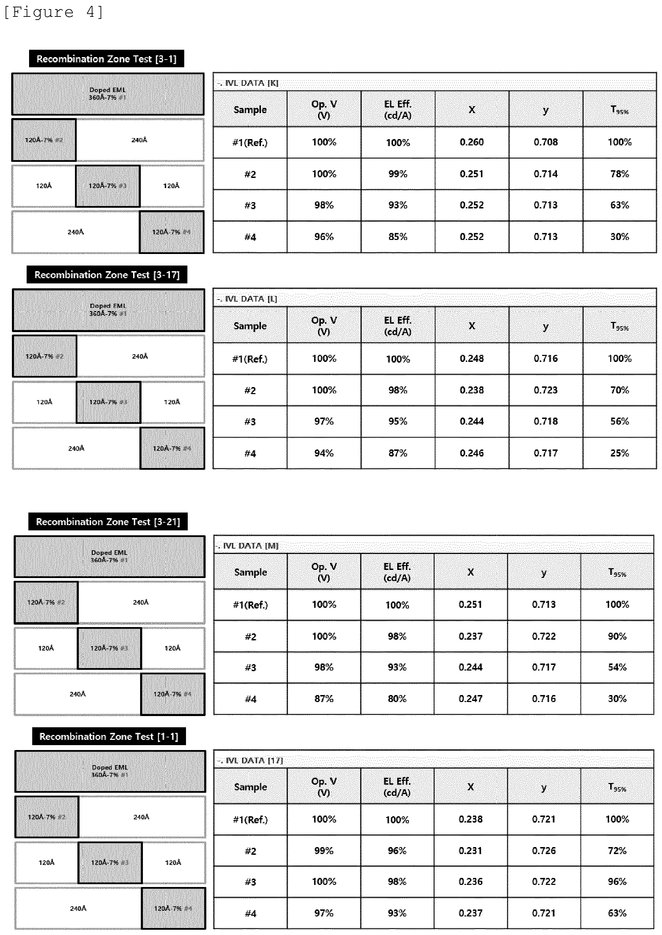

In #1 of FIG. 4, the entire light emitting layer was doped with a green phosphorescent dopant, and the doped light emitting layer was used as a comparative group. In the case of #2, only 120 Å of a colored portion (located close to a hole transport layer) was doped, and only a host was deposited onto the remaining 240 Å. In the case of #3, only 120 Å of a colored portion (located at the center of a light emitting layer) in the lower drawing was doped, and only a host was deposited onto the remaining uncolored portion. In the case of #4, only 120 Å of a colored portion (located close to a hole blocking layer) in the lower drawing was doped, and only a host was deposited onto the remaining uncolored portion.

In the case of Comparative Example Compounds 3-1 and 3-17, the RZ was located close to a hole transport layer (HTL), so that #2 had the best efficiency and service life. This shows that the electron transfer rate of the compound is faster than the hole transfer rate. It can be confirmed that Comparative Example Compound 3-21 further increases the electron transfer rate by substituting a triazine moiety responsible for LUMO with deuterium, and thus increases the service life of #2 and further decreases the service lives of #3 and #4 compared to Compounds 3-1 and 3-17.

In contrast, Compound 1-1 exhibits the highest efficiency and service life at #3 (located at the center of a light emitting layer). This is because the hole transport character is stronger, the hole transfer rate is relatively further faster, and the transfer of holes and electrons is well-balanced as described above, confirming that the RZ is located close to the center of the EML layer.

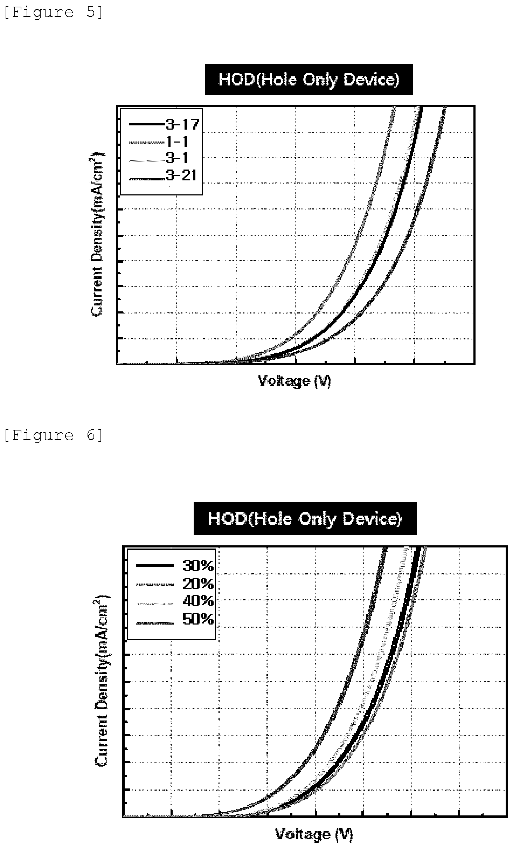

The above contents can be confirmed through an experiment of confirming the current density over voltage by manufacturing a hole only device (HOD), and the results are shown in FIG. 5.

Additionally, the following FIG. 6 shows the measurement of current density over voltage by manufacturing a hole only device (HOD) using compounds with different deuterium substitution rates for Compound 1-1. Through FIG. 6, it can be confirmed that the larger the deuterium substitution rate of the compound of the present invention is, the faster the hole transfer rate is. Through experiments, it could be confirmed through experiments that the compound of the present invention exhibits characteristics of much more balanced charge and hole transport in the device when the deuterium substitution rate is 50% or more, and thus achieves maximum efficiency and service life.

### [Explanation of Reference Numerals and Symbols]

100: Substrate
200: Positive electrode
300: Organic material layer
301: Hole injection layer
302: Hole transport layer
303: Light emitting layer
304: Hole blocking layer
305: Electron transport layer
306: Electron injection layer
400: Negative electrode

## Claims

1. A heterocyclic compound of the following Chemical Formula 1: 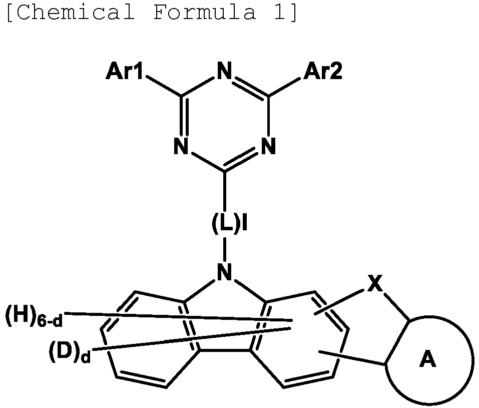 in Chemical Formula 1,
X is O; S; or CRR',
L is a direct bond; or a substituted or unsubstituted arylene group having 6 to 60 carbon atoms,
l is an integer from 1 to 3, and when l is 2 or higher, L's are the same as or different from each other,
A is an aryl ring having 6 to 60 carbon atoms, which is unsubstituted or substituted with deuterium; or a hetero ring having 2 to 60 carbon atoms, which is unsubstituted or substituted with deuterium,
R, R', Ar1 and Ar2 are each independently hydrogen; deuterium; a halogen group; a cyano group; a substituted or unsubstituted alkyl group having 1 to 60 carbon atoms; a substituted or unsubstituted cycloalkyl group having 3 to 60 carbon atoms; a substituted or unsubstituted heterocycloalkyl group having 2 to 60 carbon atoms; a substituted or unsubstituted aryl group having 6 to 60 carbon atoms; or a substituted or unsubstituted heteroaryl group having 2 to 60 carbon atoms,
H is hydrogen and D is deuterium,
d is an integer from 0 to 6, and
a deuterium content of 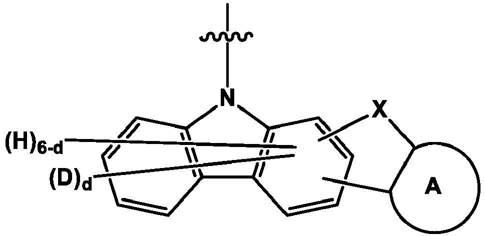 of Chemical Formula 1 is more than 0%.

2. The heterocyclic compound of claim 1, wherein Chemical Formula 1 is represented by the following Chemical Formula 1-1: 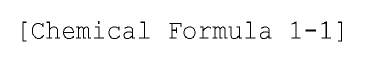 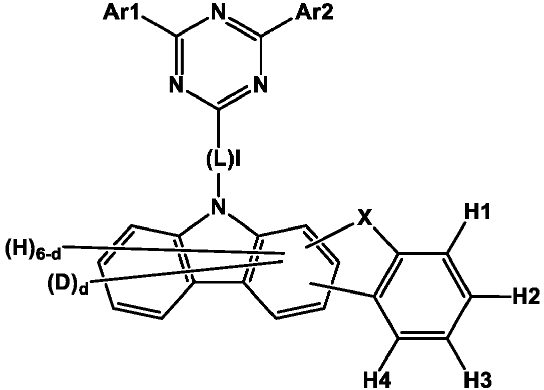 in Chemical Formula 1-1,
H1 to H4 are each independently hydrogen; or deuterium, and
the definitions of the other substituents are the same as the definitions in Chemical Formula 1.

3. The heterocyclic compound of claim 1, wherein Chemical Formula 1 is represented by any one of the following Chemical Formulae 1-1-1 to 1-1-6: 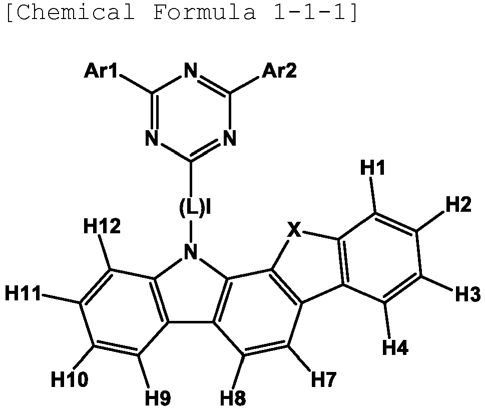 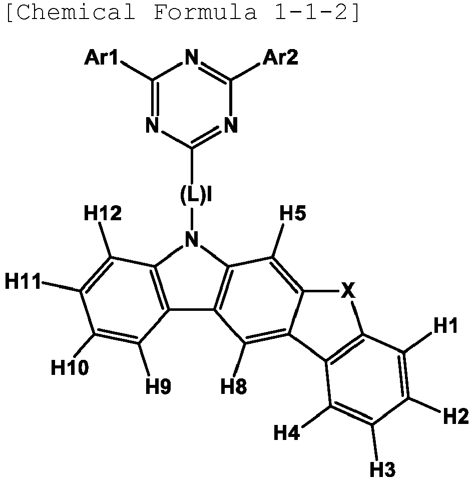 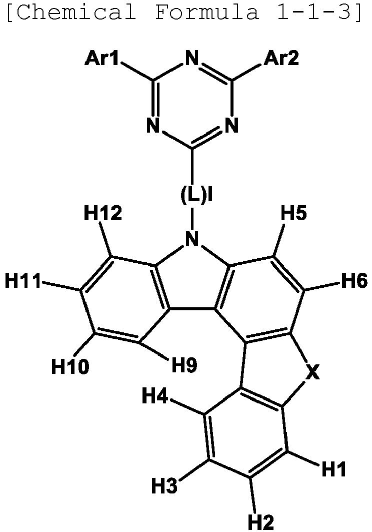 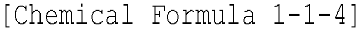 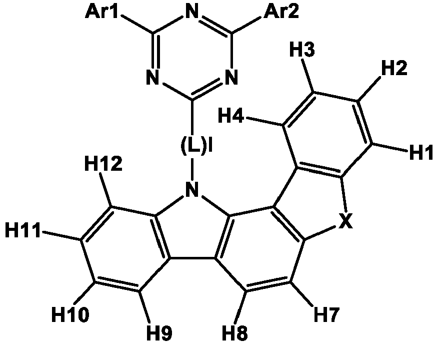 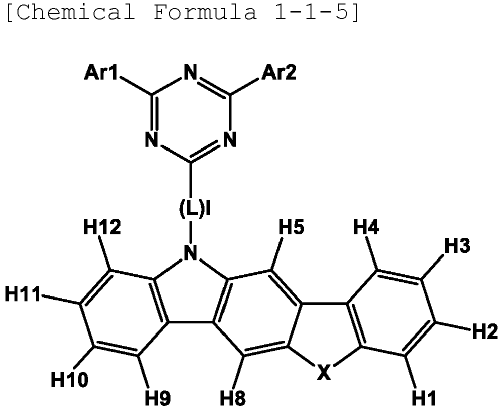 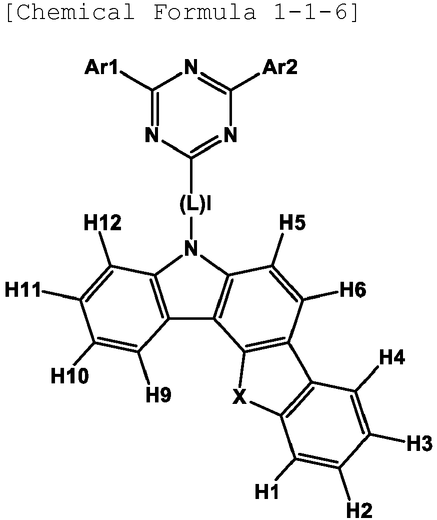 in Chemical Formulae 1-1-1 to 1-1-6,
H1 to H12 are each independently hydrogen; or deuterium, at least one of H1 to H12 is deuterium, and the definitions of the other substituents are the same as the definitions in Chemical Formula 1.

4. The heterocyclic compound of claim 1, wherein Ar1 and Ar2 are each independently a substituted or unsubstituted aryl group having 6 to 30 carbon atoms; or a heteroaryl group having 2 to 30 carbon atoms, which is substituted or unsubstituted and comprises O or S.

5. The heterocyclic compound of claim 1, wherein Chemical Formula 1 comprises the structures of the following Chemical Formulae A and B and selectively comprises the structure of the following Chemical Formula C, and a deuterium content of the following Chemical Formula B is 50% or more and 100% or less: 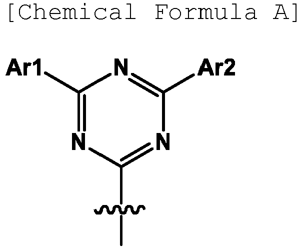 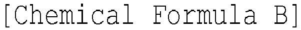 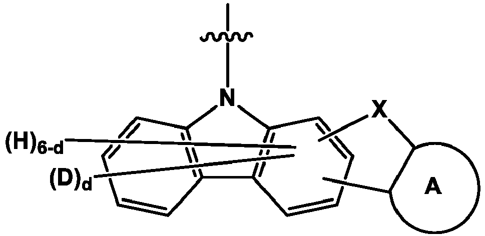 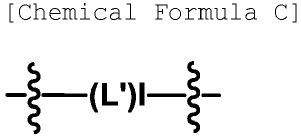 in Chemical Formulae A to C, 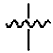 of each structure means a site that is bonded to another structure, and
L' is a substituted or unsubstituted arylene group having 6 to 60 carbon atoms, and the definitions of the other substituents are the same as the definitions in Chemical Formula 1.

6. The heterocyclic compound of claim 1, wherein Chemical Formula 1 is represented by any one of the following compounds:

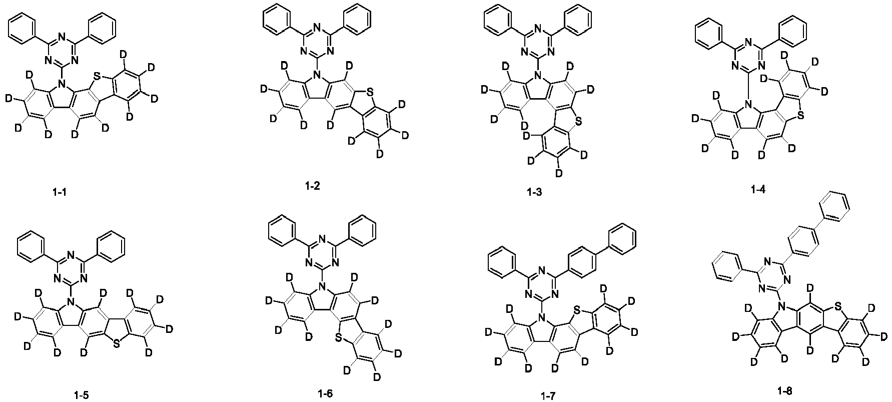

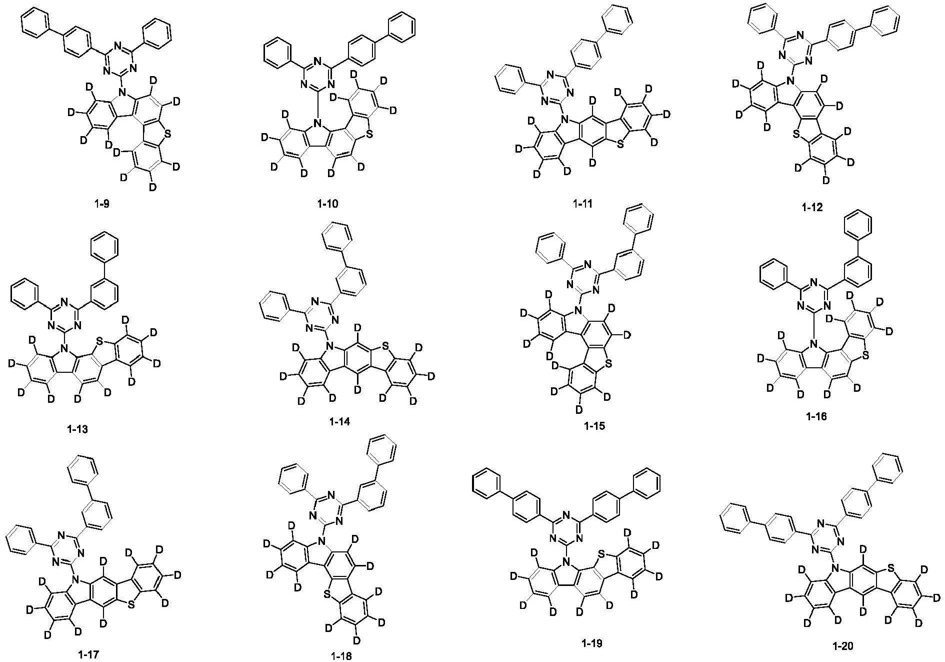

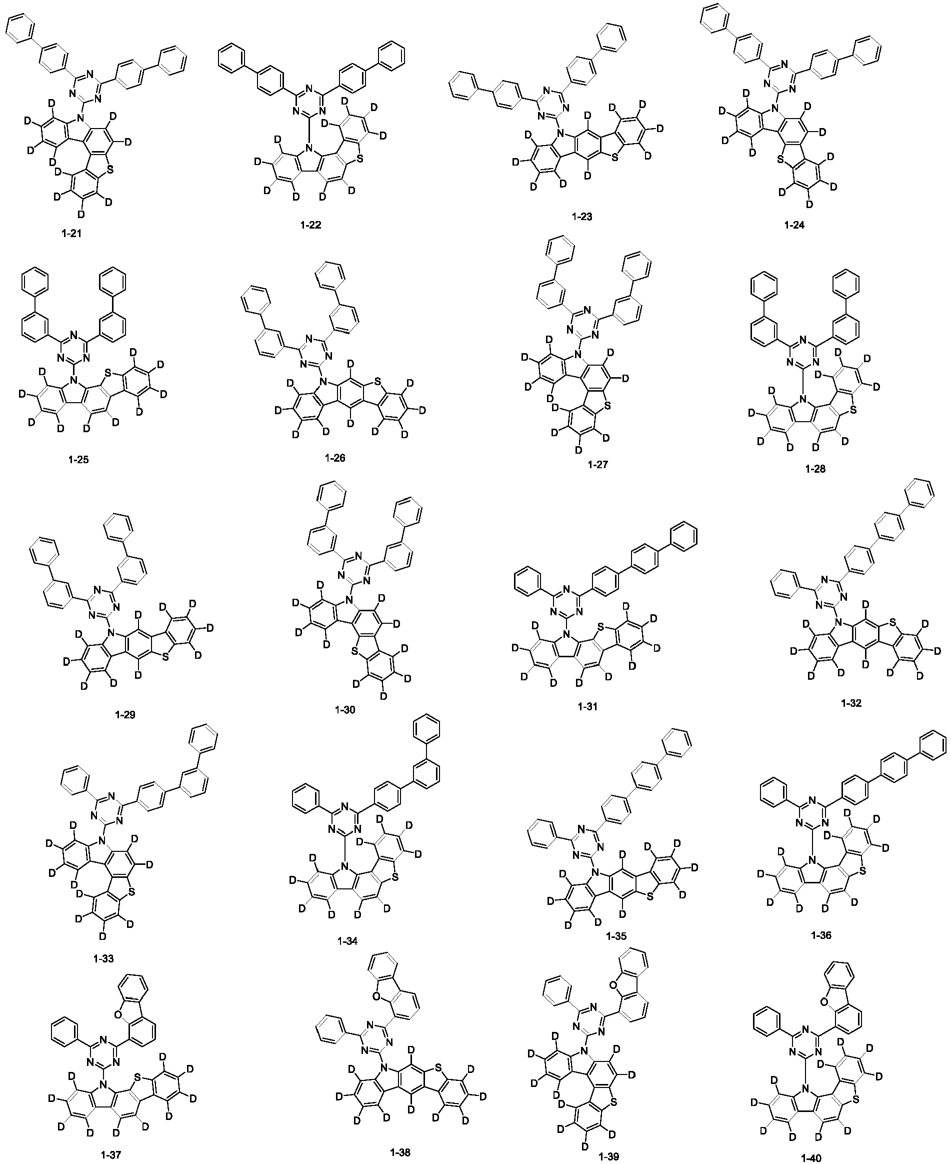

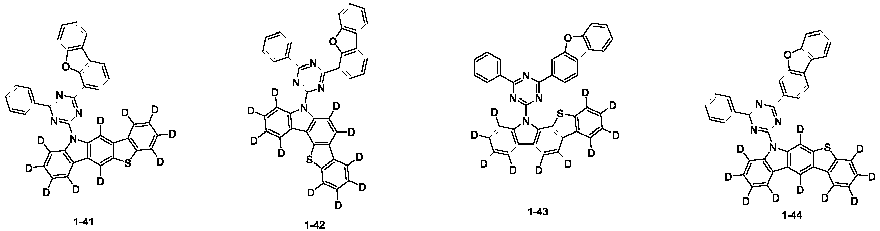

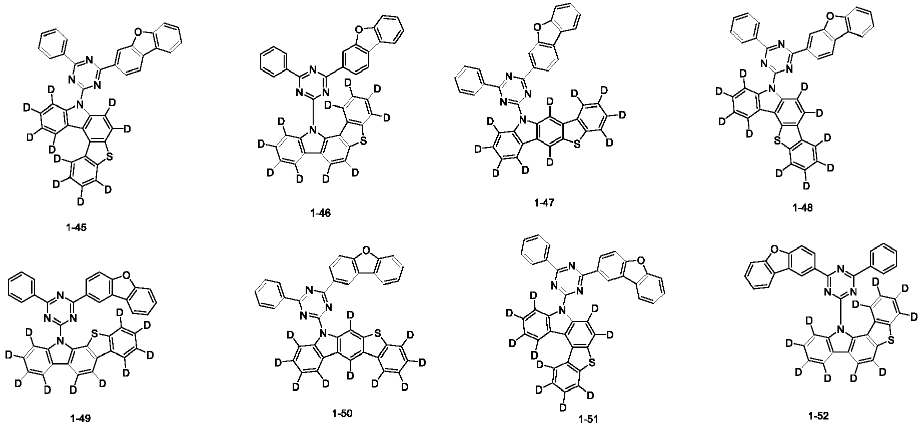

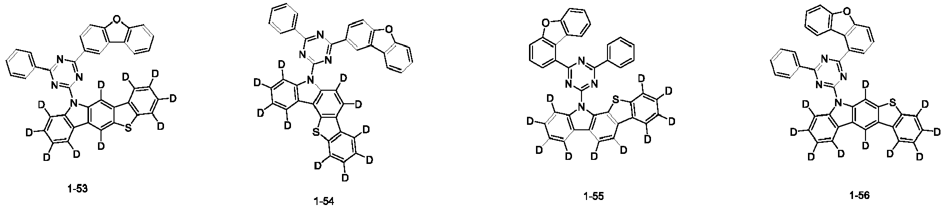

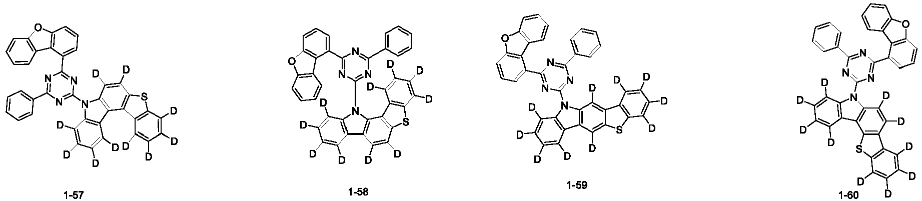

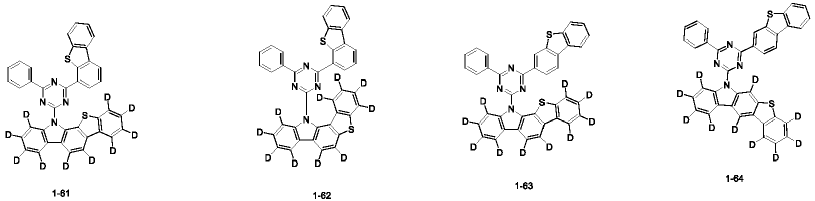

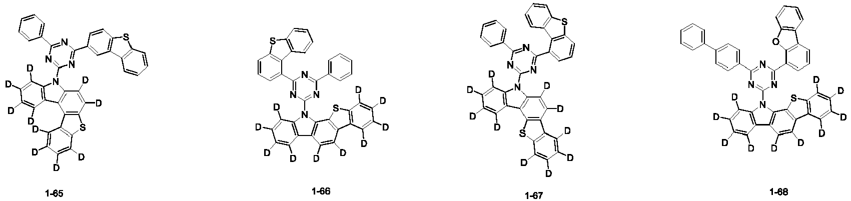

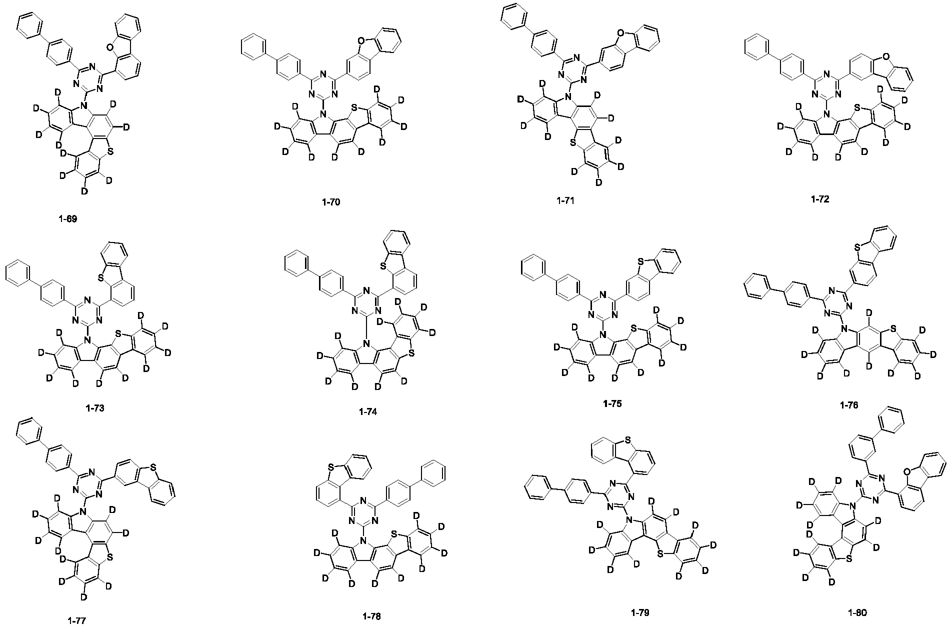

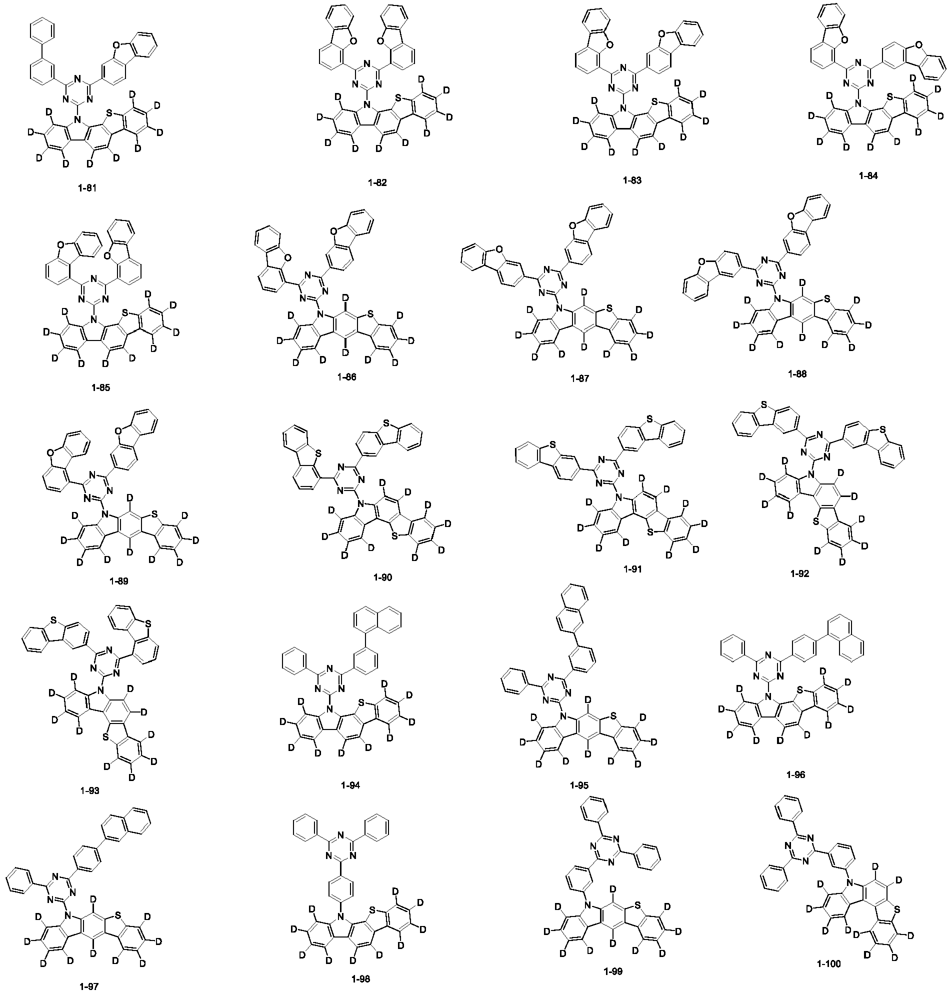

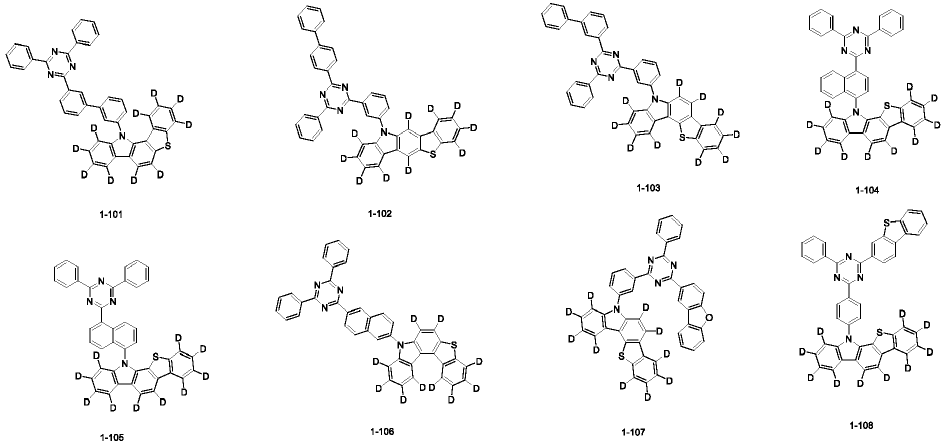

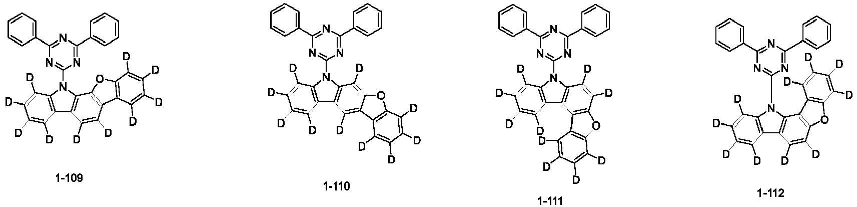

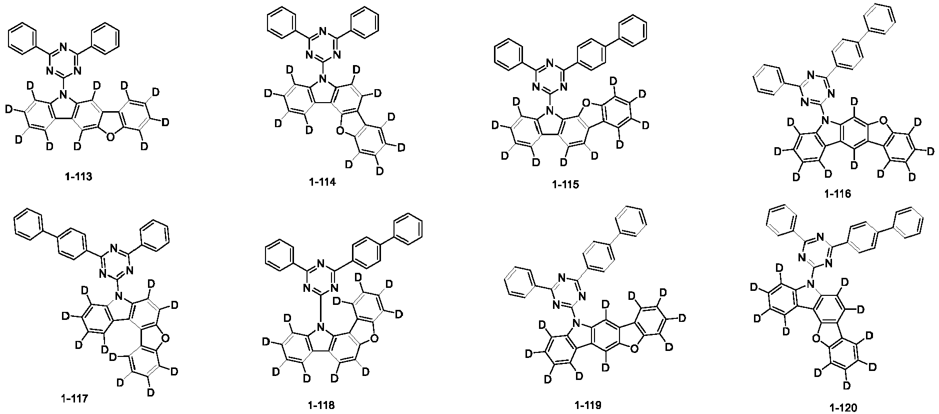

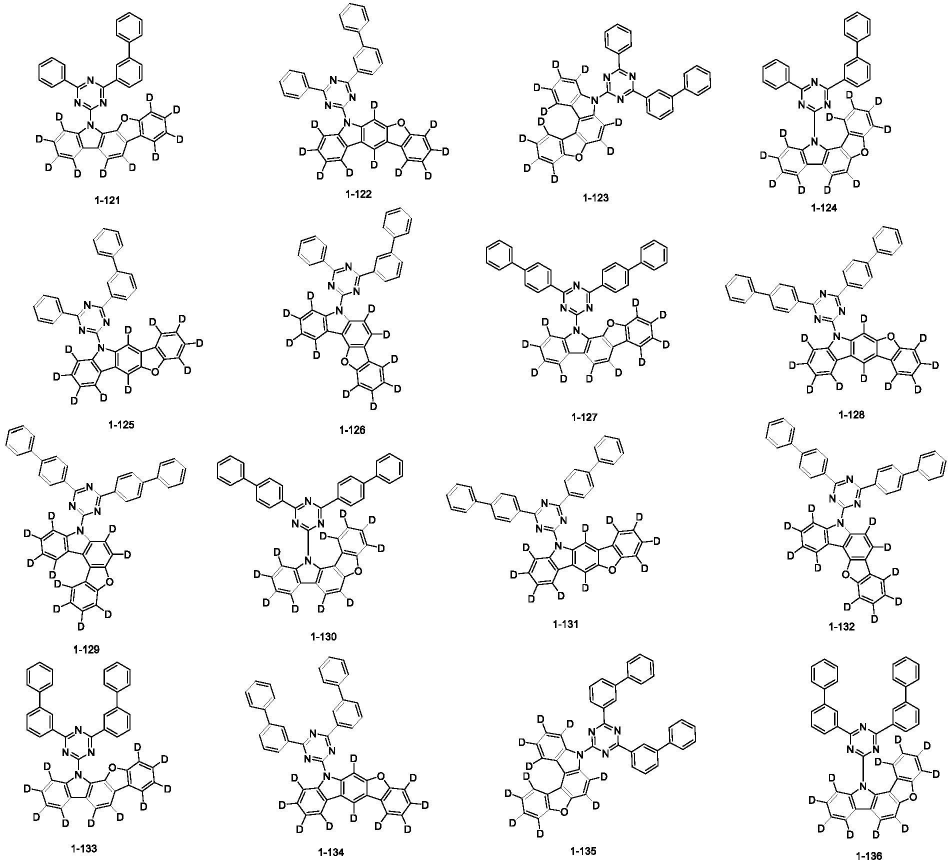

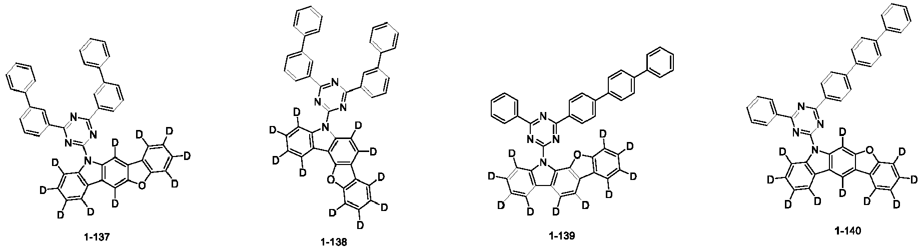

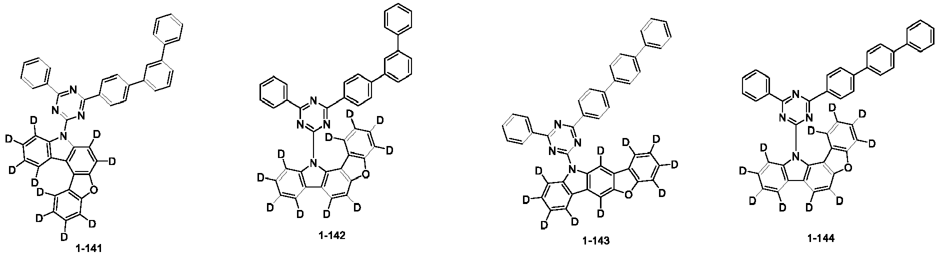

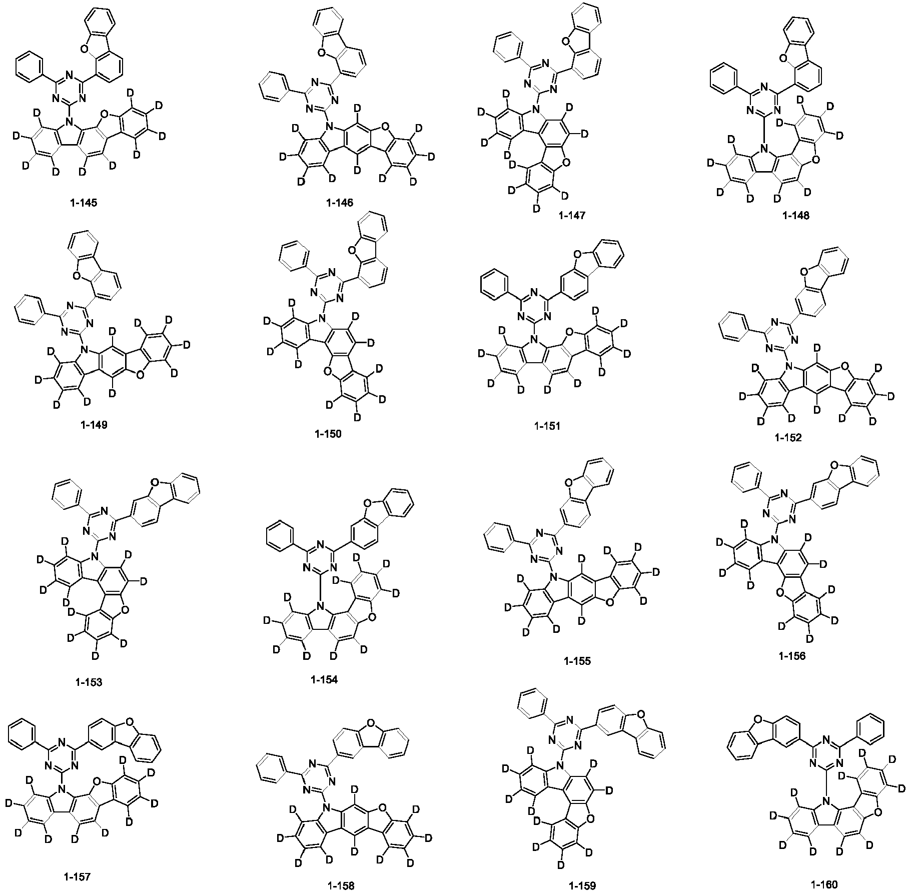

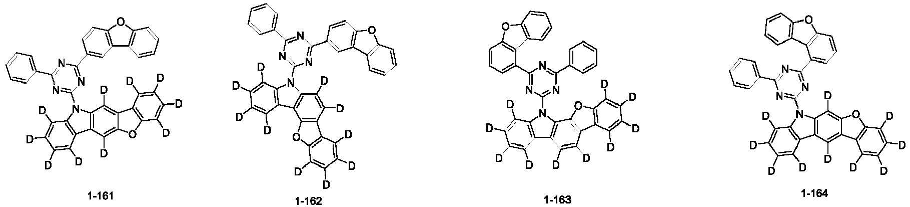

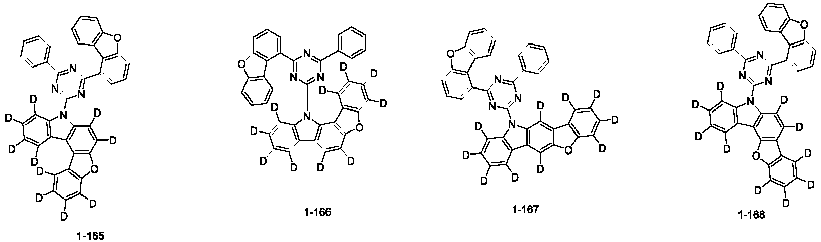

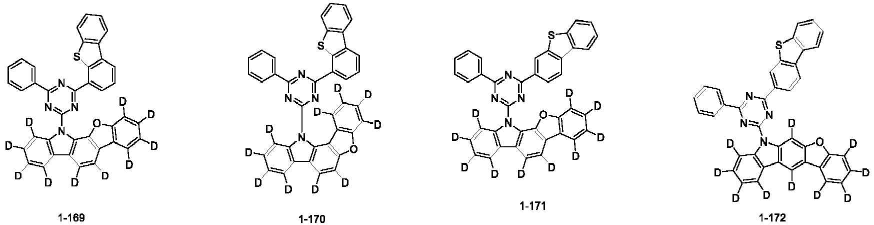

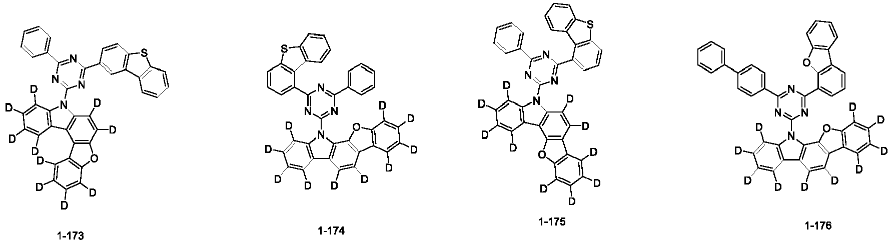

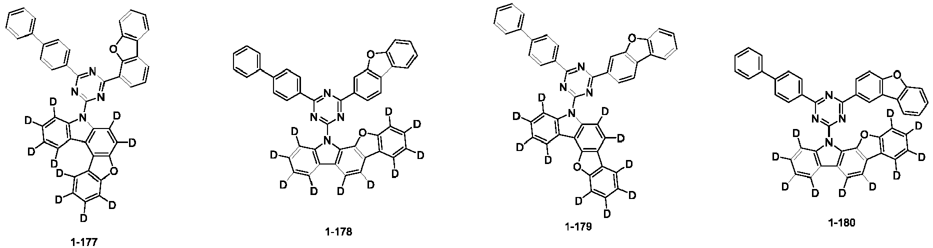

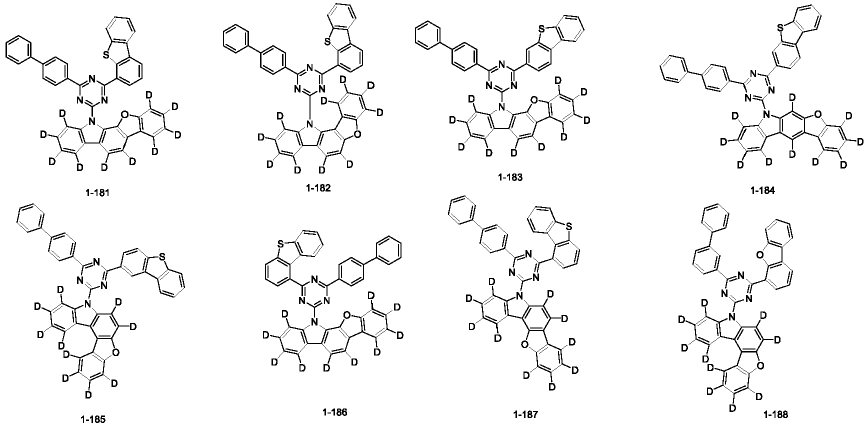

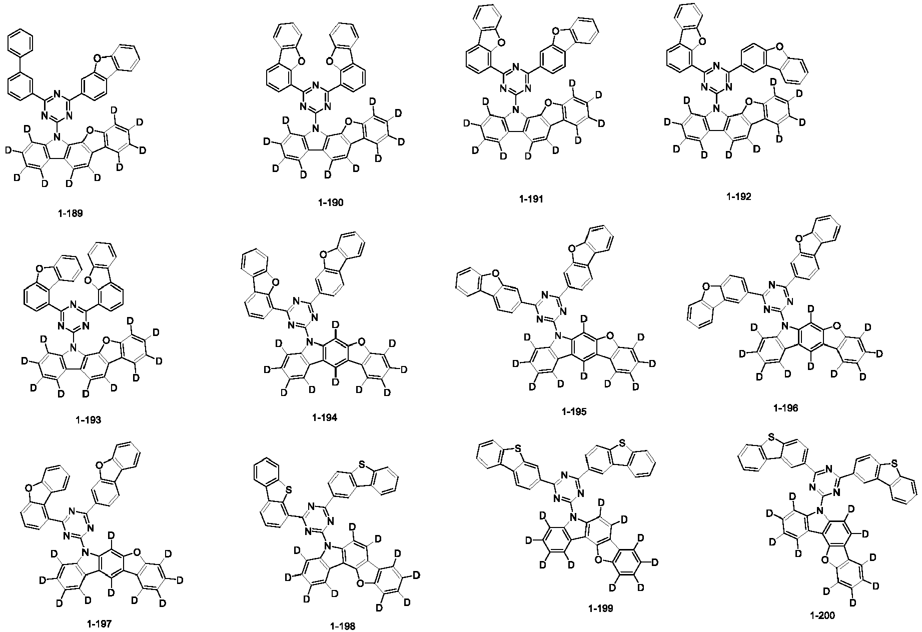

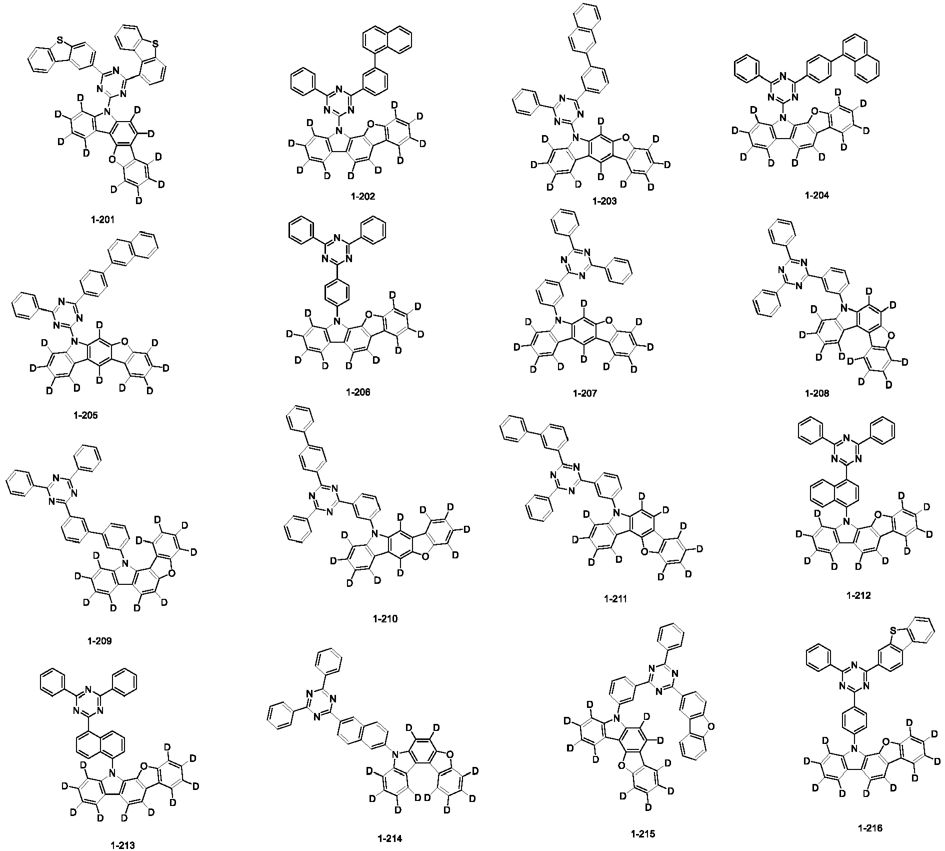

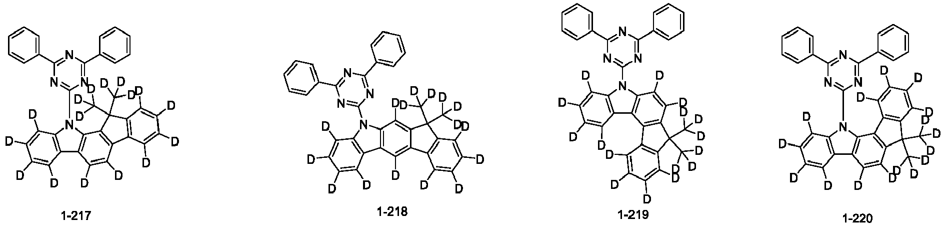

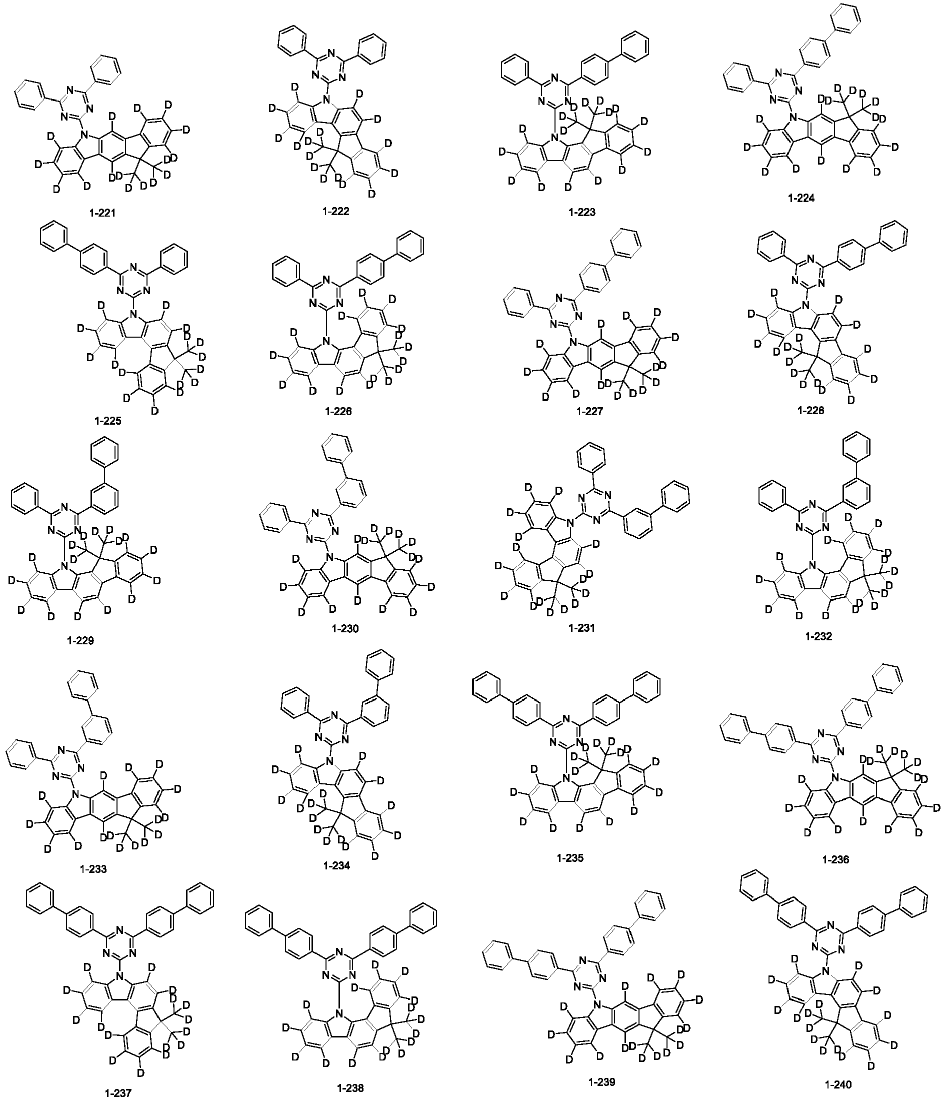

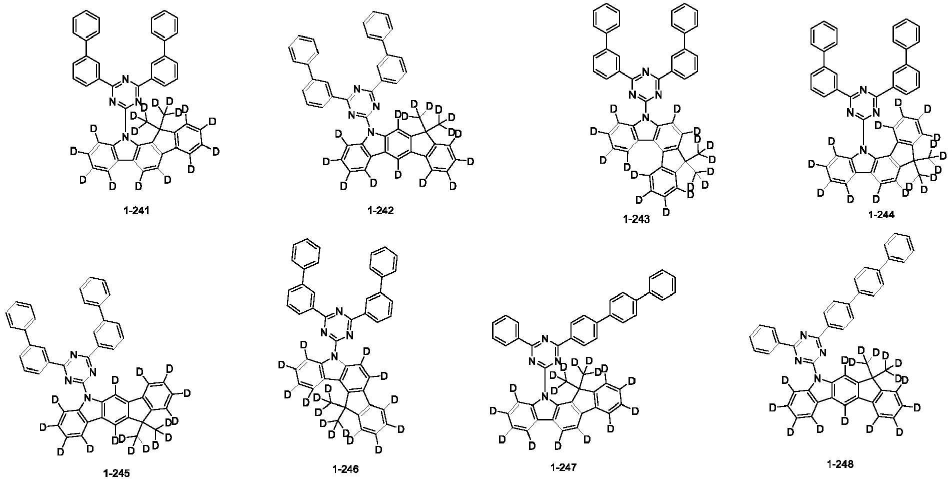

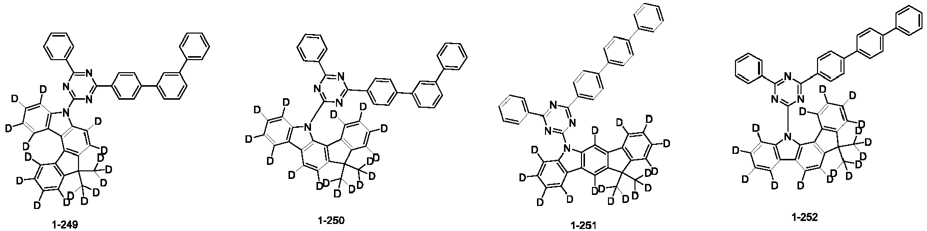

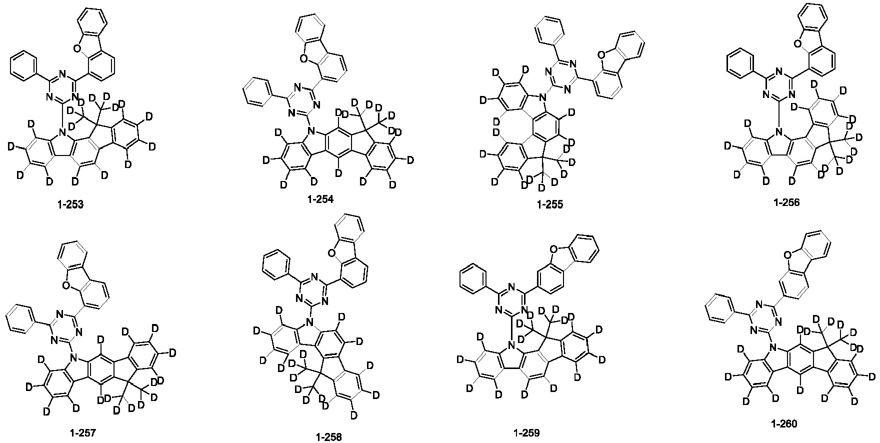

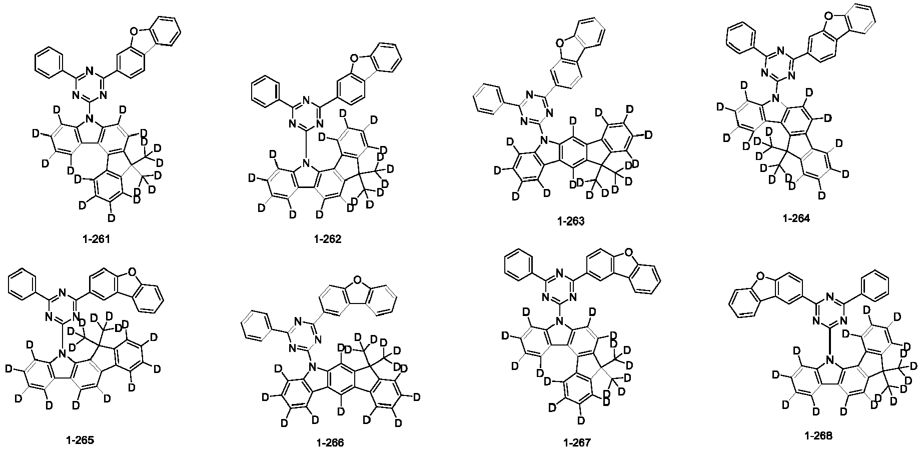

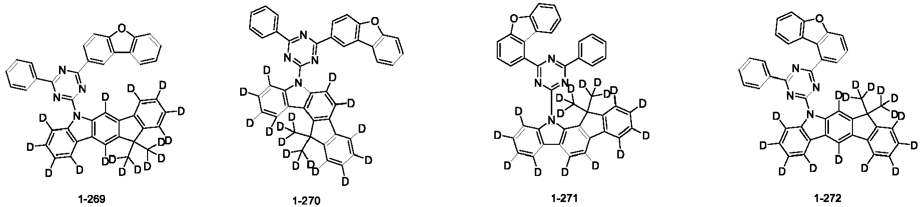

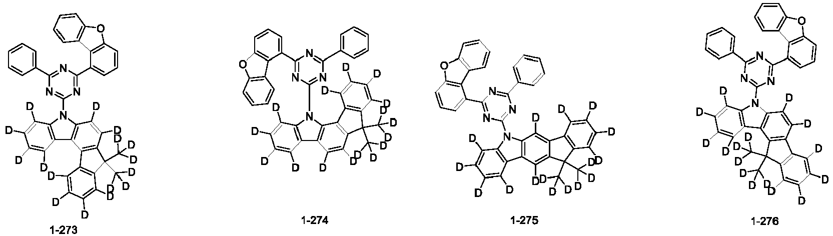

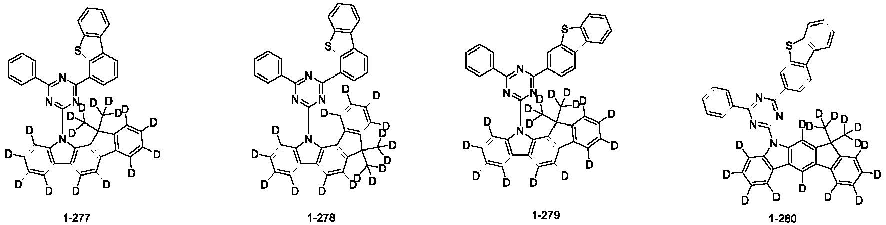

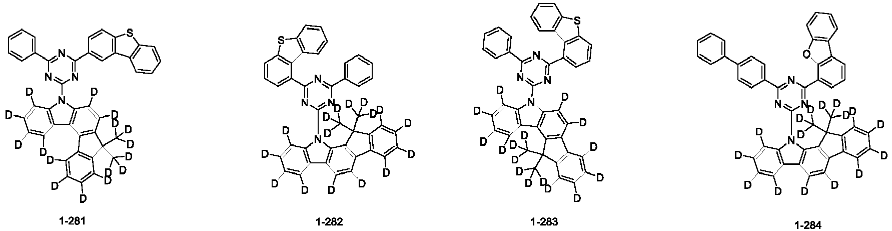

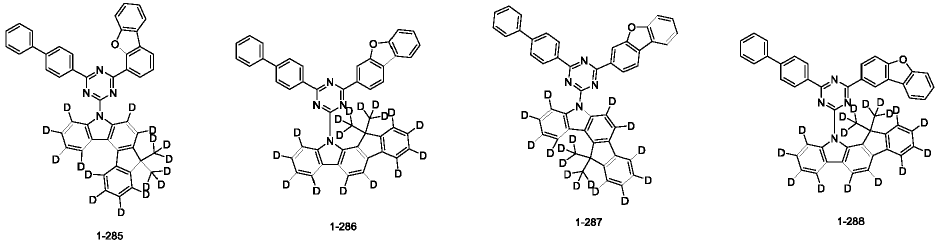

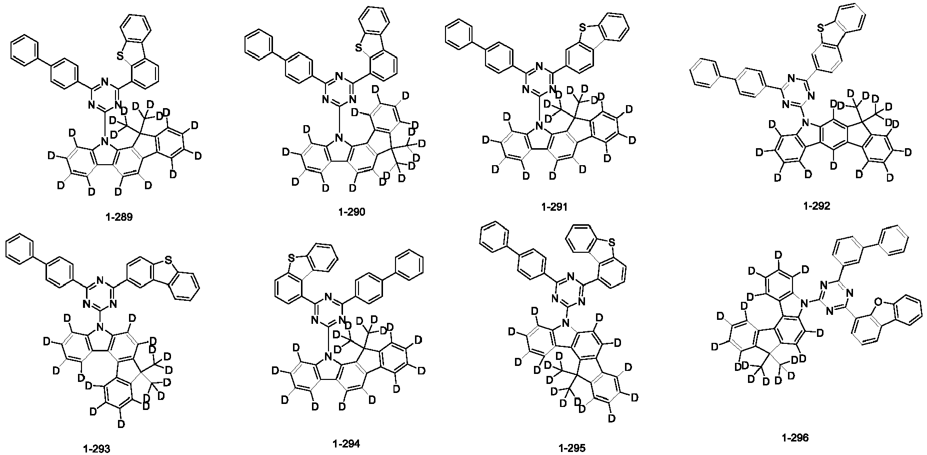

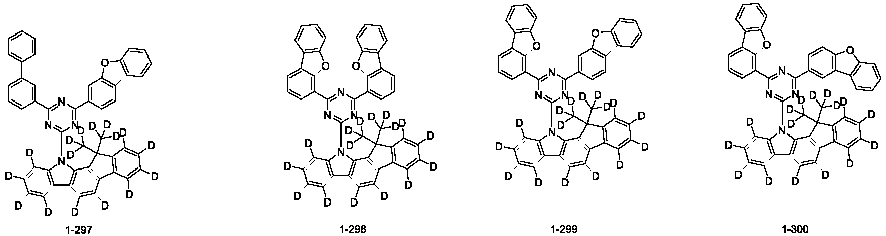

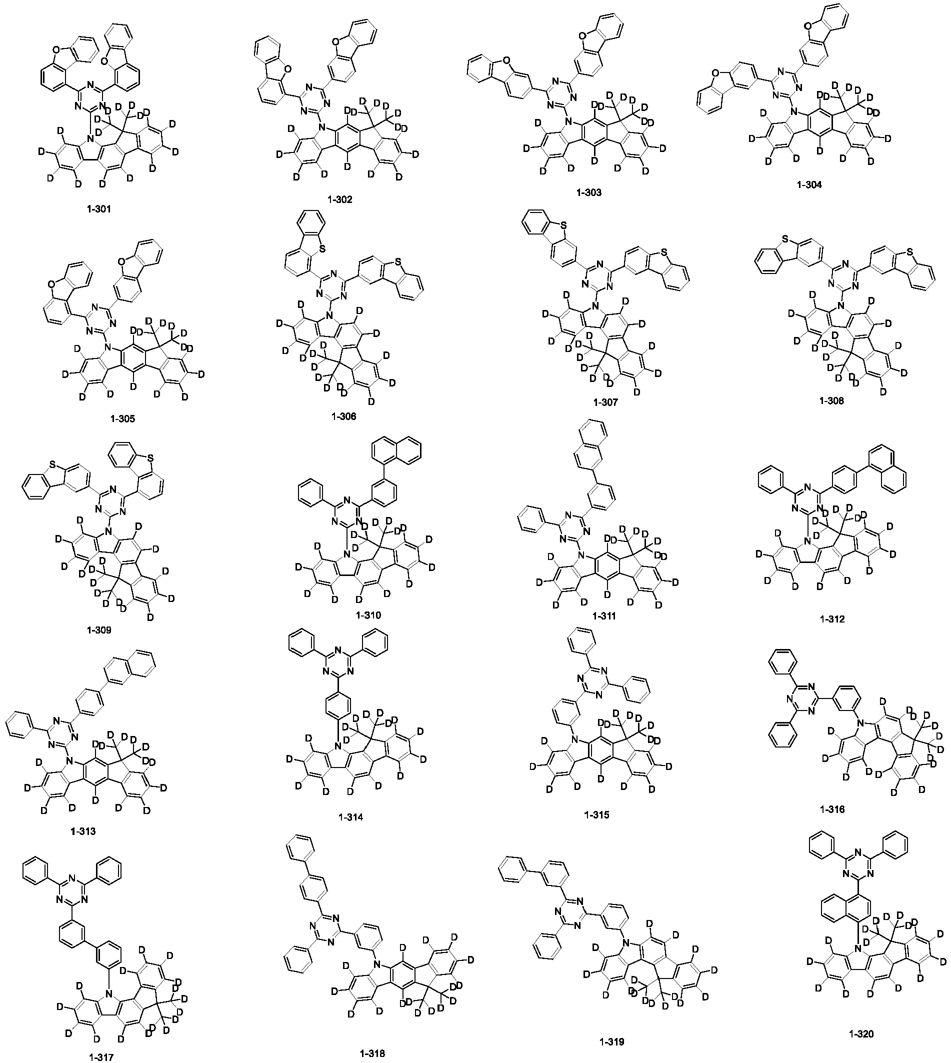

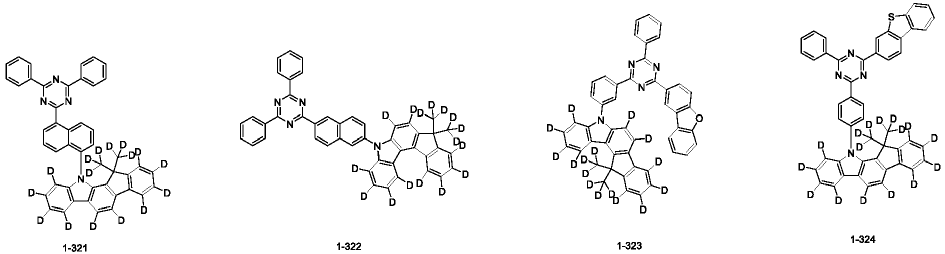

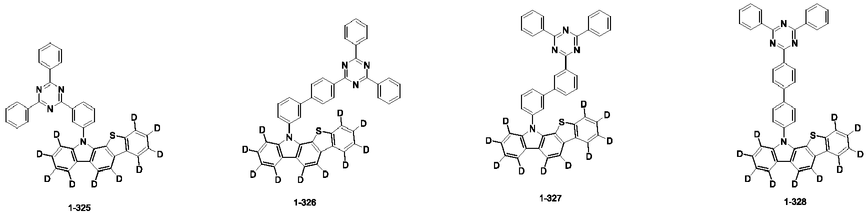

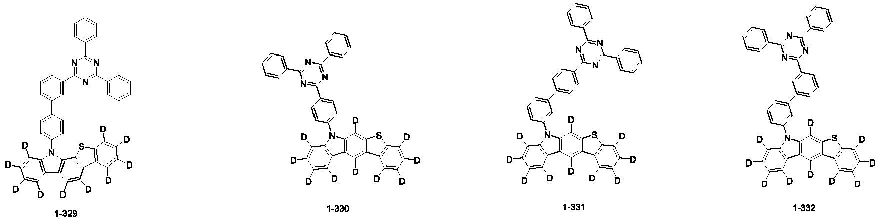

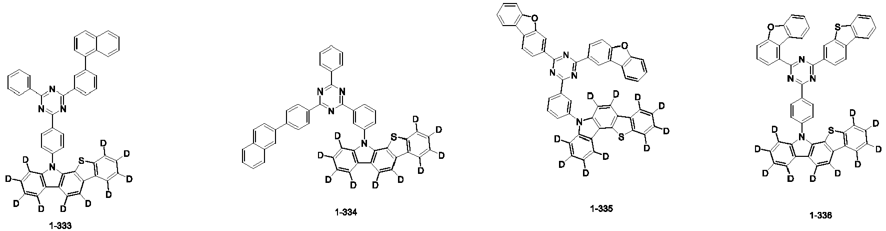

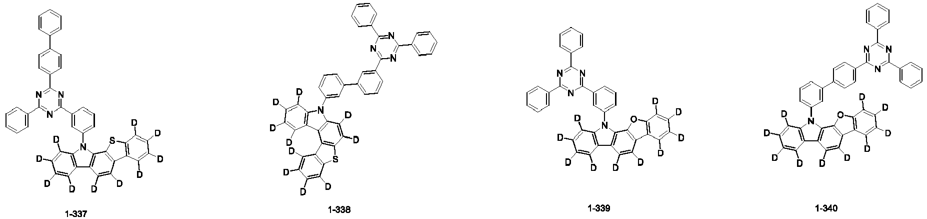

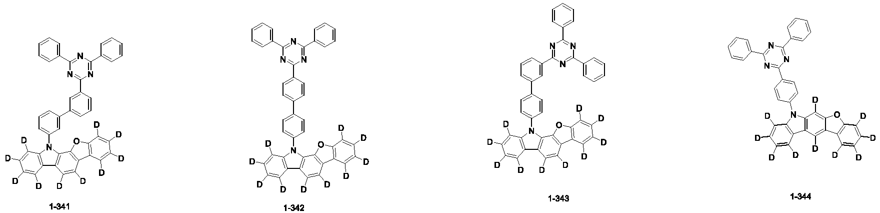

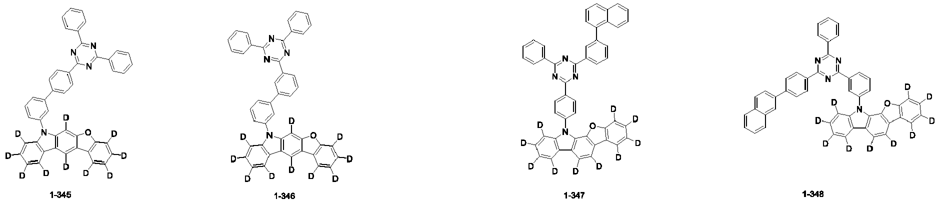

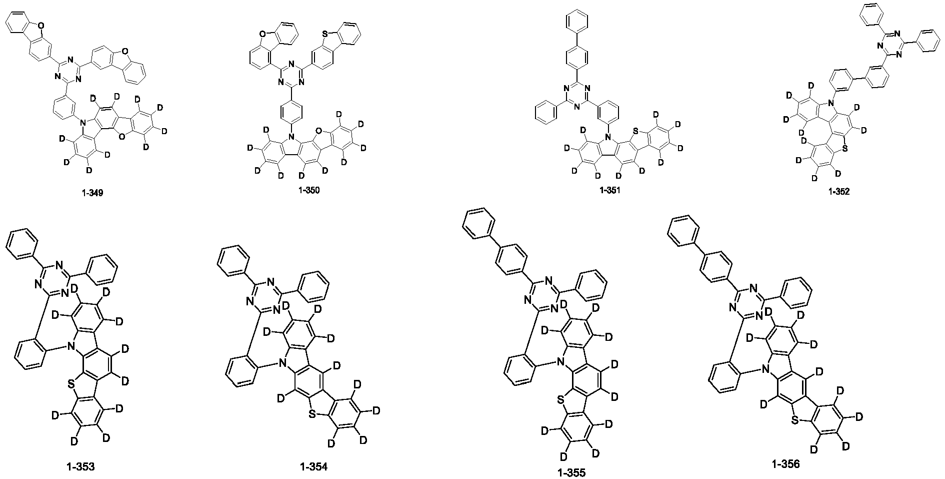

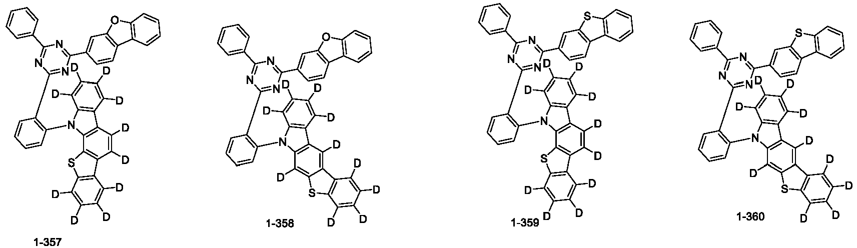

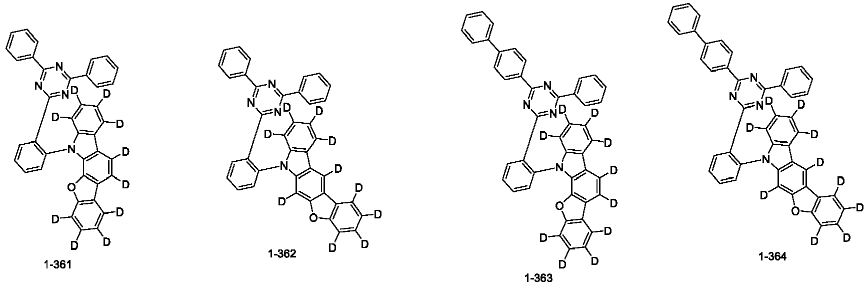

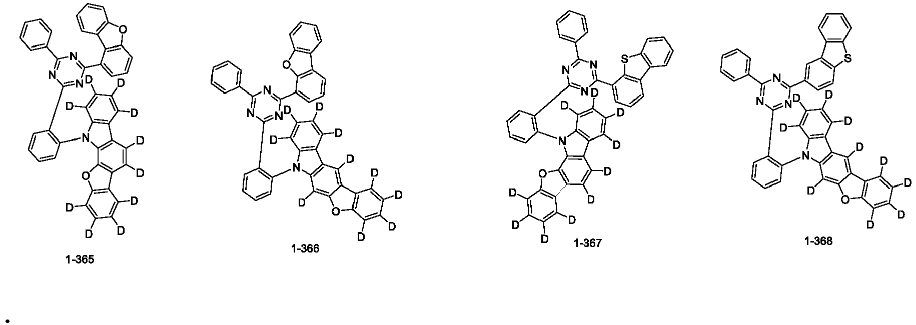

7. An organic light emitting device comprising: a first electrode; a second electrode; and an organic material layer having one or more layers provided between the first electrode and the second electrode,
wherein one or more layers of the organic material layer comprise the heterocyclic compound of any one of claims 1 to 6.

8. The organic light emitting device of claim 7, wherein the organic material layer comprising the heterocyclic compound further comprises a compound of the following Chemical Formula 2: 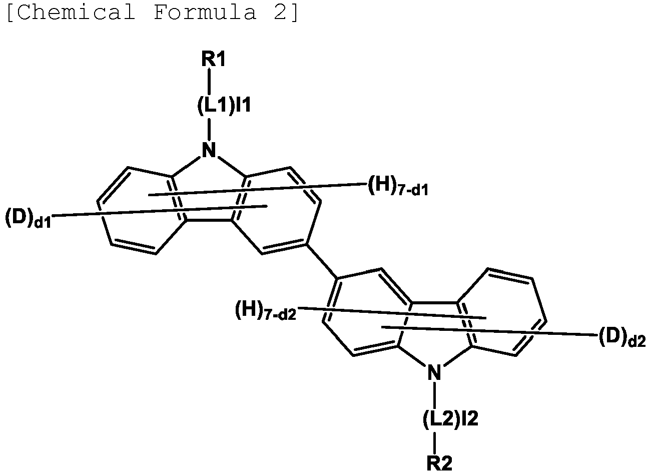 in Chemical Formula 2,
L1 and L2 are each independently a direct bond; or a substituted or unsubstituted arylene group having 6 to 60 carbon atoms,
l1 and l2 are each an integer from 1 to 3, and when l1 and l2 are each 2 or higher, substituents in the parenthesis are the same as or different from each other,
R1 and R2 are each independently hydrogen; deuterium; a halogen group; a cyano group; a substituted or unsubstituted alkyl group having 1 to 60 carbon atoms; a substituted or unsubstituted cycloalkyl group having 3 to 60 carbon atoms; a substituted or unsubstituted heterocycloalkyl group having 2 to 60 carbon atoms; a substituted or unsubstituted aryl group having 6 to 60 carbon atoms; or a substituted or unsubstituted heteroaryl group having 2 to 60 carbon atoms,
H is hydrogen and D is deuterium, and
d1 and d2 are each independently an integer from 0 to 7.

9. The organic light emitting device of claim 8, wherein the deuterium content of Chemical Formula 2 is 0% or 10% to 100%.

10. The organic light emitting device of claim 8, wherein Chemical Formula 2 is represented by any one of the following compounds:

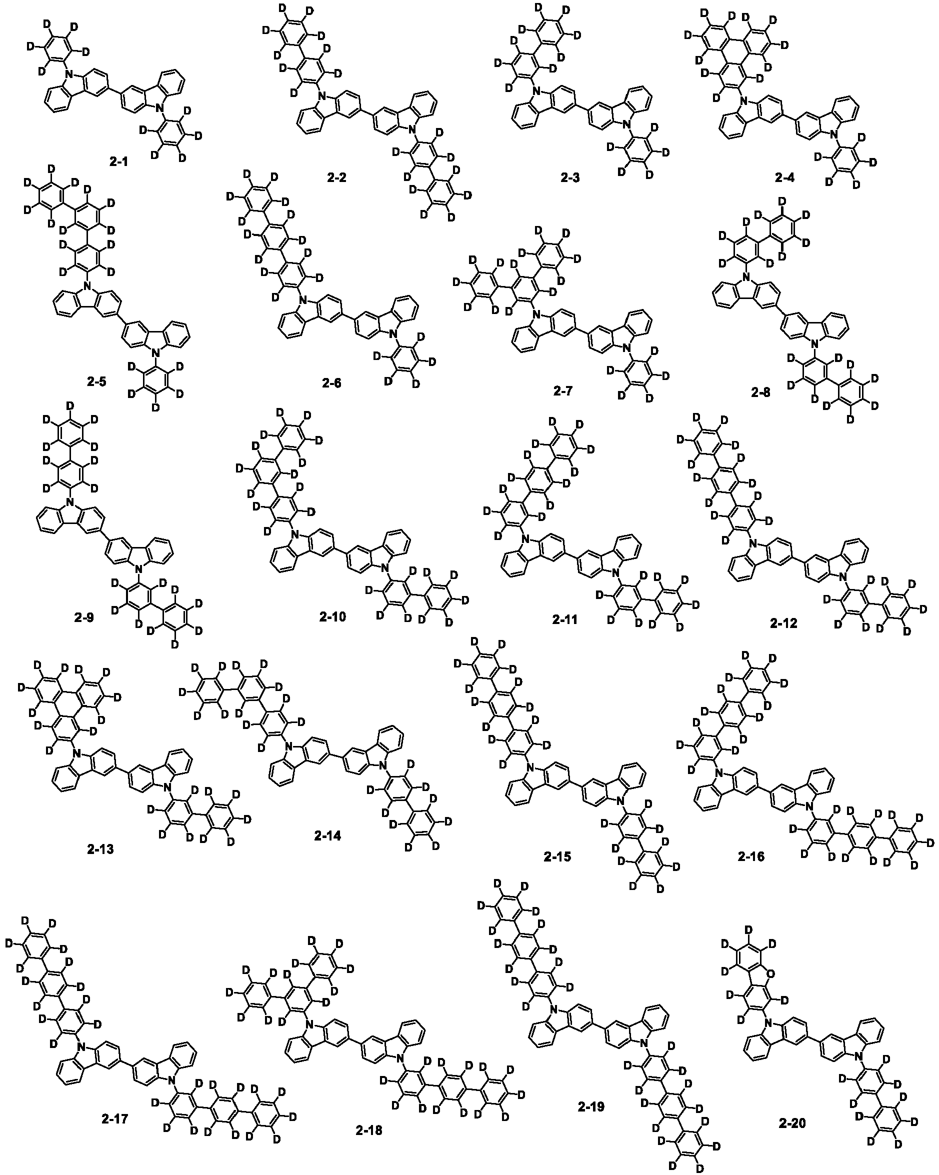

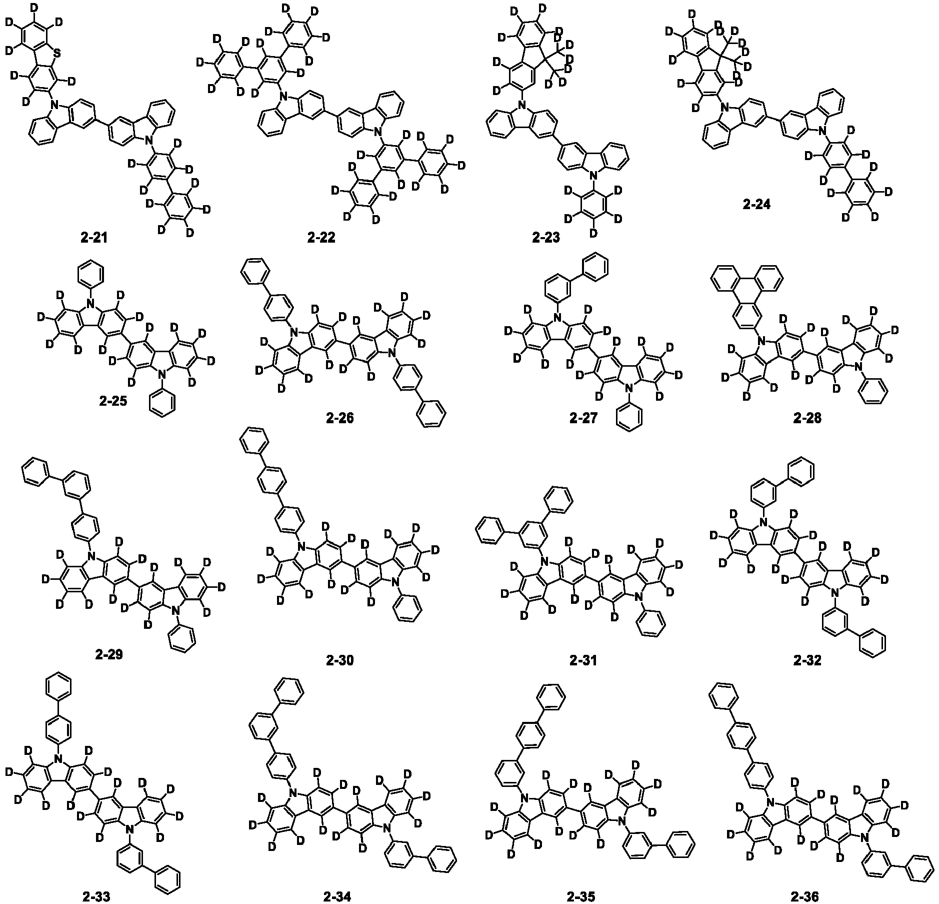

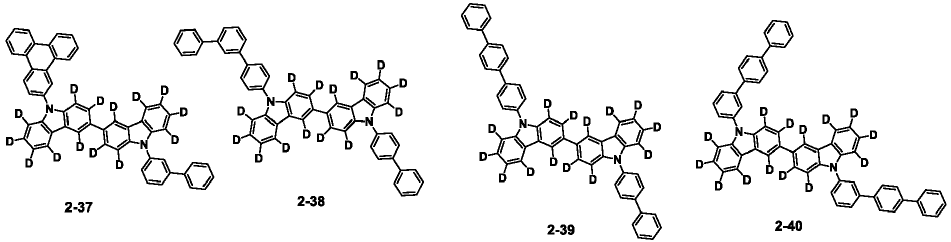

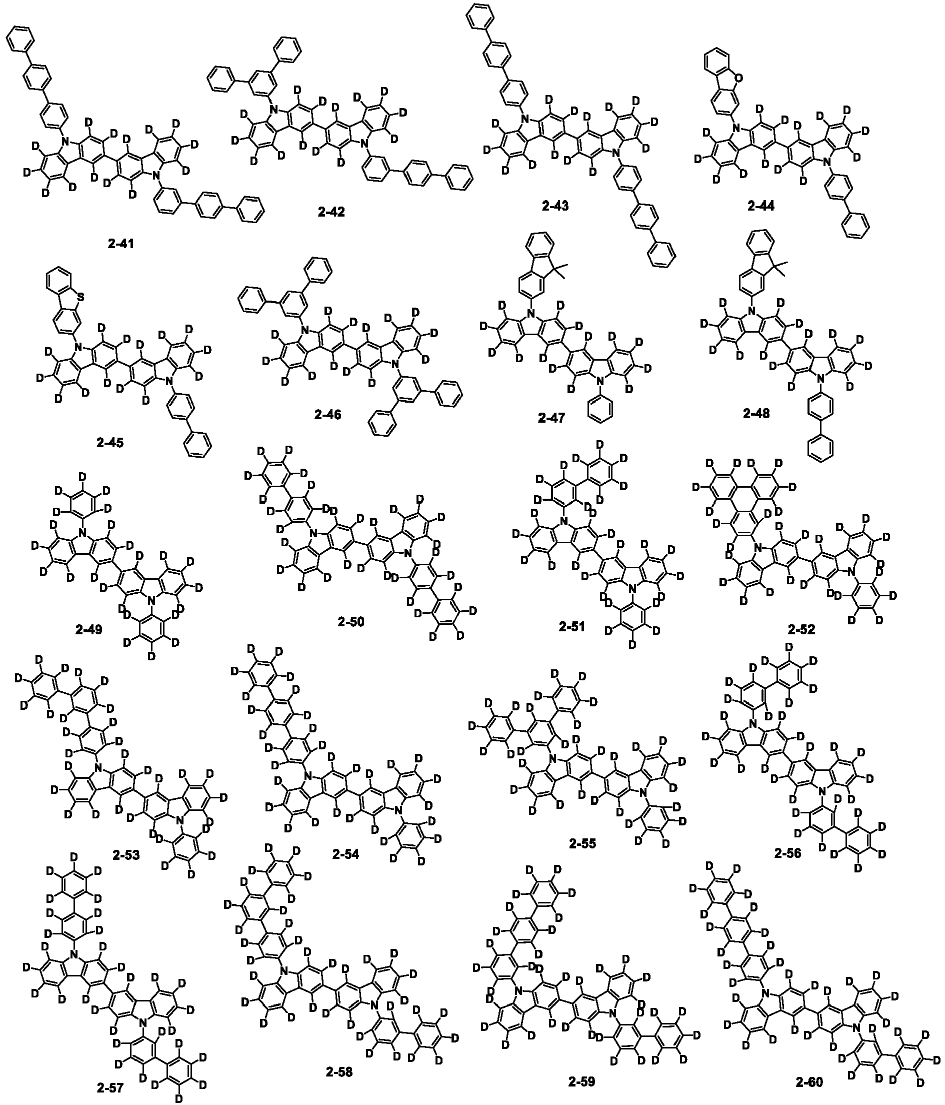

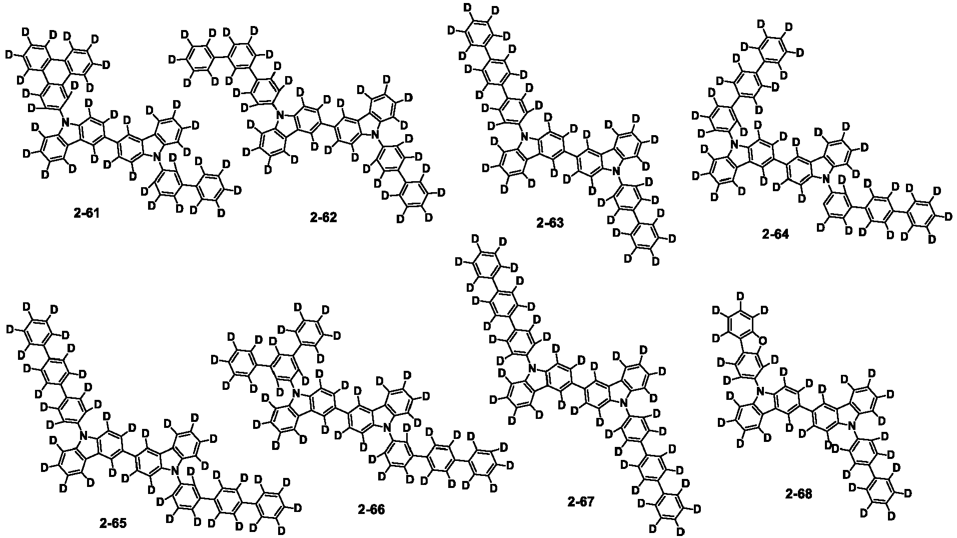

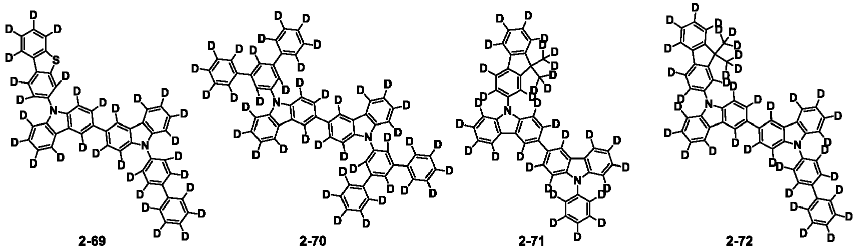

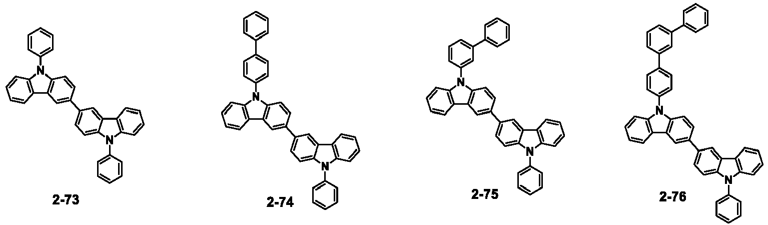

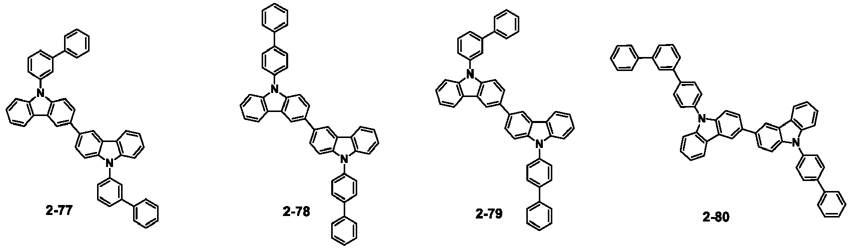

11. The organic light emitting device of claim 7, wherein the organic material layer comprises a light emitting layer, and
the light emitting layer comprises the heterocyclic compound of Chemical Formula 1.

12. The organic light emitting device of claim 8, wherein the organic material layer comprises a light emitting layer, and
the light emitting layer comprises the heterocyclic compound of Chemical Formula 1 and the compound of Chemical Formula 2.

13. The organic light emitting device of claim 7, further comprising one or more layers selected from the group consisting of a light emitting layer, a hole injection layer, a hole transport layer, an electron injection layer, an electron transport layer, an electron blocking layer and a hole blocking layer.

14. A composition for an organic material layer of an organic light emitting device, comprising the heterocyclic compound of claim 1.

15. The composition of claim 14, further comprising a compound of the following Chemical Formula 2: 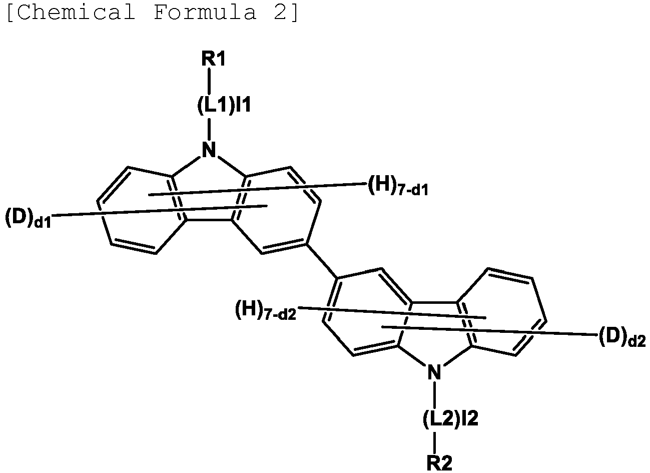 in Chemical Formula 2,
L1 and L2 are each independently a direct bond; or a substituted or unsubstituted arylene group having 6 to 60 carbon atoms,
l1 and l2 are each an integer from 1 to 3, and when l1 and l2 are each 2 or higher, substituents in the parenthesis are the same as or different from each other,
R1 and R2 are each independently hydrogen; deuterium; a halogen group; a cyano group; a substituted or unsubstituted alkyl group having 1 to 60 carbon atoms; a substituted or unsubstituted cycloalkyl group having 3 to 60 carbon atoms; a substituted or unsubstituted heterocycloalkyl group having 2 to 60 carbon atoms; a substituted or unsubstituted aryl group having 6 to 60 carbon atoms; or a substituted or unsubstituted heteroaryl group having 2 to 60 carbon atoms,
H is hydrogen and D is deuterium, and
d1 and d2 are each independently an integer from 0 to 7.

16. The composition of claim 15, wherein a weight ratio of the heterocyclic compound of Chemical Formula 1 and the compound of Chemical Formula 2 in the composition is 1:10 to 10:1.
